# EUROPEAN PATENT APPLICATION

(11) **EP 1 714 970 A1**
(43) Date of publication of application: **25.10.2006**
(21) Application number: 05008850.9
(22) Date of filing: 22.04.2005
(51) Int. Cl.: C07H 21/02, C12N 15/11, A61K 48/00, A61P 9/00, A61P 35/00, A61P 31/00, A61P 43/00

(54) **Use of inhibitors of RNAse A-family enzymes for stabilizing oligonucleotides having RNA interfering activity**

(71) Applicant: Universität des Saarlandes, 66123 Saarbrücken (DE)
(72) Inventor: Piiper, Albrecht, 69121 Heidelberg (DE); Haupenthal, Jörg, 66687 Wadern-Noswendel (DE)
(74) Representative: von Kreisler Selting Werner

(57) **Abstract**

The present invention relates to oligonucleotides having RNAi (RNA interfering) activity and inhibitors directed against a mammalian RNAse of the RNAse A-family. In more detail the present invention relates to a composition or pharmaceutical composition comprising an oligonucleotide having RNAi activity and an inhibitor directed against a mammalian RNAse of the RNAse A-family, and a method for preventing the degradation and/or the loss of activity of said oligonucleotide in a mammal, a mammalian cell culture or a mammalian tissue culture, which comprises providing said inhibitor to said mammal, mammalian cell culture or mammalian tissue culture.

## Description

The present invention relates to oligonucleotides having RNAI (RNA interfering) activity and inhibitors directed against a mammalian RNAse of the RNAse A-family. In more detail the present invention relates to a composition or pharmaceutical composition comprising an oligonucleotide having RNAi activity and an inhibitor directed against a mammalian RNAse of the RNAse A-family, and a method for preventing the degradation and/or the loss of activity of said oligonucleotide in a mammal, a mammalian cell culture or a mammalian tissue culture, which comprises providing said inhibitor to said mammal, mammalian cell culture or mammalian tissue culture.

### Background of the Invention

Small interfering RNAs (siRNA), 21-26-mer double-stranded RNAs, offer a promising approach to specifically degrade RNAs in target cells by a process termed RNA interference. For systemic application of siRNAs in humans, it is important that siRNAs remain active under physiological conditions, most notably in serum. The susceptibility of siRNAs towards degradation in serum was strongly enhanced by local accumulation of A/Us in the sequence of the siRNA, most notably at the ends of the molecule, and by 3'-overhanging bases. The present invention demonstrates that RNAse A-like RNAses degrade siRNAs in serum. The susceptibility of siRNAs towards degradation by serum or recombinant RNAse A is enhanced by 3'-overhanging bases. Most notably, inhibition of RNAse A family members prevents the degradation and activity loss of siRNAs in serum. Furthermore, the present invention shows that the degradation of siRNAs is considerably faster in human than in mouse serum, suggesting that the degradation of siRNAs by RNAse A family enzymes might be a much more challenging problem in a future therapeutic application of siRNAs in humans than in mouse models. Together, the findings of the present invention indicate that the human therapeutic potential of siRNAs can be strongly enhanced by simultaneous inhibition of RNAse A family members.

Small interfering RNAs (siRNA), 21-26-mer double-stranded RNAs (dsRNAs), serve to specifically degrade RNAs in target cells by a process termed RNA interference (RNAi) (Fire, A. et al., Nature 391:806-811 (1998); Zamore, P.D. et al., Cell 101:25-33 (2000); Elbashir, S.M. et al., Nature 411:494-498 (2001)). Because siRNAs can direct degradation of virtually any RNA in mammalian cells, there is enormous interest in exploiting the utility of siRNAs *in vivo* with the goal to use siRNAs as therapeutic agents. For instance, siRNAs have been successfully used to inhibit viral-induced liver-cell inflammation (Song, E. et al., Nat. Med. 9:347-351 (2003); Zender, L. et al., Proc. Natl. Acad. Sci. USA 100,7797-7802 (2003)), human immunodeficiency virus replication (Jacque, J.M. et al., Nature 418:435-438 (2002); Novina, C.D. et al., Nat. Med. 8:681-686 (2002)) and oncogenic K-ras allel-induced tumorigenesis (Brummelkamp, T.R. et al., Cancer Cell 2:243-247 (2002)). Notably, siRNAs targeted against Bcr-Abl, the oncogenic fusion protein that is characteristic for chronic myleogenous leukaemia, have provided excellent proof of principle for RNAi as an anti-cancer therapeutic agent. Bcr-Abl p210 can be selectively downregulated by synthetic siRNAs in cell lines (Scherr, M. et al., Blood 101:1566-1569 (2003)). Despite these proofs of principle, there are important issues and concerns about the therapeutic application of this technology, including difficulties with delivery, bio-stability, and pharmacokinetics and off-target effects (Wall, N.R. and Shi, Y., Lancet 362:1401-1403 (2003); Dykxhoorn, D.M. and Lieberman, J., Annu. Rev. Med. 56:401-423 (2005)).

For systemic application of siRNAs in humans, it is important that the siRNAs remain active under physiological conditions. In the blood, the siRNAs are exposed to serum RNAses known to degrade single-stranded RNAs within seconds. In contrast, dsRNAs are poor substrates of most RNAses and are thus more degradation-resistant than single-stranded RNAs (Libonati, M. and Gotte, G., Biochem. J. 380:311-327 (2004)). Chemical modifications such as 2'-fluoro-pyrimidines have been introduced into siRNAs to improve their biostability (Capodici, J. et al., J. Immunol. 169:5196-5201 (2002); Chiu, Y.L. and Rana, T.M., RNA 9:1034-1048 (2003); Czauderna, F. et al., Nucleic Acids Res. 31:2705-2716 (2003); Braasch, D.A. et al., Biochemistry 42:7967-7975 (2003); Layzer, J.M. et al., RNA 10:766-771 (2004); Soutschek, J. et al., Nature 432:173-178 (2004); Elmen, J. et al., Nucleic Acids Res. 33:439-447 (2005)). However, chemical modifications may reduce the silencing activity of the siRNAs. The complementary strategy, i.e. inhibition of the degradation of siRNAs, is still unexplored.

In addition to unmodified siRNA, chemically modified siRNAs may also be degraded by RNAse A-like enzymes, most notably in the blood circulation or sera. Inhibition of RNAse A-like enzymes may therefore also diminish degradation of chemically modified siRNAs in body fluids or in vitro.

It should be noted, however, that WO 03/010180 discloses the combination of naked RNA with an RNAse inhibitor and its introduction into the vascular system of a multicellular organism. The RNAse inhibitor reduces the activity of or completely inactivates an RNAse in the multicellular organism. The RNAse inhibitor can be a protein inhibitor such as RNasin® (Promega) capable of inhibiting e.g. RNase A and B (Promega product information).

WO 2004/029281 discloses methods and compositions for RNAi mediated inhibition of viral gene expression in mammals whereby the interfering RNA is a siRNA or a shRNA (short hairpin RNA). The interfering RNA is administered together with the protein inhibitor RNasin® (Promega).

However, it is well known in the art that several subtypes of RNAses belong to the group of enzymes commonly referred to as RNAses. Interestingly, there is no teaching in the prior art, which elucidates the particular RNAse responsible for the degradation of a double stranded RNA or combined RNA/DNA, in particular a siRNA in mammals, in particular humans. Accordingly, a strategy aiming for an inhibition of exactly that RNAse involved in the degradation of siRNA has not yet been disclosed or suggested in the prior art. Such inhibition of a specific RNAse is nevertheless highly desirable for therapeutic applications of siRNAs as it considerably reduces the risk of side effects and requires less amounts of inhibitor still being sufficient to engender an inhibitory effect preventing the siRNA from rapid degradation, thus offering the opportunity to fully use the therapeutic potential of the siRNA administered together with the inhibitor of the particular RNAse.

Therefore, the problem underlying the present Invention was to specifically inhibit the degradation of a double stranded RNA or combined RNA/DNA, in particular a siRNA in mammals, in particular humans, which is especially prone to rapid degradation after administration to a mammal due to RNAse enzymes present in serum. Moreover, such inhibition should require as little amounts of the RNAse inhibitor required as possible, i.e. less amounts than required by the methods known so far and inhibiting more randomly also other RNAses not involved in siRNA degradation.

### Summary of the Invention

The present application shows that out of the several RNAses present in serum of mammals, in particular humans, surprisingly RNAse A-like RNAses degrade siRNAs in serum and that the inhibition of these enzymes prevents the degradation and the loss of activity of siRNAs in serum. Thus, the data disclosed in the Examples surprisingly indicate that the human therapeutic potential of siRNAs can be considerably enhanced by simultaneous inhibition of RNAse A family members.

Accordingly, the present invention provides:
(1) a composition or pharmaceutical composition comprising an oligonucleotide having RNAi (RNA interfering) activity and an inhibitor directed against a mammalian RNAse of the RNAse A-family;
(2) the use of a composition or an inhibitor as defined in (1) above for preparing a medicament for systemic application, preferably for degradation of pathologic RNAs, and most preferably for the treatment of viral infections, malign tumors, neoplastic diseases such as cancer, in particular pancreas carcinoma, leukemia, chronic myeloic leukemia, liver cell carcinoma, colon carcinoma, pancreatic carcinoma, breast cancer, circulatory disorders, neurological disorders such as Alzheimer's disease, inherited neurodegenerative diseases such as Huntington's disease or amyotrophic lateral sclerosis, metabolic disorders, hypercholesterolemia, diabetes, atherosclerosis, genetic disorders, inflammatory disorders such as hepatitis and/or infections with parasites;
(3) the use of an inhibitor as defined in (1) above for preventing the degradation and/or the loss of activity of an oligonucleotide having RNAi activity as defined in embodiment (1) in a mammalian cell culture or a mammalian tissue culture;
(4) a method for the systemic treatment of a mammal, which comprises the administration of a pharmaceutically active amount of the composition as defined in (1) above to the mammal in need of such treatment; and
(5) a method for preventing the degradation and/or the loss of activity of an oligonucleotide having RNAi activity as defined in (1) above in a mammal, a mammalian cell culture or a mammalian tissue culture, which comprises providing the inhibitor as defined in (1) above to said mammal, mammalian cell culture or mammalian tissue culture.

### Brief Description of the Figures

All Figures show the results of the separation of the samples indicated by gel electrophoresis on polyacrylamide gels (PAGE).
Figure 1: Degradation of siRNA against STAT3 in sera of different origin.
Figure 2: The degradation of siRNAs strongly depends on its sequence.
Figure 3: RNAseOut prevents degradation and the loss of silencing activity of siRNAs serum.
Figure 4: Depletion of RNAse A immunoreactivity prevents serum-induced degradation of siRNAs, whereas recombinant RNAse A mimics it.

### Sequence Listing Free Text

- SEQ ID NO:1: STAT3 siRNA sense strand
- SEQ ID NO:2: STAT3 siRNA antisense strand
- SEQ ID NO:3: STAT3 19bp dsRNA sense strand
- SEQ ID NO:4: STAT3 19bp dsRNA antisense strand
- SEQ ID NO:5: Plk1 siRNA sense strand
- SEQ ID NO:6: Plk1 siRNA antisense strand
- SEQ ID NO:7: beta-Catenin siRNA sense strand
- SEQ ID NO:8: beta-Catenin siRNA antisense strand
- SEQ ID NO:9: Human K-Ras protein
- SEQ ID NO:10: Human K-Ras cDNA
- SEQ ID NO:11: Human K-Ras siRNA 1 sense strand
- SEQ ID NO:12: Human K-Ras siRNA 2 sense strand
- SEQ ID NO:13: Human K-Ras siRNA 3 sense strand
- SEQ ID NO:14: Human K-Ras siRNA 4 sense strand
- SEQ ID NO:15: Human K-Ras siRNA 5 sense strand
- SEQ ID NO:16: H-RAS protein
- SEQ ID NO:17: H-RAS cDNA
- SEQ ID NO:18: H-RAS siRNA 1 sense strand
- SEQ ID NO:19: H-RAS siRNA 2 sense strand
- SEQ ID NO:20: H-RAS siRNA 3 sense strand
- SEQ ID NO:21: H-RAS siRNA 4 sense strand
- SEQ ID NO:22: H-RAS siRNA 5 sense strand
- SEQ ID NO:23: Human VEGF protein
- SEQ ID NO:24: Human VEGF cDNA
- SEQ ID NO:25: Human VEGF siRNA 1 sense strand
- SEQ ID NO:26: Human VEGF siRNA 2 sense strand
- SEQ ID NO:27: Human VEGF siRNA 3 sense strand
- SEQ ID NO:28: Human VEGF siRNA 4 sense strand
- SEQ ID NO:29: Human VEGF siRNA 5 sense strand
- SEQ ID NO:30: Human VEGF siRNA 6 sense strand
- SEQ ID NO:31: Human VEGF siRNA 7 sense strand
- SEQ ID NO:32: Human VEGF siRNA 8 sense strand
- SEQ ID NO:33: Human VEGF siRNA 9 sense strand
- SEQ ID NO:34: Human VEGF siRNA 10 sense strand
- SEQ ID NO:35: Human IGF2 protein
- SEQ ID NO:36: Human IGF2 cDNA
- SEQ ID NO:37: Human IGF2 siRNA 1 sense strand
- SEQ ID NO:38: Human IGF2 siRNA 2 sense strand
- SEQ ID NO:39: Human IGF2 siRNA 3 sense strand
- SEQ ID NO:40: Human IGF2 siRNA 4 sense strand
- SEQ ID NO:41: Human IGF2 siRNA 5 sense strand
- SEQ ID NO:42: Human IGF2 siRNA 6 sense strand
- SEQ ID NO:43: Human IGF2 siRNA 7 sense strand
- SEQ ID NO:44: Human IGF2 siRNA 8 sense strand
- SEQ ID NO:45: Human IGF2 siRNA 9 sense strand
- SEQ ID NO:46: Human EGFR protein
- SEQ ID NO:47: Human EGFR cDNA
- SEQ ID NO:48: Human EGFR siRNA 1 sense strand
- SEQ ID NO:49: Human EGFR siRNA 2 sense strand
- SEQ ID NO:50: Human EGFR siRNA 3 sense strand
- SEQ ID NO:51: Human EGFR siRNA 4 sense strand
- SEQ ID NO:52: Human EGFR siRNA 5 sense strand
- SEQ ID NO:53: Human EGFR siRNA 6 sense strand
- SEQ ID NO:54: Human EGFR siRNA 7 sense strand
- SEQ ID NO:55: Human EGFR siRNA 8 sense strand
- SEQ ID NO:56: Human c-erb-B-2 protein
- SEQ ID NO:57: Human c-erb-B-2 cDNA
- SEQ ID NO:58: Human c-erb-B-2 siRNA 1 sense strand
- SEQ ID NO:59: Human c-erb-B-2 siRNA 2 sense strand
- SEQ ID NO:60: Human c-erb-B-2 siRNA 3 sense strand
- SEQ ID NO:61: Human c-erb-B-2 siRNA 4 sense strand
- SEQ ID NO:62: Human c-erb-B-2 siRNA 5 sense strand
- SEQ ID NO:63: Human c-erb-B-2 siRNA 6 sense strand
- SEQ ID NO:64: Human c-erb-B-2 siRNA 7 sense strand
- SEQ ID NO:65: Human c-erb-B-2 siRNA 8 sense strand
- SEQ ID NO:66: Human c-erb-B-2 siRNA 9 sense strand
- SEQ ID NO:67: Human cyclin B1 (CCNB1) protein
- SEQ ID NO:68: Human cyclin B1 (CCNB1) cDNA
- SEQ ID NO:69: Human cyclin B1 (CCNB1) siRNA 1 sense strand
- SEQ ID NO:70: Human cyclin B1 (CCNB1) siRNA 2 sense strand
- SEQ ID NO:71: Human cyclin B1 (CCNB1) siRNA 3 sense strand
- SEQ ID NO:72: Human cyclin 81 (CCNB1) siRNA 4 sense strand
- SEQ ID NO:73: Human cyclin B1 (CCNB1) siRNA 5 sense strand
- SEQ ID NO:74: Human cyclin B1 (CCNB1) siRNA 6 sense strand
- SEQ ID NO:75: Human cyclin B1 (CCNB1) siRNA 7 sense strand
- SEQ ID NO:76: Human cyclin D1 protein
- SEQ ID NO:77: Human cyclin D1 cDNA
- SEQ ID NO:78: Human cyclin D1 siRNA 1 sense strand
- SEQ ID NO:79: Human cyclin D1 siRNA 2 sense strand
- SEQ ID NO:80: Human cyclin D1 siRNA 3 sense strand
- SEQ ID NO:81: Human cyclin D1 siRNA 4 sense strand
- SEQ ID NO:82: Human cyclin D1 siRNA 5 sense strand
- SEQ ID NO:83: Human cyclin D1 siRNA 6 sense strand
- SEQ ID NO:84: Human cyclin D1 siRNA 7 sense strand
- SEQ ID NO:85: Human cyclin D1 siRNA 8 sense strand
- SEQ ID NO:86: Human cyclin D1 siRNA 9 sense strand
- SEQ ID NO:87: Human bcl-2 protein
- SEQ ID NO:88: Human bcl-2 cDNA
- SEQ ID NO:89: Human bcl-2 siRNA 1 sense strand
- SEQ ID NO:90: Human bcl-2 siRNA 2 sense strand
- SEQ ID NO:91: Human bcl-2 siRNA 3 sense strand
- SEQ ID NO:92: Human bcl-2 siRNA 4 sense strand
- SEQ ID NO:93: Human bcl-2 siRNA 5 sense strand
- SEQ ID NO:94: Human bcl-2 siRNA 6 sense strand
- SEQ ID NO:95: Human bcl-2 siRNA 7 sense strand
- SEQ ID NO:96: Human bcl-2 siRNA 8 sense strand
- SEQ ID NO:97: Human bcl-2 siRNA 9 sense strand
- SEQ ID NO:98: Human bcl-2 siRNA 10 sense strand
- SEQ ID NO:99: Human acetyl LDL receptor protein
- SEQ ID NO:100: Human acetyl LDL receptor cDNA
- SEQ ID NO:101: Human acetyl LDL receptor siRNA 1 sense strand
- SEQ ID NO:102: Human acetyl LDL receptor siRNA 2 sense strand
- SEQ ID NO:103: Human acetyl LDL receptor siRNA 3 sense strand
- SEQ ID NO:104: Human acetyl LDL receptor siRNA 4 sense strand
- SEQ ID NO:105: Human mTor protein
- SEQ ID NO:106: Human mTor cDNA
- SEQ ID NO:107: Human mTor siRNA 1 sense strand
- SEQ ID NO:108: Human mTor siRNA 2 sense strand
- SEQ ID NO:109: Human mTor siRNA 3 sense strand
- SEQ ID NO:110: Human mTor siRNA 4 sense strand
- SEQ ID NO:111: Human mTor siRNA 5 sense strand
- SEQ ID NO:112: Human mTor siRNA 6 sense strand
- SEQ ID NO:113: Human AKT1 protein
- SEQ ID NO:114: Human AKT1 cDNA
- SEQ ID NO:115: Human AKT1 siRNA 1 sense strand
- SEQ ID NO:116: Human AKT1 siRNA 2 sense strand
- SEQ ID NO:117: Human AKT1 siRNA 3 sense strand
- SEQ ID NO:118: Human AKT1 siRNA 4 sense strand
- SEQ ID NO:119: Human AKT1 siRNA 5 sense strand
- SEQ ID NO:120: Human AKT1 siRNA 6 sense strand
- SEQ ID NO:121: Human AKT1 siRNA 7 sense strand
- SEQ ID NO:122: Human AKT1 siRNA 8 sense strand
- SEQ ID NO:123: Human AKT1 siRNA 9 sense strand
- SEQ ID NO:124: Human AKT1 siRNA 10 sense strand
- SEQ ID NO:125: Human AKT2 protein
- SEQ ID NO:126: Human AKT2 cDNA
- SEQ ID NO:127: Human AKT2 siRNA 1 sense strand
- SEQ ID NO:128: Human AKT2 siRNA 2 sense strand
- SEQ ID NO: 129: Human AKT2 siRNA 3 sense strand
- SEQ ID NO:130: Human AKT2 siRNA 4 sense strand
- SEQ ID NO:131: Human AKT2 siRNA 5 sense strand
- SEQ ID NO:132: Human AKT2 siRNA 6 sense strand
- SEQ ID NO:133: Human AKT2 siRNA 7 sense strand
- SEQ ID NO:134: Human Akt-3 protein
- SEQ ID NO:135: Human Akt-3 cDNA
- SEQ ID NO:136: Human Akt-3 siRNA 1 sense strand
- SEQ ID NO:137: Human Akt-3 siRNA 2 sense strand
- SEQ ID NO:138: Human Akt-3 siRNA 3 sense strand
- SEQ ID NO:139: Human Akt-3 siRNA 4 sense strand
- SEQ ID NO:140: Human Akt-3 siRNA 5 sense strand
- SEQ ID NO:141: PDK1 protein
- SEQ ID NO:142: PDK1 cDNA
- SEQ ID NO:143: PDK1 siRNA 1 sense strand
- SEQ ID NO:144: PDK1 siRNA 2 sense strand
- SEQ ID NO:145: PDK1 siRNA 3 sense strand
- SEQ ID NO:146: PDK1 siRNA 4 sense strand
- SEQ ID NO:147: C-abl protein
- SEQ ID NO:148: C-abl cDNA
- SEQ ID NO:149: C-abl siRNA 1 sense strand
- SEQ ID NO:150: C-abl siRNA 2 sense strand
- SEQ ID NO:151: C-abl siRNA 3 sense strand
- SEQ ID NO:152: C-abl siRNA 4 sense strand
- SEQ ID NO:153: C-myc oncogene protein
- SEQ ID NO:154: C-myc oncogene cDNA
- SEQ ID NO:155: C-myc oncogene siRNA 1 sense strand
- SEQ ID NO:156: C-myc oncogene siRNA 2 sense strand
- SEQ ID NO:157: C-myc oncogene siRNA 3 sense strand
- SEQ ID NO:158: C-myc oncogene siRNA 4 sense strand
- SEQ ID NO:159: C-myc oncogene siRNA 5 sense strand
- SEQ ID NO:160: C-myc oncogene siRNA 6 sense strand
- SEQ ID NO:161: C-myc oncogene siRNA 7 sense strand
- SEQ ID NO:162: C-myc oncogene siRNA 8 sense strand
- SEQ ID NO:163: TGFalpha protein
- SEQ ID NO:164: TGFalpha cDNA
- SEQ ID NO:165: TGFalpha siRNA 1 sense strand
- SEQ ID NO:166: TGFalpha siRNA 2 sense strand
- SEQ ID NO:167: TGFalpha siRNA 3 sense strand
- SEQ ID NO:168: TGFalpha siRNA 4 sense strand
- SEQ ID NO:169: TGFalpha siRNA 5 sense strand
- SEQ ID NO:170: TGFalpha siRNA 6 sense strand
- SEQ ID NO:171: TGFalpha siRNA 7 sense strand
- SEQ ID NO:172: TGFalpha siRNA 8 sense strand
- SEQ ID NO:173: TGFalpha siRNA 9 sense strand
- SEQ ID NO:174: TGFalpha siRNA 10 sense strand
- SEQ ID NO:175: Human beta-catenin protein
- SEQ ID NO:176: Human beta-catenin cDNA
- SEQ ID NO:177: Human beta-catenin siRNA 1 sense strand
- SEQ ID NO:178: Human beta-catenin siRNA 2 sense strand
- SEQ ID NO:179: Human beta-catenin siRNA 3 sense strand
- SEQ ID NO:180: Human beta-catenin siRNA 4 sense strand
- SEQ ID NO:181: Human surviving protein
- SEQ ID NO:182: Human surviving cDNA
- SEQ ID NO:183: Human surviving siRNA 1 sense strand
- SEQ ID NO:184: Human surviving siRNA 2 sense strand
- SEQ ID NO:185: siRNA 1 sense strand
- SEQ ID NO:186: siRNA 1 antisense strand
- SEQ ID NO:187: siRNA 2 sense strand
- SEQ ID NO:188: siRNA 2 antisense strand
- SEQ ID NO:189: siRNA 3 sense strand
- SEQ ID NO:190: siRNA 3 antisense strand
- SEQ ID NO:191: siRNA 4 sense strand
- SEQ ID NO:192: siRNA 4 antisense strand
- SEQ ID NO:193: siRNA 5 sense strand
- SEQ ID NO:194: siRNA 5 antisense strand
- SEQ ID NO:195: siRNA 6 sense strand
- SEQ ID NO:196: siRNA 6 antisense strand
- SEQ ID NO:197: siRNA 7 sense strand
- SEQ ID NO:198: siRNA 7 antisense strand
- SEQ ID NO:199: siRNA 8 sense strand
- SEQ ID NO:200: siRNA 8 antisense strand

### Detailed Description of the Invention

The present invention is the first to show that siRNAs are degraded by RNAse A-like enzymes in serum and that inhibition of RNAse A family enzymes prevented the degradation of siRNAs as well as their loss of activity in serum, thus providing a feasible strategy to prevent loss of activity of the siRNAs upon their systemic application in mammals, most notably in humans. In combination with modifications of the siRNAs that may improve their ability to penetrate into cells (Capodici, J. et al., J. Immunol. 169:5196-5201 (2002); Soutschek, J. et al., Nature 432:173-178 (2004); Schiffelers, R.M. et al., Nucleic Acids Res. 32:e149 (2004)), the inhibition of degradation of the siRNAs may allow the use of much lower doses of siRNAs in a possible future therapeutic application in humans. This could be important to avoid off-target effects of siRNAs, most notably the interferon response (Bridge, A.J. et al., Nat. Genet. 34:263-264 (2003); Sledz, C.A. et al., Nat. Cell. Biol. 5:834-839 (2003)) or activation of Toll-like receptors (Alexopoulou, L. et al., Nature 413:732-738 (2001)). Thus, the data disclosed in the Examples indicate that the human therapeutic potential of siRNAs can be considerably enhanced by simultaneous inhibition of RNAse A family members.

The "oligonucleotide" according to the present invention is a molecule comprising or consisting of from 10 to 100, preferably from 19 to 30, more preferably from 19 to 27, yet more preferably from 21 to 27, and most preferably 21 nucleotides. Said oligonucleotide comprises or consists of RNA-nucleotides or RNA- and DNA-nucleotides and exhibits RNAi activity.

Interference with mRNA expression during gene expression or the degradation of virus-encoded RNA is commonly referred to in the abbreviated format "RNAI".

"RNAi (RNA interfering) activity" according to the present invention is to be understood as the ability to initiate or at least participate in a process leading to the specific degradation of one or more (messenger) RNAs in one or more target cells by a process termed "RNA interference"; such process is known in the art for a while yet (Fire, A. et al., Nature 391:806-811 (1998); Zamore, P.D. et al., Cell 101:25-33 (2000); Elbashir, S.M. et al., Nature 411:494-498 (2001)). For instance, a process leading to the specific degradation of mRNAs in a target cell involves the formation of a so-called "RNA-induced silencing complex (RISC)" under participation of a siRNA. The RISC then targets the messenger RNA (mRNA) for degradation (Radhakrishnan, P.I. et al., Drugs of the Future 28(1): 51-59 (2003); Sontheimer, E.J., Nat. Rev. Mol. Cell Biol. 6(2): 127-138 (2005)).

A "nucleotide" according to the present invention Is a nucleotide occurring in natural DNA or RNA not having undergone chemical modification. However, said nucleotide can be chemically modified. It is preferred said nucleotide not being chemically modified. Particularly preferred is a nucleotide which is not chemically modified in order to enhance the stability, i.e. in order to prevent the degradation and/or the loss of activity of the nucleotide and/or the oligonucleotide or a part thereof in the presence of an RNAse, in particular an RNAse of the RNAse A-family, e.g. in blood serum or any other body fluid of a mammal, in particular humans. However, the nucleotide according to the present invention may nevertheless be chemically synthesized.

A "combined RNA/DNA" (briefly "RNA/DNA") is referred to as an oligonucleotide comprising or consisting of RNA-nucleotides and DNA-nucleotides, preferably thymidine (T), wherein said nucleotides are situated in the polynucleotide preferably in a manner such that all the DNA-nucleotides occur consecutively at each of the termini of each strand, preferably at one terminus of each strand and most preferably at the 3'-terminus of each strand. The term "partly double stranded" (partly ds) refers to a structure (strandedness) of the oligonucleotide concerned, which consists of double stranded domains and one or more regions which show the structural motif of a hairpin loop. However, in addition to one or more hairpin loops the oligonucleotide can contain one or more other loop structural motifs such as bulge loop or interior loop.

The term "siRNA" ("small interfering RNA") according to the present invention refers to a double stranded RNA (dsRNA) or a double stranded combined RNA/DNA (dsRNA/DNA) having RNAi (RNA interfering) activity wherein the dsRNA or dsRNA/DNA comprises from 19 to 30, preferably from 19 to 27, more preferably from 21 to 27, yet more preferably 21 nucleotides, still more preferably the siRNA comprises a nucleotide sequence selected from any of the sequences according to SEQ ID NOs 1-8, 11-15, 18-22, 25-34, 37-45, 48-55, 58-66, 69-75, 78-86, 89-98, 101-104, 107-112, 115-124, 127-133, 136-140, 143-146, 149-152, 155-162, 165-174, 177-180, and 183-184, most preferably the siRNA corresponds to a nucleotide sequence selected from any of the sequences according to SEQ ID NOs 1-8 or has a sense strand corresponding to a nucleotide sequence selected from any of the sequences according to SEQ ID NOs 11-15, 18-22, 25-34, 37-45, 48-55, 58-66, 69-75, 78-86, 89-98, 101-104, 107-112, 115-124, 127-133, 136-140, 143-146, 149-152, 155-162, 165-174, 177-180, and 183-184.

The term "shRNA" ("short hairpin RNA") according to the present invention refers to a partly dsRNA or partly dsRNA/DNA having RNAi activity, wherein the RNA or combined RNA/DNA comprises from 40 to 70, preferably from 50 to 60, and most preferably 55 nucleotides.

A "precursor" of a siRNA is an oligonucleotide according to the present invention, in particular a completely or partly dsRNA, which is cut enzymatically, e.g. by the enzyme complex Dicer, to generate said siRNA.

The siRNA according to the present invention may contain any of the nucleotides A and U at the 3'-end, preferably the last 1 to 5 consecutive, more preferably the last 2 to 4 consecutive nucleotides at the 3'-end are A or U, yet more preferably said 3'-end is on the sense strand, and most preferably the last nucleotide on the sense strand before an overhanging T at the 3'-end is A and the first nucleotide from the 5'-end on the antisense strand is U.

An "overhanging nucleotide" ("overhanging base" or briefly "overhang") according to the present invention is a nucleotide, which extends a first strand (sense strand/antisense strand) of an oligonucleotide, in particular a completely or partly dsRNA or dsRNA/DNA, a siRNA, such that said nucleotide Is not situated opposite a corresponding nucleotide of the complementary second strand and is hence not involved in base pairing with a nucleotide of the complementary second strand (antisense strand/sense strand).

A "3'-overhanging nucleotide" ("3'-overhanging base" or briefly "3'-overhang") refers to an "overhanging nucleotide" ("overhanging base" or "overhang") at the 3'-terminus of a strand of the dsRNA or dsRNA/DNA, in particular siRNA.

A person skilled in the art is well aware of how to find and design an oligonucleotide having RNAi (RNA interfering) activity, particularly siRNAs and shRNAs having RNAi activity (see e.g. Radhakrishnan, P.I. et al., Drugs of the Future 28(1); 51-59 (2003); Elbashir, S.M. et al., Nature 411:494-498 (2001); Khvorova, A., Cell 115:209-216 (2003)).

The siRNAs according to the present invention may be obtained commercially, from a suitable cell after introduction of an appropriately engineered vector, e.g. a plasmid, or by artificial synthesis using methods and protocols well-known in the art (see e.g. Radhakrishnan, P.I. et al., Drugs of the Future 28(1): 51-59 (2003) and references cited therein).

The term "RNAse A" refers to the RNAse A isolated from bovine serum, and having the properties as described in the literature (Sorrentino, S. and Libonati, M., FEBS Letters 404:1-5 (1997); Libonati, M. and Gotte, G., Biochem. J. 380(2):311-327 (2004)).

The term "RNAse A-family" refers to a family of mammalian enzymes consisting of RNAse A and RNAse A-like RNAses.

The term "RNAse A-like RNAse" refers to all mammalian RNAses having functional and structural properties similar to those of bovine RNAse A as described in detail in the literature (Sorrentino, S. and Libonati, M., FEBS Letters 404:1-5 (1997) and references cited therein). Particular RNAse A-like RNAses are pancreatic type RNAses (ptRNAses), in particular ptRNAse 1, and human pancreatic RNAse (hpRNAse).

The terms "mammal" or "mammalian" refer to any mammal known, e.g. rodents such as mouse and rat, cow, including humans. However, mammals being larger than rodents, in particular humans, are particularly considered relevant within the scope of the present invention.

The terms "inhibitor" and " inhibitor directed against a mammalian RNAse of the RNAse A-family" are used herein having the same meaning and refer to any compound, which inhibits an RNAse as defined according to the present invention, so that said RNAse is not anymore capable of degrading an oligonucleotide as defined according to the present invention.

The inhibitor according to the present invention can be a protein, preferably an acidic protein, more preferably an antibody or an antibody fragment, yet more preferably an antibody directed against an RNAse of the RNAse A-family, still more preferably an antibody directed against RNAse A, and most preferably an antibody directed against hpRNAse or a fragment of said antibodies.

The antibodies according to the present invention can be neutralizing antibodies. A neutralizing antibody binds to its substrate, e.g. an RNAse, in a way that the biological activity of the substrate is impaired.

Said protein or antibody fragments can be obtained by recombinant methods known in the art. A suitable recombinant, acidic protein is RNaseOUT^{™}, which is commercially available from Invitrogen Corporation. Other suitable RNAse inhibitors are e.g. the recombinant proteins RNasin® (commercially available from Promega Corporation) and RiboLock^{™} (commercially available from Fermentas GmbH).

However, proteins being similar to the aforementioned ones and which result from the addition, deletion or substitution of one or more amino acids, preferably 1 to 10 amino acids are suitable as well.

Moreover, the inhibitor can be a compound of low molecular weight of from 50 to 1500 dalton, preferably from 100 to 1000 dalton, more preferably from 200 to 850 dalton and most preferably from 150 to 500 dalton.

The concentration of the inhibitor in serum, particularly in human serum is in the range of from 0.01 to 5 U/µl, preferably from 0.1 to 3 U/µl, and most preferably is 3 U/µl (inhibitor/serum).

The decomposition of siRNAs in human serum was completely inhibited by 3 U/µl of RNAseOUT^{™}, and inhibited to half maximum by using 0.1 U/µl.

The concentration of the inhibitor is given in units U of inhibitor per µl of serum, whereby unit U is defined as follows: One unit of the inhibitor inhibits the activity of 5 ng RNAse A by 50%.

A "pharmaceutically acceptable carrier" according to the present invention is a lipid, in particular a liposome, and other carriers (e.g. nanoparticles) well known in the art which may additionally contain components that enhance RNA uptake by the cell, stabilize the annealed strands, or otherwise increase inhibition of the target gene.

The compositions of the present invention can be introduced into the target cell in a number of different ways. The dsRNA, dsRNA/DNA or siRNA may be administered separately from the inhibitor of the RNAse and/or by a different method than the inhibitor of the RNAse is administered if so required.

For example, in the case of an embryo, the compositions are conveniently administered by microinjection. Other methods of introducing nucleic acids into a cell are suitable as well and include bombardment by particles covered by the composition, soaking the cell or organism in a solution of the composition, electroporation of cell membranes in the presence of the composition, liposome- mediated delivery of the composition and transfection mediated by chemicals such as calcium phosphate, viral infection, transformation, and the like.

The composition, in particular the dsRNA, dsRNA/DNA or siRNA may be introduced along with components that enhance RNA uptake by the cell, stabilize the annealed strands, or otherwise increase inhibition of the target gene. In the case of a cell culture or tissue explant, the cells are conveniently incubated in a solution containing the composition or the composition is introduced into the cell by lipid-mediated transfection; in the case of a whole animal or humans, the composition is conveniently introduced by injection or perfusion into a cavity or interstitial space of an organism, or systemically via oral, topical, parenteral (including subcutaneous, intramuscular and intravenous administration), vaginal, rectal, intranasal, ophthalmic, or intraperitoneal administration. In addition, the composition can be administered via an implantable extended release device. Methods for oral introduction include direct mixing of the composition with food of the organism.

The composition is typically administered in an amount that allows delivery of at least one copy of dsRNA, dsRNA/DNA or siRNA per target cell. The amount of composition administered to a cell, tissue, or organism depends on the nature of the cell, tissue, or organism, the nature of the target gene, and the nature of the dsRNA, dsRNA/DNA or siRNA, and can readily be optimized to obtain the desired level of gene inhibition.

"Systemic administration" involves any kind of administration of the compositions according to the present invention to an organism, which allows for a distribution of the compositions within the organism which is not locally limited by means of the method of administration employed. Such a systemic administration may for instance be an oral, topical, parenteral (including subcutaneous, intramuscular and intravenous administration), vaginal, rectal into the intestine, e.g. using a suppository, intranasal, ophthalmic, or intraperitoneal administration, or be accomplished by absorption through the skin or by any other known method suitable for systemic administration.

The compositions or the inhibitor of the present invention are effective as medicaments for the treatment of various diseases such as viral infections, malign tumors, neoplastic diseases such as cancer, in particular pancreas carcinoma, leukemia, chronic myeloic leukemia, liver cell carcinoma, colon carcinoma, pancreatic carcinoma, breast cancer, circulatory disorders, neurological disorders such as Alzheimer's disease, inherited neurodegenerative diseases such as Huntington's disease or amyotrophic lateral sclerosis, metabolic disorders, hypercholesterolemia, diabetes, atherosclerosis, genetic disorders, inflammatory disorders such as hepatitis and/or infections with parasites.

Moreover, the compositions of the present invention are effective for the degradation of any pathologic RNA.

### Pharmaceutical Uses of the siRNAs

The siRNAs consisting of or comprising any of the sequences identified by the following SEQ ID NOs, and pharmaceutical compositions containing said siRNAs can be used for the treatment of certain disorders indicated as follows:
- SEQ ID NOs:11-15: malign tumors, in particular pancreas carcinoma
- SEQ ID NOs:18-22: malign tumors
- SEQ ID NOs:25-34: malign tumors
- SEQ ID NOs:37-45: malign tumors, in particular liver cell carcinoma (hepatocellular carcinoma)
- SEQ ID NOs:48-55: carcinomas, in particular lung carcinoma, colon carcinoma, pancreatic carcinoma, breast cancer
- SEQ ID NOs:58-66: carcinomas, in particular breast cancer
- SEQ ID NOs:69-75: carcinomas
- SEQ ID NOs:78-86: malign tumors
- SEQ ID NOs:89-98: malign tumors
- SEQ ID NOs:101-104: hypercholesterolemia, atherosclerosis
- SEQ ID NOs:107-112: diabetes, malign tumors
- SEQ ID NOs:115-124: diabetes, malign tumors
- SEQ ID NOs:127-133: diabetes, malign tumors
- SEQ ID NOs: 136-140: diabetes, malign tumors
- SEQ ID NOs:143-146: diabetes, malign tumors
- SEQ ID NOs:149-152: chronic myeloic leukemia
- SEQ ID NOs:155-162: malign tumors, in particular liver cell carcinoma
- SEQ ID NOs:165-174: malign tumors
- SEQ ID NOs:177-180: malign tumors
- SEQ ID NOs:183-184: malign tumors

### Examples

### General Methods

RNAs and annealing: The RNAs were obtained as single strands from Dharmacon (Lafayette, CO). For annealing, complementary and deprotected single stranded RNAs were incubated at 60°C for 45 min. Then, the RNAs were allowed to cool down to room temperature for 30 min. The duplex RNAs were desalted according to the protocol of the manufacturer (Dharmacon, Lafayette, CO) and dissolved in siRNA buffer.

Sequences of the siRNAs used in the Examples:
STAT3 siRNA:
   5'- UGAGUUGAAUUAUCAGCUUdTdT -3'
   3'- dTdTACUCAACUUAAUAGUCGAA -5'
STAT3 19 bp dsRNA:
   5'- UGAGUUGAAUUAUCAGCUU -3'
   3'- ACUCAACUUAAUAGUCGAA -5'
Plk1 siRNA:
   5'- AGACCUACCUCCGGAUCAAdTdT -3'
   3'- dTdTUCUGGAUGGAGGCCUAGUU -5'
β-Catenin siRNA:
   5'- UGCCGUUCGCCUUCAUUAUdTdT -3'
   3'- dTdTACGGCAAGCGGAAGUAAUA -5'

SiRNA degradation assay: 1.5 µg duplex RNA were incubated in 15 µl of human serum (prepared from healthy donors), mouse serum (freshly prepared) or fetal bovine serum (Invitrogen, Grand Island, NY) for various duration at 37°C. Where indicated, 40-70 units of RNAseOUT (Invitrogen) were added to the serum prior to the addition of the duplex RNAs. Alternatively, the siRNAs were incubated in the presence of the indicated amount of recombinant RNAse A (Sigma, St. Louis, MO).

For extraction of the duplex RNAs, 20 µl of RNAse-free water, 3.5 µl Z M NaAc (pH 5.2) and 100 µl phenol were sequentially added to the serum. The samples were mixed, incubated on ice for 10 min, and centrifuged at 10,000 g for 8 min. The supernatants were collected and mixed with an equal volume of chloroform. After an additional centrifugation (10,000 g for 3 min), the supernatants were separated in 20% polyacrylamide gels.

RNA gel electrophoresis: sRNA or siRNA samples were separated in 20% native polyacrylamide gels. RNAs were visualized by silver staining.

Cell culture and transfection of Huh7 cells: Huh7 cells were grown on 24-well tissue culture plates in RPMI1640-medium ("RPMI 1640") supplemented with 10% FCS and antibiotics (Invitrogen) to 40% confluence. 1 h prior to the transfection, the cells were switched to serum-reduced medium (OptI-MEM I, Invitrogen). The duplex RNAs were transfected into the cells using 1 µl of Lipofectamine 2000 (Invitrogen) according to the recommendation of the manufacturer. After 6 h of incubation, the medium was replaced by complete medium.

Immunodepletion of RNAse A-like RNAses from serum: Sera were preincubated with protein G-Sepharose (Amersham-Pharmacia, Piscataway, NJ) for 1 h. The supernatants were divided in two aliquots. One aliquot was incubated with an anti-RNAse A antibody (Acris Antibodies, Hiddenhausen, Germany), the other aliquot with an equal amount of an irrelevant antibody (anti-STAT3, Santa Cruz, Santa Cruz, CA) for 2 h at 4°C with gentle rocking, followed by the addition of protein G-Sepharose (Amersham-Pharmacia) and continuation of the incubation for 1 h. After brief centrifugation, the supernatants were used for the siRNA degradation assay.

Protein electrophoresis and Immunoblotting: The samples were matched for protein concentration and separated on SDS polyacrylamide gels as described previously (Elez, R. et al., Oncogene 22:69-80 (2003)). Gel-resolved proteins were electrotransferred to nitrocellulose membranes, and immunoblotted with anti-STAT3 (Santa Cruz, Santa Cruz, CA) or anti-Plk1 (BD Biosciences, San Diego, CA), which was performed as recently described (Piiper, A. et al., J. Biol. Chem. 277:43623-43630 (2002); Piiper, A. et al., J. Biol. Chem. 278:7065-7072 (2003)). Equal loading of the lanes was controlled by Ponceau S staining. Antigen-antibody complexes were visualized using horseradish peroxidase (HRP)-conjugated antibody and the enhanced chemiluminescence system (Pierce, Rockford, IL). After scanning, the densities of the bands were quantified densitometrically using the Gelscan Professional V5.02 software (BioSciTec, Frankfurt, Germany).

### Example 1: SiRNAs are degraded via stable intermediates in serum

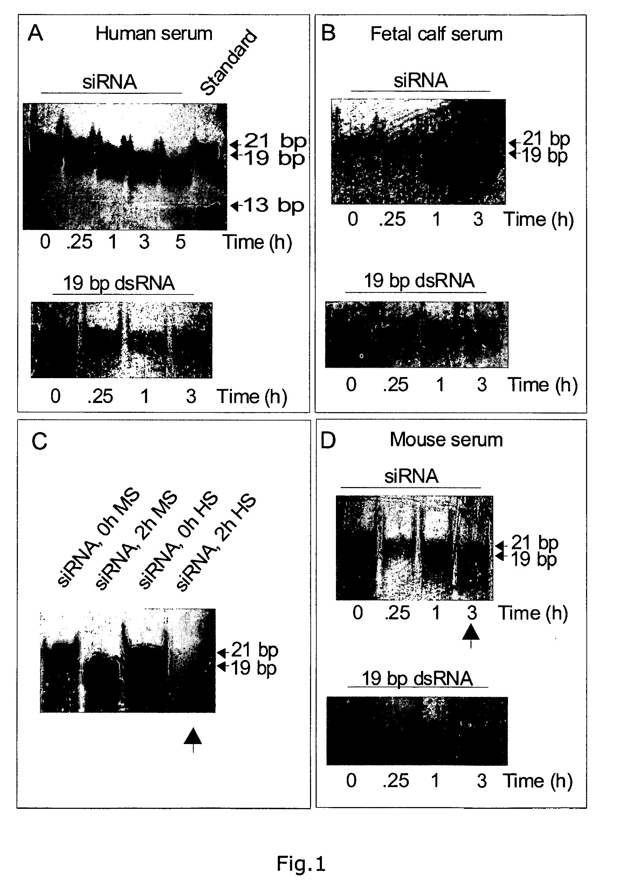

The elucidation of the influence of serum on the integrity of siRNAs and the processes that limit their silencing activity is of great importance for a possible future therapeutic application of siRNAs and is likely to result in feasible strategies to prevent loss of biological activity upon their systemic application in mammals. To explore the mode of degradation of siRNAs in serum, synthetic 21 bp siRNAs containing 3'-dTdT-overhangs (against STAT3) were incubated in mouse, bovine or human serum for various duration at 37°C followed by their extraction and separation in polyacrylamide gels. The RNA was visualized in the gels by silver-staining. In human serum, the siRNA became progressively shortened up to degradation intermediates considerably shorter than the 19 bp blund end dsRNA (Figure 1A and 1C). In bovine serum, the degradation of the siRNA differed from that in human serum in that the total amount of RNA declined considerably within the observation period of 3 h and the kinetics of degradation was slower, i.e. significant amounts of RNA still co-migrated with the 19 bp dsRNA (Figure 1B). Because mouse models are widely used to investigate the possible therapeutic potential of siRNAs (Song, E. et al., Nat. Med. 9:347-351 (2003); Soutschek, J. et al., Nature 432:173-178 (2004)), we also examined the stability of siRNAs in mouse serum. The kinetics of digestion of the siRNA in mouse serum resembled that in bovine serum (Fig. 1B with 1D). After 2-3 h of incubation, the RNA extracted from mouse or bovine serum migrated only slightly faster than the intact siRNA and instead comigrated with a synthetic 19-mer blunt end dsRNA corresponding to the siRNA (Figure 1C and 1D). An important implication of these findings is that the systemic application of siRNAs in mice may not reflect their degradation in human serum, in which siRNAs were considerably more rapidly degraded. When siRNAs are injected i. v. by the high-pressure method as frequently performed to test the effects of siRNA upon their systemic application (Lewis, D.L, et al., Nat. Genet. 32:107-108 (2002); Xia, H. et al., Nature Biotechnol. 20:1006-1010 (2002); Song, E. et al., Nat. Med. 9:347-351 (2003)), the siRNAs are likely to escape degradation in the circulation of the mouse because of the short time of exposure of the siRNAs to serum RNAses due to their rapid uptake into the target cells. The study leading to the present invention indicates that a possible future therapeutic application of siRNAs in humans will require a higher biostability of the siRNAs than essential for down-modulation of target RNA during hydrodynamic application of siRNAs in mice.

### Example 2: The degradation of siRNAs strongly depends on its sequence

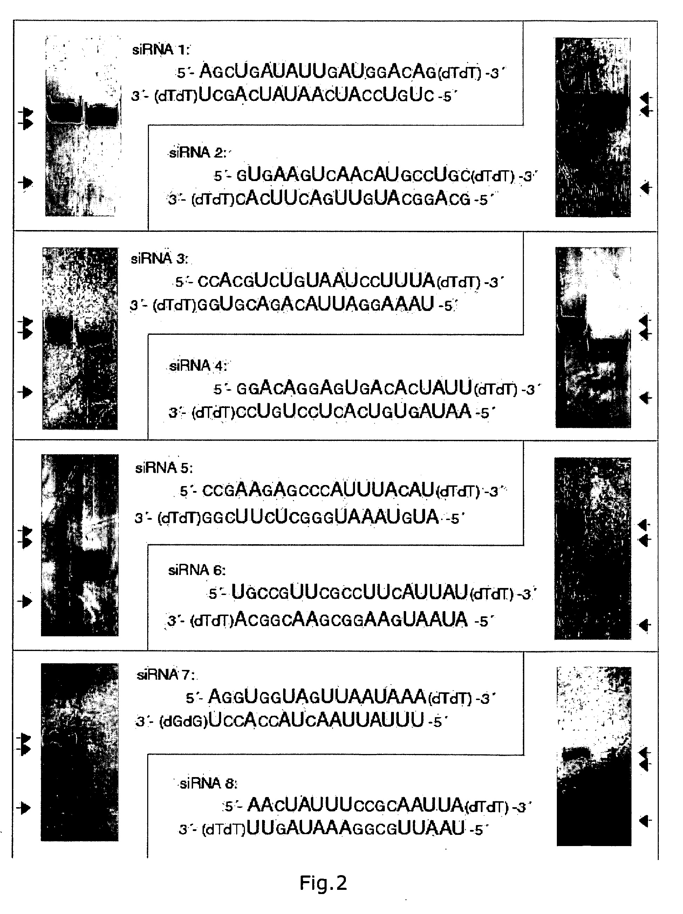

To investigate the Influence of the sequence of the siRNA on its degradation in human serum, different siRNAs were exposed to human serum for 2 h followed by their extraction and separation in polyacrylamide gels. As illustrated in Figure 2, the kinetics of degradation of the siRNAs strongly depended on their sequence. siRNAs containing only few A/U at the ends of the molecule (siRNA 1 and 2) were very stable, showing only minimal degradation after an incubation of 2 h in human serum. However, functional siRNAs or micro-RNAs contain a high flexibility at the 5'-end of the antisense strand of the duplex RNA and relative instability at the cleavage site in the middle of the siRNA molecule (Khvorova et al., Cell 115:209-216 (2003); Schwarz et al., Cell 115:199-208 (2003); Silva et al., Nat. Genet. 35:303-305 (2003)). These properties of the siRNA appear to be important to lead the antisense stand into RISC (Schwarz et al., Cell 115:199-208 (2003); Tomari and Zamore, Genes Dev. 19:517-529 (2005)) and thus strongly restrict the choice of functional siRNAs within the target RNA sequence. siRNA molecules containing 4 consecutive A/Us in the 5'-end of the antisense strand were relatively degradation-resistant in human serum (Figure 2, siRNA 3 and 4). However, further destabilization of the 5'-end of the antisense strand of the siRNA molecule resulted in considerably shorter degradation products (siRNAs 5 and 6) or even in a degradation into products smaller than 13 bp (siRNA 7) within 2 h of incubation. Degradation of the siRNA into products of less than 13 bp was also observed for an siRNA enriched in A/U at both ends of the molecule (siRNA 8). These data demonstrate that the susceptibility of siRNAs towards degradation in serum depends on their sequence and is strongly enhanced by thermodynamic instability preferably at the ends of the molecule. 5'-flexibility in the antisense strand of the siRNA is nevertheless required for siRNA activity (Khvorova et al., Cell 115:209-216 (2003); Schwarz et al., Cell 115:199-208 (2003); Silva et al., Nat. Genet. 35:303-305 (2003)).

### Example 3: 3'-Overhanging bases enhance the susceptibility of siRNAs to degradation by serum RNAses

To determine the influence of the 3'-overhangs on the stability of siRNAs in serum, we investigated the effect of the three different sera on the integrity of the 19 bp blunt end dsRNA whose sequence corresponded to the STAT3-siRNA. This blunt ended dsRNA was more resistant towards degradation in all three different sera in comparison to the siRNA (Figure 1A, 1C, 1D). The enhanced stability of the 19 bp blunt end dsRNA compared to the corresponding siRNA was particularly striking in human serum (Figure 1A). These data indicate that the 3'-overhangs enhanced the susceptibility of siRNAs to degradation by serum RNAses.

### Example 4: Inhibition of RNAse A-like enzymes ablates degradation of siRNAs in serum

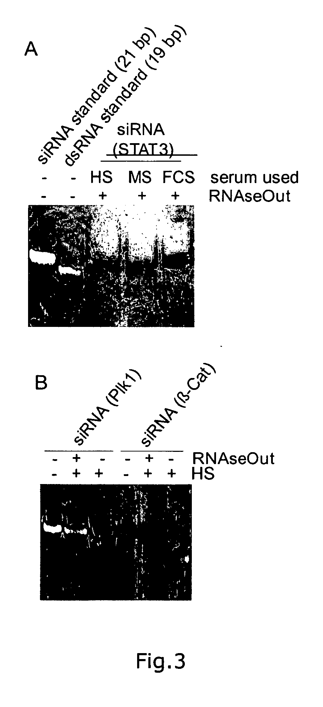

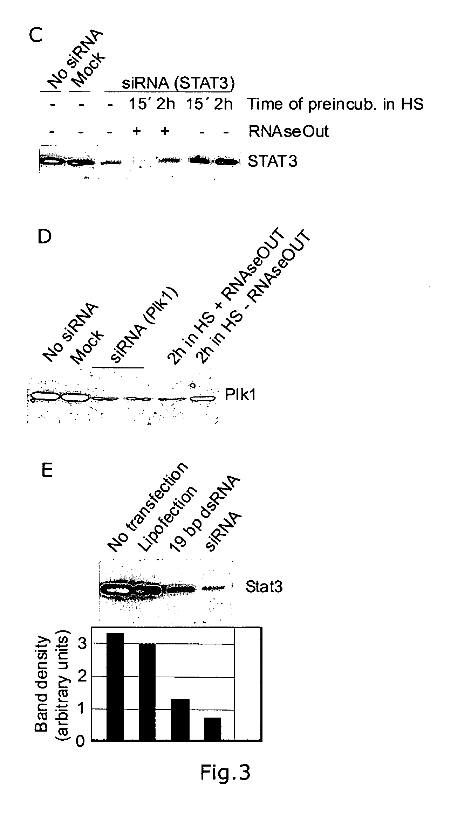

Pancreatic type RNAses, for which bovine RNAse A is the prototype, possess helix-destabilizing activity towards dsRNA and digest dsRNA, although to a much lower rate than single-stranded RNA (Libonati, M. and Gotte, G., Biochem. J. 380:311-327 (2004)). Most notably, human pancreatic RNAse (HP-RNAse), which is structurally and functionally very similar to bovine RNAse A, accounts for approximately 70% of RNAse activity contained in human serum (Weickmann, J.L. and Glitz, D.G., J. Biol. Chem. 257:8705-8710 (1982); Morita, T. et al., J. Biochem. 99:17-25 (1986); Sorrentino, S. et al., Biochim. Biophys. Acta 998:97-101 (1989)). Thus, members of the RNAse A family were good candidates to mediate degradation of siRNAs in serum. To address a possible role of RNAse A-like RNAses in the degradation of siRNAs in serum, we studied the effect of RNAse A family inhibition by RNAseOut on serum-induced siRNA degradation. As illustrated in Figure 3, RNAseOut completely prevented the degradation of all different siRNAs tested (as shown for STAT3, Plk1 and β-catenin) in human, mouse and bovine serum. Similar results were obtained with RlboLock and RNAsin, two other commercially available inhibitors of RNAse A family enzymes (data not shown). These data suggest that RNAse A family enzymes constitute the major enzymatic activity that degrades siRNAs in serum and that specific inhibition of these RNAses strongly increases the half life of siRNAs in serum.

### Example 5: Inhibition of RNAse A-like RNAses prevents serum-induced loss of silencing activity of siRNAs

The RNAi activity declines dramatically when the RNA duplex length drops below 19 bp (Elbashir, S.M. et al., EMBO J. 20:6877-6888 (2001); Czauderna, F. et al., Nucleic Acids Res. 31:2705-2716 (2003)). To investigate if RNAse A family enzyme inhibition protects siRNAs from inactivation in serum, the siRNA raised against STAT3 was exposed to human serum for 15 min or 2 h, followed by their extraction. Equal amounts of the extracted RNAs as well as of non-exposed siRNAs were transfected into Huh7 cells. 72 h after transfection, down-modulation of STAT3 was determined by immunoblotting. As shown in Figure 3C, RNAseOut completely prevented the loss of silencing activity of the siRNA upon their preincubation in human serum for 15' or 2 h. RNAseOut also prevented the loss of RNAI activity of a siRNA against polo-like kinase 1 (Plk1) (Figure 3D), a possible target for an anti-tumor therapy (Elez, R. et al., Biochem. Biophys. Res. Commun. 269:352-356 (2000); Elez, R. et al., Oncogene 22:69-80 (2003)). These data indicate that inhibition of RNAse A family enzymes prevents the loss of silencing activity of siRNAs in serum, which might be of great importance for a possible future therapy with siRNAs in humans.

Because the two 3'-overhanging bases appear to sensitize the siRNAs for serum-induced degradation, one strategy to reduce the rate of degradation in serum would be to use blunt end dsRNAs instead of siRNAs containing 3'-overhanging bases. However, this may reduce their silencing activity (Elbashir, S.M. et al., EMBO J. 20:6877-6888 (2001)). To investigate if prevention of the loss of the 3'-overhangs of the siRNAs is important for the biological activity of the duplex RNAs, we compared the silencing activity of the STAT3-siRNA containing two 3'-overhanging bases (dTdT) with that of the corresponding 19 bp blunt end dsRNA. We found that in agreement with the results from Tuschl and colleagues (Elbashir, S.M. et al., EMBO J. 20:6877-6888 (2001)), the 21 bp siRNA containing the 3'-overhangs was approximately two times more effective in eliciting RNAi than the 19 bp blunt end dsRNA (Figure 3E), indicating that the prevention of the loss of the 3'-overhangs of siRNAs in serum may enhance their silencing efficacy in humans compared to the utilization of the corresponding blunt end 19 bp dsRNAs.

### Example 6: Human serum contains RNAse A immunoreactivity and depletion of serum with anti-RNAse A prevents the degradation of siRNA in serum

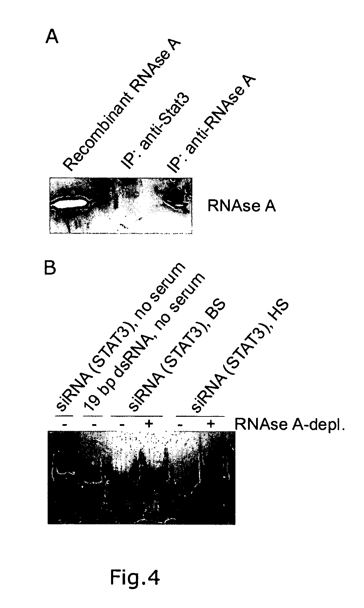

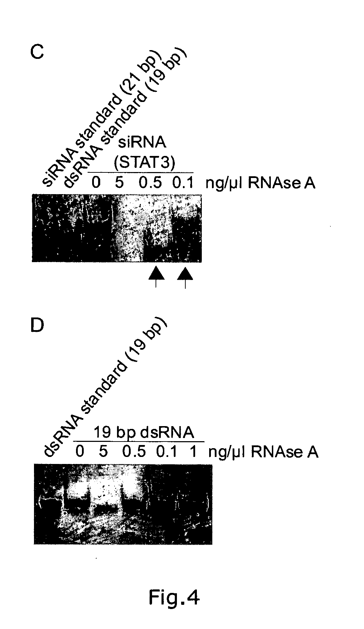

Antibodies raised against bovine RNAse A recognize RNAses in human serum, indicating the presence of RNAses closely related to bovine RNAse A in human serum. Most notably, HP-RNAse is recognized by anti-RNAse A antibodies and constitutes a significant proportion of all RNAses contained in human serum (Weickmann, J.L. and Glitz, D.G, J. Biol. Chem. 257:8705-8710 (1982); Morita, T. et al., J. Biochem. (Tokyo) 99:17-25 (1986); Sorrentino, S. et al., Biochem. Acta 998:97-101 (1989)). To investigate if RNAse A family enzymes were detectable In our sera that could mediate the degradation of siRNAs in serum, we examined human and bovine sera for the presence of RNAse A immunoreactivity using an antibody raised against bovine RNAse A. Using this antibody, a band at 14 kDa comigrating with bovine recombinant RNAse A was identified in human serum (Figure 4A). As HP-RNAse has a similar molecular mass, it is possible that HP-RNAse is the major enzymatic activity that degrades siRNAs in serum. Depletion of RNAse A immunoreactivity from human or bovine serum ablated the degradation of the siRNA in these sera (Figure 4B), supporting the notion that RNAse A-like enzymes mediate the degradation of siRNAs in serum.

### Example 7: Exposure of siRNAs to recombinant RNAse A mimics serum-induced degradation of siRNA

To obtain further information on the functional properties of the RNAse that degrades siRNAs in serum, we compared the degradation of siRNAs in serum with that obtained upon exposure to recombinant RNAse A. As shown in Figure 4C, the degradation pattern of the siRNA in human and mouse sera could be mimicked by 0.5 ng/µl (compare Figure 1C, last lane marked by an arrow with Figure 4C, lane marked by left arrow) and 0.1 ng/µl (compare Figure 1D, lane 4 marked by an arrow with Figure 4C, last lane) of recombinant RNAse A, respectively. Furthermore, similar to the stability of the duplex RNAs in serum, degradation of the blunt end dsRNA by recombinant RNAse A required approximately 50-fold higher amounts of RNAse A than essential for the degradation of the 21-mer siRNA (compare Figures 4C and 4D). Together, these data indicate that RNAse A-like RNAses constitute the major enzymatic activity that degrades siRNAs in serum and thus may provide a feasible strategy to prevent loss of biological activity of siRNAs in the blood circulation.

RNAse A family enzymes have a much higher affinity for single-stranded RNA than dsRNA (Libonati and Gotte, Biochem. J. 380:311-327 (2004)). Our result that 3'-overhangs and A/U rich domains within siRNAs preferably at the ends of the siRNAs confer the siRNAs susceptible to degradation by RNAse A-like RNAses is in agreement with the properties of RNAse A and its proposed mechanism of action of dsRNA: the 3' overhangs may allow RNAse A to bind to and then to digest single-stranded domains generated by thermal fluctuation within the dsRNA secondary structure, thus allowing its degradation (Libonati and Gotte, Biochem. J. 380:311-327 (2004)). The extent of single strand formation by thermal fluctuation highly depends on A/U content of that particular sequence and thus may explain why A/U rich stretches render siRNAs susceptible for degradation by serum-derived RNAses.

### Detailed Description of the Figures

Figure 1: A chemically synthesized siRNA against STAT3 and a 19 bp dsRNA were incubated in human (Figure 1A, Figure 1C), bovine (B) or mouse serum (C, D) for the indicated duration at 37°C. Thereafter, the RNAs were extracted and separated in non-denaturing polyacrylamide gels. As molecular weight standards, non-treated 21, 19 and 13 bp dsRNAs were co-electrophorezed (Figure 1A, last lane). Horizontal arrows indicate the migration of 21 bp siRNA and 19 bp dsRNA, respectively, which had not been exposed to serum. Vertical arrows refer to the respective lanes also marked by arrows in Figure 4. HS, human serum; MS, mouse serum.
Figure 2: The siRNAs were incubated in human serum for 2 h at 37°C. Thereafter, the RNAs were extracted and separated in non-denaturing polyacrylamide gels. The arrows denote the position of non-treated 21, 19 and 13 bp dsRNAs co-electrophorezed as molecular weight standards.
Figure 3: siRNAs of STAT3 (Figure 3A), Plk1 or β-catenin (β-Cat) (Figure 3B) were incubated in human (HS), mouse (MS) or bovine serum (BS) for 2 h at 37°C in the presence or absence of RNAseOut (70 units/assay). Non-treated siRNA and the 19 bp dsRNA were co-electrophorezed as molecular weight markers. RNAs were visualized by silver-staining.
   Figure 3C and D: siRNA raised against human STAT3 (C) or Plk1 (D) were preincubated in human serum (HS) for the indicated duration followed by their extraction. The extracted as well as non-preincubated siRNAs were transfected into Huh7 cells. 72 h (Figure 3C) or 48 h (Figure 3D) after transfection, STAT3 and Plk1 expression were determined by immunoblotting. (Figure 3E): 3'-overhangs increase the gene silencing effect of siRNA compared to the corresponding 19 bp blunt end dsRNA. 20 nM of siRNA raised against human 5TAT3 or the 19 bp dsRNA were transfected into Huh7 cells by lipofection. 48 h after transfection, STAT3 expression in the cells was determined by immunoblotting. The density of bands was determined densitometrically.
Figure 4: (Figure 4A): Presence of RNAse A immunoreactivity in human serum. 100 µl of serum was incubated with protein G-sepharose for 1 h. The supernatant was immunoprecipitated with 10 µg anti-RNAse A or an irrelevant antibody (anti-STAT3) for 2 h at 4°C followed by the addition of protein G-sepharose. Immune complexes as well as recombinant RNAse A were analyzed by anti-RNAse A immunoblotting. (Figure 4B): Human (N5) or bovine serum (BS) were depleted with anti-RNAse A (+) or an irrelevant antibody (-) as described in the Methods section. Then, the STA3 siRNA was incubated in these sera for 2 h, followed by RNA extraction, gel separation and silver staining. Figure 4C and 4D: The STAT3 siRNA (C) or the 19bp dsRNA (D) were incubated with the indicated amount of recombinant RNAse A for 2 h followed by the extraction of the RNA, electrophoretic separation and silver staining.

## Claims

1. A composition or pharmaceutical composition comprising an oligonucleotide having RNAi (RNA interfering) activity and an inhibitor directed against a mammalian RNAse of the RNAse A-family.

2. The composition according to claim 1, wherein the oligonucleotide is a completely or partly double stranded RNA (dsRNA) or combined RNA/DNA (dsRNA/DNA), a partly double stranded RNA (dsRNA) or combined RNA/DNA (dsRNA/DNA) having a loop structure, a small interfering RNA (siRNA), a short hairpin RNA (shRNA) or a precursor thereof, preferably is a siRNA, more preferably is a siRNA which comprises from 19 to 30, yet more preferably from 19 to 27, still more preferably from 21 to 27, and most preferably 21 nucleotides.

3. The composition according to claim 2, wherein the siRNA
(i) does contain any of the nucleotides A and U at the 3'-end, preferably the last 1 to 5, more preferably the last 2 to 4 consecutive nucleotides at the 3'-end are A or U, yet more preferably said 3'-end is on the sense strand, and most preferably the last nucleotide on the sense strand before an overhanging T at the 3'-end is A and the first nucleotide from the 5'-end on the antisense strand is U; and/or
(ii) has from 0 to 3, preferably from 0 to 2, more preferably 1 or 2, and most preferably 2 overhanging nucleotides at the 3'-terminus of each strand; and/or
(iii) has overhanging DNA-nucleotides at the 3'-terminus of each strand; and/or
(iv) has overhanging DNA-nucleotides which are thymidine (T); and/or
(v) comprises a nucleotide sequence selected from any of the sequences according to SEQ ID NOs 1-8, 11-15, 18-22, 25-34, 37-45, 48-55, 58-66, 69-75, 78-86, 89-98, 101-104, 107-112, 115-124, 127-133, 136-140, 143-146, 149-152, 155-162, 165-174, 177-180, and 183-184, preferably corresponds to any of the sequences according to SEQ ID NOs 1-8 or has a sense strand corresponding to a nucleotide sequence selected from any of the sequences according to SEQ ID NOs 11-15, 18-22, 25-34, 37-45, 48-55, 58-66, 69-75, 78-86, 89-98, 101-104, 107-112, 115-124, 127-133, 136-140, 143-146, 149-152, 155-162, 165-174, 177-180, and 183-184.

4. The composition according to any of claims 1 to 3, wherein
(i) the inhibitor is directed against RNAse A or an RNAse A-like RNAse, preferably an ptRNAse (pancreatic type RNAse), more preferably a human ptRNAse 1 and most preferably hpRNAse (human pancreatic RNAse); and/or
(ii) the inhibitor is a protein, preferably is an acidic protein, more preferably is an antibody or an antibody fragment, yet more preferably is an antibody directed against an RNAse of the RNAse A-family, still more preferably is an antibody directed against RNAse A, and most preferably is an antibody directed against hpRNAse or a fragment thereof; and/or
(iii) the inhibitor is a compound of low molecular weight of from 50 to 1500 dalton, preferably of from 100 to 1000 dalton, more preferably of from 200 to 850 dalton, and most preferably of from 150 to 500 dalton; and/or
(iv) the concentration of inhibitor is in the range of from 0.01 to 5 U/µl, preferably from 0.1 to 3 U/µl, and most preferably 3 U/µl (inhibitor/serum).

5. The pharmaceutical composition according to any of claims 1 to 5 further comprising a pharmaceutical acceptable carrier.

6. Use of a composition as defined in any of claims 1 to 5 or an inhibitor as defined in claim 1 or 4 for preparing a medicament for systemic application, preferably for systemic application of an oligonucleotide having RNAi activity.

7. The use according to claim 6, wherein the medicament is suitable for degradation of pathologic RNAs, preferably for the treatment of viral infections, malign tumors, neoplastic diseases such as cancer, in particular pancreas carcinoma, leukemia, chronic myeloic leukemia, liver cell carcinoma, colon carcinoma, pancreatic carcinoma, breast cancer, circulatory disorders, neurological disorders such as Alzheimer's disease, inherited neurodegenerative diseases such as Huntington's disease or amyotrophic lateral sclerosis, metabolic disorders, hypercholesterolemia, diabetes, atherosclerosis, genetic disorders, inflammatory disorders such as hepatitis and/or infections with parasites.

8. Use of an inhibitor as defined in claim 1 or 4 for preventing the degradation and/or the loss of activity of an oligonucleotide having RNAi activity as defined in any of claims 1 to 3 in a mammalian cell culture or a mammalian tissue culture.

9. A method for the systemic treatment of a mammal, which comprises the administration of a pharmaceutically active amount of the composition as defined in any of claims 1 to 5 to the mammal in need of such treatment.

10. A method for preventing the degradation and/or the loss of activity of an oligonucleotide having RNAi activity as defined in any of claims 1 to 3 in a mammal, a mammalian cell culture or a mammalian tissue culture, which comprises providing the inhibitor as defined in claim 1 or 4 to said mammal, mammalian cell culture or mammalian tissue culture.


SEQUENCE LISTING

<110>  Universität des Saarlandes

<120>  Use of Inhibitors of RNAse A-Family Enzymes for Stabilizing
       Oligonucleotides Having RNA Interfering Activity

<130>  050640ep

<160>  200

<170>  PatentIn version 3.3

<210>  1
<211>  21
<212>  DNA/RNA
<213>  Artificial

<220>
<223>  STAT3 siRNA sense strand

<400>  1
ugaguugaau uaucagcuut t
21


<210>  2
<211>  21
<212>  DNA/RNA
<213>  Artificial

<220>
<223>  STAT3 siRNA antisense strand

<400>  2
ttacucaacu uaauagucga a
21


<210>  3
<211>  19
<212>  RNA
<213>  Artificial

<220>
<223>  STAT3 19bp dsRNA sense strand

<400>  3
ugaguugaau uaucagcuu
19


<210>  4
<211>  19
<212>  RNA
<213>  Artificial

<220>
<223>  STAT3 19bp dsRNA antisense strand

<400>  4
acucaacuua auagucgaa
19


<210>  5
<211>  21
<212>  DNA/RNA
<213>  Artificial

<220>
<223>  Plk1 siRNA sense strand

<400>  5
agaccuaccu ccggaucaat t
21


<210>  6
<211>  21
<212>  DNA/RNA
<213>  Artificial

<220>
<223>  Plk1 siRNA antisense strand

<400>  6
ttucuggaug gaggccuagu u
21


<210>  7
<211>  21
<212>  DNA/RNA
<213>  Artificial

<220>
<223>  beta-Catenin siRNA sense strand

<400>  7
ugccguucgc cuucauuaut t
21


<210>  8
<211>  21
<212>  DNA/RNA
<213>  Artificial

<220>
<223>  beta-Catenin siRNA antisense strand

<400>  8

ttacggcaag cggaaguaau a
21


<210> 9
<211> 188
<212> PRT
<213> Homo sapiens

<400> 9

Met Thr Glu Tyr Lys Leu Val Val Val Gly Ala Cys Gly Val Gly Lys
1               5                   10                  15

Ser Ala Leu Thr Ile Gln Leu Ile Gln Asn His Phe Val Asp Glu Tyr
            20                  25                  30

Asp Pro Thr Ile Glu Asp Ser Tyr Arg Lys Gln Val Val Ile Asp Gly
        35                  40                  45

Glu Thr Cys Leu Leu Asp Ile Leu Asp Thr Ala Gly Gln Glu Glu Tyr
    50                  55                  60

Ser Ala Met Arg Asp Gln Tyr Met Arg Thr Gly Glu Gly Phe Leu Cys
65                  70                  75                  80

Val Phe Ala Ile Asn Asn Thr Lys Ser Phe Glu Asp Ile His His Tyr
                85                  90                  95

Arg Glu Gln Ile Lys Arg Val Lys Asp Ser Glu Asp Val Pro Met Val
            100                 105                 110

Leu Val Gly Asn Lys Cys Asp Leu Pro Ser Arg Thr Val Asp Thr Lys
        115                 120                 125

Gln Ala Gln Asp Leu Ala Arg Ser Tyr Gly Ile Pro Phe Ile Glu Thr
    130                 135                 140

Ser Ala Lys Thr Arg Gln Gly Val Asp Asp Ala Phe Tyr Thr Leu Val
145                 150                 155                 160

Arg Glu Ile Arg Lys His Lys Glu Lys Met Ser Lys Asp Gly Lys Lys
                165                 170                 175

Lys Lys Lys Lys Ser Lys Thr Lys Cys Val Ile Met
            180                 185

<210> 10
<211> 5775
<212> DNA
<213> Homo sapiens

<400> 10
   tcctaggcgg cggccgcggc ggcggaggca gcagcggcgg cggcagtggc ggcggcgaag
   60
   gtggcggcgg ctcggccagt actcccggcc cccgccattt cggactggga gcgagcgcgg
   120
   cgcaggcact gaaggcggcg gcggggccag aggctcagcg gctcccaggt gcgggagaga
   180
   ggcctgctga aaatgactga atataaactt gtggtagttg gagcttgtgg cgtaggcaag
   240
   agtgccttga cgatacagct aattcagaat cattttgtgg acgaatatga tccaacaata
   300
   gaggattcct acaggaagca agtagtaatt gatggagaaa cctgtctctt ggatattctc
   360
   gacacagcag gtcaagagga gtacagtgca atgagggacc agtacatgag gactggggag
   420
   ggctttcttt gtgtatttgc cataaataat actaaatcat ttgaagatat tcaccattat
   480
   agagaacaaa ttaaaagagt taaggactct gaagatgtac ctatggtcct agtaggaaat
   540
   aaatgtgatt tgccttctag aacagtagac acaaaacagg ctcaggactt agcaagaagt
   600
   tatggaattc cttttattga aacatcagca aagacaagac agggtgttga tgatgccttc
   660
   tatacattag ttcgagaaat tcgaaaacat aaagaaaaga tgagcaaaga tggtaaaaag
   720
   aagaaaaaga agtcaaagac aaagtgtgta attatgtaaa tacaatttgt acttttttct
   780
   taaggcatac tagtacaagt ggtaattttt gtacattaca ctaaattatt agcatttgtt
   840
   ttagcattac ctaatttttt tcctgctcca tgcagactgt tagcttttac cttaaatgct
   900
   tattttaaaa tgacagtgga agtttttttt tcctcgaagt gccagtattc ccagagtttt
   960
   ggtttttgaa ctagcaatgc ctgtgaaaaa gaaactgaat acctaagatt tctgtcttgg
   1020
   ggtttttggt gcatgcagtt gattacttct tatttttctt accaagtgtg aatgttggtg
   1080
   tgaaacaaat taatgaagct tttgaatcat ccctattctg tgttttatct agtcacataa
   1140
   atggattaat tactaatttc agttgagacc ttctaattgg tttttactga aacattgagg
   1200
   gacacaaatt tatgggcttc ctgatgatga ttcttctagg catcatgtcc tatagtttgt
   1260
   catccctgat gaatgtaaag ttacactgtt cacaaaggtt ttgtctcctt tccactgcta
   1320
   ttagtcatgg tcactctccc caaaatatta tattttttct ataaaaagaa aaaaatggaa
   1380
   aaaaattaca aggcaatgga aactattata aggccatttc cttttcacat tagataaatt
   1440
   actataaaga ctcctaatag ctttttcctg ttaaggcaga cccagtatga atgggattat
   1500
   tatagcaacc attttggggc tatatttaca tgctactaaa tttttataat aattgaaaag
   1560
   attttaacaa gtataaaaaa attctcatag gaattaaatg tagtctccct gtgtcagact
   1620
   gctctttcat agtataactt taaatctttt cttcaacttg agtctttgaa gatagtttta
   1680
   attctgcttg tgacattaaa agattatttg ggccagttat agcttattag gtgttgaaga
   1740
   gaccaaggtt gcaagccagg ccctgtgtga accttgagct ttcatagaga gtttcacagc
   1800
   atggactgtg tgccccacgg tcatccgagt ggttgtacga tgcattggtt agtcaaaaat
   1860
   ggggagggac tagggcagtt tggatagctc aacaagatac aatctcactc tgtggtggtc
   1920
   ctgctgacaa atcaagagca ttgcttttgt ttcttaagaa aacaaactct tttttaaaaa
   1980
   ttacttttaa atattaactc aaaagttgag attttggggt ggtggtgtgc caagacatta
   2040
   attttttttt taaacaatga agtgaaaaag ttttacaatc tctaggtttg gctagttctc
   2100
   ttaacactgg ttaaattaac attgcataaa cacttttcaa gtctgatcca tatttaataa
   2160
   tgctttaaaa taaaaataaa aacaatcctt ttgataaatt taaaatgtta cttattttaa
   2220
   aataaatgaa gtgagatggc atggtgaggt gaaagtatca ctggactagg ttgttggtga
   2280
   cttaggttct agataggtgt cttttaggac tctgattttg aggacatcac ttactatcca
   2340
   tttcttcatg ttaaaagaag tcatctcaaa ctcttagttt ttttttttta cactatgtga
   2400
   tttatattcc atttacataa ggatacactt atttgtcaag ctcagcacaa tctgtaaatt
   2460
   tttaacctat gttacaccat cttcagtgcc agtcttgggc aaaattgtgc aagaggtgaa
   2520
   gtttatattt gaatatccat tctcgtttta ggactcttct tccatattag tgtcatcttg
   2580
   cctccctacc ttccacatgc cccatgactt gatgcagttt taatacttgt aattccccta
   2640
   accataagat ttactgctgc tgtggatatc tccatgaagt tttcccactg agtcacatca
   2700
   gaaatgccct acatcttatt ttcctcaggg ctcaagagaa tctgacagat accataaagg
   2760
   gatttgacct aatcactaat tttcaggtgg tggctgatgc tttgaacatc tctttgctgc
   2820
   ccaatccatt agcgacagta ggatttttca accctggtat gaatagacag aaccctatcc
   2880
   agtggaagga gaatttaata aagatagtgc agaaagaatt ccttaggtaa tctataacta
   2940
   ggactactcc tggtaacagt aatacattcc attgttttag taaccagaaa tcttcatgca
   3000
   atgaaaaata ctttaattca tgaagcttac tttttttttt ttggtgtcag agtctcgctc
   3060
   ttgtcaccca ggctggaatg cagtggcgcc atctcagctc actgcaacct tccatcttcc
   3120
   caggttcaag cgattctcgt gcctcggcct cctgagtagc tgggattaca ggcgtgtgca
   3180
   ctacactcaa ctaatttttg tatttttagg agagacgggg tttcacctgt tggccaggct
   3240
   ggtctcgaac tcctgacctc aagtgattca cccaccttgg cctcataaac ctgttttgca
   3300
   gaactcattt attcagcaaa tatttattga gtgcctacca gatgccagtc accgcacaag
   3360
   gcactgggta tatggtatcc ccaaacaaga gacataatcc cggtccttag gtactgctag
   3420
   tgtggtctgt aatatcttac taaggccttt ggtatacgac ccagagataa cacgatgcgt
   3480
   attttagttt tgcaaagaag gggtttggtc tctgtgccag ctctataatt gttttgctac
   3540
   gattccactg aaactcttcg atcaagctac tttatgtaaa tcacttcatt gttttaaagg
   3600
   aataaacttg attatattgt tttttatttt ggcataactg tgattctttt aggacaatta
   3660
   ctgtacacat taaggtgtat gtcagatatt catattgacc caaatgtgta atattccagt
   3720
   tttctctgca taagtaatta aaatatactt aaaaattaat agttttatct gggtacaaat
   3780
   aaacagtgcc tgaactagtt cacagacaag ggaaacttct atgtaaaaat cactatgatt
   3840
   tctgaattgc tatgtgaaac tacagatctt tggaacactg tttaggtagg gtgttaagac
   3900
   ttgacacagt acctcgtttc tacacagaga aagaaatggc catacttcag gaactgcagt
   3960
   gcttatgagg ggatatttag gcctcttgaa tttttgatgt agatgggcat ttttttaagg
   4020
   tagtggttaa ttacctttat gtgaactttg aatggtttaa caaaagattt gtttttgtag
   4080
   agattttaaa gggggagaat tctagaaata aatgttacct aattattaca gccttaaaga
   4140
   caaaaatcct tgttgaagtt tttttaaaaa aagactaaat tacatagact taggcattaa
   4200
   catgtttgtg gaagaatata gcagacgtat attgtatcat ttgagtgaat gttcccaagt
   4260
   aggcattcta ggctctattt aactgagtca cactgcatag gaatttagaa cctaactttt
   4320
   ataggttatc aaaactgttg tcaccattgc acaattttgt cctaatatat acatagaaac
   4380
   tttgtggggc atgttaagtt acagtttgca caagttcatc tcatttgtat tccattgatt
   4440
   tttttttttc ttctaaacat tttttcttca aaacagtata tataactttt tttaggggat
   4500
   tttttttaga cagcaaaaaa ctatctgaag atttccattt gtcaaaaagt aatgatttct
   4560
   tgataattgt gtagtgaatg ttttttagaa cccagcagtt accttgaaag ctgaatttat
   4620
   atttagtaac ttctgtgtta atactggata gcatgaattc tgcattgaga aactgaatag
   4680
   ctgtcataaa atgctttctt tcctaaagaa agatactcac atgagttctt gaagaatagt
   4740
   cataactaga ttaagatctg tgttttagtt taatagtttg aagtgcctgt ttgggataat
   4800
   gataggtaat ttagatgaat ttaggggaaa aaaaagttat ctgcagttat gttgagggcc
   4860
   catctctccc cccacacccc cacagagcta actgggttac agtgttttat ccgaaagttt
   4920
   ccaattccac tgtcttgtgt tttcatgttg aaaatacttt tgcatttttc ctttgagtgc
   4980
   caatttctta ctagtactat ttcttaatgt aacatgttta cctggcctgt cttttaacta
   5040
   tttttgtata gtgtaaactg aaacatgcac attttgtaca ttgtgctttc ttttgtgggt
   5100
   catatgcagt gtgatccagt tgttttccat catttggttg cgctgaccta ggaatgttgg
   5160
   tcatatcaaa cattaaaaat gaccactctt ttaatgaaat taacttttaa atgtttatag
   5220
   gagtatgtgc tgtgaagtga tctaaaattt gtaatatttt tgtcatgaac tgtactactc
   5280
   ctaattattg taatgtaata aaaatagtta cagtgactat gagtgtgtat ttattcatgc
   5340
   aaatttgaac tgtttgcccc gaaatggata tggatacttt ataagccata gacactatag
   5400
   tataccagtg aatcttttat gcagcttgtt agaagtatcc ttttattttc taaaaggtgc
   5460
   tgtggatatt atgtaaaggc gtgtttgctt aaacaatttt ccatatttag aagtagatgc
   5520
   aaaacaaatc tgcctttatg acaaaaaaat aggataacat tatttattta tttcctttta
   5580
   tcaataaggt aattgataca caacaggtga cttggtttta ggcccaaagg tagcagcagc
   5640
   aacattaata atggaaataa ttgaatagtt agttatgtat gttaatgcca gtcaccagca
   5700
   ggctatttca aggtcagaag taatgactcc atacatatta tttatttcta taactacatt
   5760
   taaatcatta ccagg
   5775


<210> 11
<211> 19
<212> RNA
<213> Artificial

<220>
<223> Human K-Ras siRNA 1 sense strand

<400> 11
agagugccuu gacgauaca
19


<210> 12
<211> 19
<212> RNA
<213> Artificial

<220>
<223> Human K-Ras siRNA 2 sense strand

<400> 12
cgaauaugau ccaacaaua
19


<210> 13
<211> 19
<212> RNA
<213> Artificial

<220>
<223> Human K-Ras siRNA 3 sense strand

<400> 13
ucaccauuau agagaacaa
19


<210> 14
<211> 19

<212>  RNA
<213>  Artificial

<220>
<223>  Human K-Ras siRNA 4 sense strand

<400>  14
ggacgaauau gauccaaca
19


<210>  15
<211>  19
<212>  RNA
<213>  Artificial

<220>
<223>  Human K-Ras siRNA 5 sense strand

<400>  15
aaggcaaugg aaacuauua
19


<210>  16
<211>  189
<212>  PRT
<213>  Homo sapiens

<400>  16

<210>  17
<211>  570
<212>  DNA
<213>  Homo sapiens

<400>  17
atgacggaat ataagctggt ggtggtgggc gccggcggtg tgggcaagag tgcgctgacc
60

atccagctga tccagaacca ttttgtggac gaatacgacc ccactataga ggattcctac
120

cggaagcagg tggtcattga tggggagacg tgcctgttgg acatcctgga taccgccggc
180

caggaggagt acagcgccat gcgggaccag tacatgcgca ccggggaggg cttcctgtgt
240

gtgtttgcca tcaacaacac caagtctttt gaggacatcc accagtacag ggagcagatc
300

aaacgggtga aggactcgga tgacgtgccc atggtgctgg tggggaacaa gtgtgacctg
360

gctgcacgca ctgtggaatc tcggcaggct caggacctcg cccgaagcta cggcatcccc
420

tacatcgaga cctcggccaa gacccggcag ggagtggagg atgccttcta cacgttggtg
480

cgtgagatcc ggcagcacaa gctgcggaag ctgaaccctc ctgatgagag tggccccggc
540

tgcatgagct gcaagtgtgt gctctcctga
570

<210> 18
<211> 19
<212> RNA
<213> Artificial

<220>
<223> H-RAS siRNA 1 sense strand

<400> 18
gaguggagga ugccuucua
19


<210> 19
<211> 19
<212> RNA
<213> Artificial

<220>
<223> H-RAS siRNA 2 sense strand

<400> 19
gaggacaucc accaguaca
19


<210> 20
<211> 19
<212> RNA
<213> Artificial

<220>
<223> H-RAS siRNA 3 sense strand

<400> 20
ugcacgcacu guggaaucu
19


<210> 21
<211> 19
<212> RNA
<213> Artificial

<220>
<223> H-RAS siRNA 4 sense strand

<400> 21
ugaggacauc caccaguac
19


<210> 22
<211> 19
<212> RNA

<213>  Artificial

<220>
<223>  H-RAS siRNA 5 sense strand

<400>  22
ucuacacguu ggugcguga
19


<210>  23
<211>  191
<212>  PRT
<213>  Homo sapiens

<400>  23

Met Asn Phe Leu Leu Ser Trp Val His Trp Ser Leu Ala Leu Leu Leu
1               5                   10                  15

Tyr Leu His His Ala Lys Trp Ser Gln Ala Ala Pro Met Ala Glu Gly
            20                  25                  30

Gly Gly Gln Asn His His Glu Val Val Lys Phe Met Asp Val Tyr Gln
        35                  40                  45

Arg Ser Tyr Cys His Pro Ile Glu Thr Leu Val Asp Ile Phe Gln Glu
    50                  55                  60

Tyr Pro Asp Glu Ile Glu Tyr Ile Phe Lys Pro Ser Cys Val Pro Leu
65                  70                  75                  80

Met Arg Cys Gly Gly Cys Cys Asn Asp Glu Gly Leu Glu Cys Val Pro
                85                  90                  95

Thr Glu Glu Ser Asn Ile Thr Met Gln Ile Met Arg Ile Lys Pro His
            100                 105                 110

Gln Gly Gln His Ile Gly Glu Met Ser Phe Leu Gln His Asn Lys Cys
        115                 120                 125

Glu Cys Arg Pro Lys Lys Asp Arg Ala Arg Gln Glu Asn Pro Cys Gly
    130                 135                 140

Pro Cys Ser Glu Arg Arg Lys His Leu Phe Val Gln Asp Pro Gln Thr
145                 150                 155                 160

Cys Lys Cys Ser Cys Lys Asn Thr Asp Ser Arg Cys Lys Ala Arg Gln
                165                 170                 175

Leu Glu Leu Asn Glu Arg Thr Cys Arg Cys Asp Lys Pro Arg Arg
            180                 185                 190

<210> 24
<211> 990
<212> DNA
<213> Homo sapiens

<400> 24
cagtgtgctg gcggcccggc gcgagccggc ccggccccgg tcgggcctcc gaaaccatga
60

actttctgct gtcttgggtg cattggagcc tcgccttgct gctctacctc caccatgcca
120

agtggtccca ggctgcaccc atggcagaag gaggagggca gaatcatcac gaagtggtga
180

agttcatgga tgtctatcag cgcagctact gccatccaat cgagaccctg gtggacatct
240

tccaggagta ccctgatgag atcgagtaca tcttcaagcc atcctgtgtg cccctgatgc
300

gatgcggggg ctgctgcaat gacgagggcc tggagtgtgt gcccactgag gagtccaaca
360

tcaccatgca gattatgcgg atcaaacctc accaaggcca gcacatagga gagatgagct
420

tcctacagca caacaaatgt gaatgcagac caaagaaaga tagagcaaga caagaaaatc
480

cctgtgggcc ttgctcagag cggagaaagc atttgtttgt acaagatccg cagacgtgta
540

aatgttcctg caaaaacaca gactcgcgtt gcaaggcgag gcagcttgag ttaaacgaac
600

gtacttgcag atgtgacaag ccgaggcggt gagccgggca ggaggaagga gcctccctca
660

gggtttcggg aaccagatct ctcaccagga aagactgata cagaacgatc gatacagaaa
720

ccacgctgcc gccaccacac catcaccatc gacagaacag tccttaatcc agaaacctga
780

aatgaaggaa gaggagactc tgcgcagagc actttgggtc cggagggcga gactccggcg
840

gaagcattcc cgggcgggtg acccagcacg gtccctcttg gaattggatt cgccatttta
900

tttttcttgc tgctaaatca ccgagcccgg aagattagag agttttattt ctgggattcc
960

tgtagacaca ccgcggccgc cagcacactg
990

<210> 25
<211> 19
<212> RNA
<213> Artificial

<220>
<223> Human VEGF siRNA 1 sense strand

<400> 25
cgaggcagcu ugaguuaaa
19

<210> 26
<211> 19
<212> RNA
<213> Artificial

<220>
<223> Human VEGF siRNA 2 sense strand

<400> 26
acaucaccau gcagauuau
19

<210> 27
<211> 19
<212> RNA
<213> Artificial

<220>
<223> Human VEGF siRNA 3 sense strand

<400> 27
agaacgaucg auacagaaa
19

<210> 28
<211> 19
<212> RNA
<213> Artificial

<220>
<223> Human VEGF siRNA 4 sense strand

<400> 28
agaacgaucg auacagaaa
19

<210> 29
<211> 19
<212> RNA
<213> Artificial

<220>
<223> Human VEGF siRNA 5 sense strand

<400> 29
gaaagauaga gcaagacaa
19

<210> 30
<211> 19
<212> RNA
<213> Artificial

<220>
<223> Human VEGF siRNA 6 sense strand

<400> 30
agauccgcag acguguaaa
19

<210> 31
<211> 19
<212> RNA
<213> Artificial

<220>
<223> Human VEGF siRNA 7 sense strand

<400> 31
gauccgcaga cguguaaau
19

<210> 32
<211> 19
<212> RNA
<213> Artificial

<220>
<223> Human VEGF siRNA 8 sense strand

<400> 32
aguucaugga ugucuauca
19

<210>  33
<211>  19
<212>  RNA
<213>  Artificial

<220>
<223>  Human VEGF siRNA 9 sense strand

<400>  33
uuaugcggau caaaccuca
19


<210>  34
<211>  19
<212>  RNA
<213>  Artificial

<220>
<223>  Human VEGF siRNA 10 sense strand

<400>  34
gcuuccuaca gcacaacaa
19


<210>  35
<211>  180
<212>  PRT
<213>  Homo sapiens

<400>  35

<210> 36
<211> 1356
<212> DNA
<213> Homo sapiens

<400> 36
ttctcccgca accttccctt cgctccctcc cgtccccccc agctcctagc ctccgactcc
60

ctccccccct cacgcccgcc ctctcgcctt cgccgaacca aagtggatta attacacgct
120

ttctgtttct ctccgtgctg ttctctcccg ctgtgcgcct gcccgcctct cgctgtcctc
180

tctccccctc gccctctctt cggccccccc ctttcacgtt cactctgtct ctcccactat
240

ctctgccccc ctctatcctt gatacaacag ctgacctcat ttcccgatac cttttccccc
300

ccgaaaagta caacatctgg cccgccccag cccgaagaca gcccgtcctc cctggacaat
360

cagacgaatt ctcccccccc ccccaaaaaa aaaagccatc cccccgctct gccccgtcgc
420

acattcggcc cccgcgactc ggccagagcg gcgctggcag aggagtgtcc ggcaggaggg
480

ccaacgcccg ctgttcggtt tgcgacacgc agcagggagg tgggcggcag cgtcgccggc
540

ttccagacac caatgggaat cccaatgggg aagtcgatgc tggtgcttct caccttcttg
600

gccttcgcct cgtgctgcat tgctgcttac cgccccagtg agaccctgtg cggcggggag
660

ctggtggaca ccctccagtt cgtctgtggg gaccgcggct tctacttcag caggcccgca
720

agccgtgtga gccgtcgcag ccgtggcatc gttgaggagt gctgtttccg cagctgtgac
780

ctggccctcc tggagacgta ctgtgctacc cccgccaagt ccgagaggga cgtgtcgacc
840

cctccgaccg tgcttccgga caacttcccc agataccccg tgggcaagtt cttccaatat
900

gacacctgga agcagtccac ccagcgcctg cgcaggggcc tgcctgccct cctgcgtgcc
960

cgccggggtc acgtgctcgc caaggagctc gaggcgttca gggaggccaa acgtcaccgt
1020

cccctgattg ctctacccac ccaagacccc gcccacgggg gcgccccccc agagatggcc
1080

agcaatcgga agtgagcaaa actgccgcaa gtctgcagcc cggcgccacc atcctgcagc
1140

ctcctcctga ccacggacgt ttccatcagg ttccatcccg aaaatctctc ggttccacgt
1200

ccccctgggg cttctcctga cccagtcccc gtgccccgcc tccccgaaac aggctactct
1260

cctcggcccc ctccatcggg ctgaggaagc acagcagcat cttcaaacat gtacaaaatc
1320

gattggcttt aaacaccctt cacataccct cccccc
1356


<210> 37
<211> 19
<212> RNA
<213> Artificial

<220>
<223> Human IGF2 siRNA 1 sense strand

<400> 37
gccgaaccaa aguggauua
19


<210> 38

<211>  19
<212>  RNA
<213>  Artificial

<220>
<223>  Human IGF2 siRNA 2 sense strand

<400>  38
ccgaaccaaa guggauuaa
19


<210>  39
<211>  19
<212>  RNA
<213>  Artificial

<220>
<223>  Human IGF2 siRNA 3 sense strand

<400>  39
cgaaccaaag uggauuaau
19


<210>  40
<211>  19
<212>  RNA
<213>  Artificial

<220>
<223>  Human IGF2 siRNA 4 sense strand

<400>  40
cuggagacgu acugugcua
19


<210>  41
<211>  19
<212>  RNA
<213>  Artificial

<220>
<223>  Human IGF2 siRNA 5 sense strand

<400>  41
aaucgauugg cuuuaaaca
19


<210>  42
<211>  19
<212>  RNA
<213>  Artificial

<220>

<223>  Human IGF2 siRNA 6 sense strand

<400>  42
gcaaucggaa gugagcaaa
19


<210>  43
<211>  19
<212>  RNA
<213>  Artificial

<220>
<223>  Human IGF2 siRNA 7 sense strand

<400>  43
accacggacg uuuccauca
19


<210>  44
<211>  19
<212>  RNA
<213>  Artificial

<220>
<223>  Human IGF2 siRNA 8 sense strand

<400>  44
aaaucgauug gcuuuaaac
19


<210>  45
<211>  19
<212>  RNA
<213>  Artificial

<220>
<223>  Human IGF2 siRNA 9 sense strand

<400>  45
agauggccag caaucggaa
19


<210>  46
<211>  1210
<212>  PRT
<213>  Homo sapiens

<400>  46

<210>  47
<211>  5616
<212>  DNA
<213>  Homo sapiens

<400>  47
ccccggcgca gcgcggccgc agcagcctcc gccccccgca cggtgtgagc gcccgacgcg
60

gccgaggcgg ccggagtccc gagctagccc cggcggccgc cgccgcccag accggacgac
120

aggccacctc gtcggcgtcc gcccgagtcc ccgcctcgcc gccaacgcca caaccaccgc
180

gcacggcccc ctgactccgt ccagtattga tcgggagagc cggagcgagc tcttcgggga
240

gcagcgatgc gaccctccgg gacggccggg gcagcgctcc tggcgctgct ggctgcgctc
300

tgcccggcga gtcgggctct ggaggaaaag aaagtttgcc aaggcacgag taacaagctc
360

acgcagttgg gcacttttga agatcatttt ctcagcctcc agaggatgtt caataactgt
420

gaggtggtcc ttgggaattt ggaaattacc tatgtgcaga ggaattatga tctttccttc
480

ttaaagacca tccaggaggt ggctggttat gtcctcattg ccctcaacac agtggagcga
540

attcctttgg aaaacctgca gatcatcaga ggaaatatgt actacgaaaa ttcctatgcc
600

ttagcagtct tatctaacta tgatgcaaat aaaaccggac tgaaggagct gcccatgaga
660

aatttacagg aaatcctgca tggcgccgtg cggttcagca acaaccctgc cctgtgcaac
720

gtggagagca tccagtggcg ggacatagtc agcagtgact ttctcagcaa catgtcgatg
780

gacttccaga accacctggg cagctgccaa aagtgtgatc caagctgtcc caatgggagc
840

tgctggggtg caggagagga gaactgccag aaactgacca aaatcatctg tgcccagcag
900

tgctccgggc gctgccgtgg caagtccccc agtgactgct gccacaacca gtgtgctgca
960

ggctgcacag gcccccggga gagcgactgc ctggtctgcc gcaaattccg agacgaagcc
1020

acgtgcaagg acacctgccc cccactcatg ctctacaacc ccaccacgta ccagatggat
1080

gtgaaccccg agggcaaata cagctttggt gccacctgcg tgaagaagtg tccccgtaat
1140

tatgtggtga cagatcacgg ctcgtgcgtc cgagcctgtg gggccgacag ctatgagatg
1200

gaggaagacg gcgtccgcaa gtgtaagaag tgcgaagggc cttgccgcaa agtgtgtaac
1260

ggaataggta ttggtgaatt taaagactca ctctccataa atgctacgaa tattaaacac
1320

ttcaaaaact gcacctccat cagtggcgat ctccacatcc tgccggtggc atttaggggt
1380

gactccttca cacatactcc tcctctggat ccacaggaac tggatattct gaaaaccgta
1440

aaggaaatca cagggttttt gctgattcag gcttggcctg aaaacaggac ggacctccat
1500

gcctttgaga acctagaaat catacgcggc aggaccaagc aacatggtca gttttctctt
1560

gcagtcgtca gcctgaacat aacatccttg ggattacgct ccctcaagga gataagtgat
1620

ggagatgtga taatttcagg aaacaaaaat ttgtgctatg caaatacaat aaactggaaa
1680

aaactgtttg ggacctccgg tcagaaaacc aaaattataa gcaacagagg tgaaaacagc
1740

tgcaaggcca caggccaggt ctgccatgcc ttgtgctccc ccgagggctg ctggggcccg
1800

gagcccaggg actgcgtctc ttgccggaat gtcagccgag gcagggaatg cgtggacaag
1860

tgcaaccttc tggagggtga gccaagggag tttgtggaga actctgagtg catacagtgc
1920

cacccagagt gcctgcctca ggccatgaac atcacctgca caggacgggg accagacaac
1980

tgtatccagt gtgcccacta cattgacggc ccccactgcg tcaagacctg cccggcagga
2040

gtcatgggag aaaacaacac cctggtctgg aagtacgcag acgccggcca tgtgtgccac
2100

ctgtgccatc caaactgcac ctacggatgc actgggccag gtcttgaagg ctgtccaacg
2160

aatgggccta agatcccgtc catcgccact gggatggtgg gggccctcct cttgctgctg
2220

gtggtggccc tggggatcgg cctcttcatg cgaaggcgcc acatcgttcg gaagcgcacg
2280

ctgcggaggc tgctgcagga gagggagctt gtggagcctc ttacacccag tggagaagct
2340

cccaaccaag ctctcttgag gatcttgaag gaaactgaat tcaaaaagat caaagtgctg
2400

ggctccggtg cgttcggcac ggtgtataag ggactctgga tcccagaagg tgagaaagtt
2460

aaaattcccg tcgctatcaa ggaattaaga gaagcaacat ctccgaaagc caacaaggaa
2520

atcctcgatg aagcctacgt gatggccagc gtggacaacc cccacgtgtg ccgcctgctg
2580

ggcatctgcc tcacctccac cgtgcagctc atcacgcagc tcatgccctt cggctgcctc
2640

ctggactatg tccgggaaca caaagacaat attggctccc agtacctgct caactggtgt
2700

gtgcagatcg caaagggcat gaactacttg gaggaccgtc gcttggtgca ccgcgacctg
2760

gcagccagga acgtactggt gaaaacaccg cagcatgtca agatcacaga ttttgggctg
2820

gccaaactgc tgggtgcgga agagaaagaa taccatgcag aaggaggcaa agtgcctatc
2880

aagtggatgg cattggaatc aattttacac agaatctata cccaccagag tgatgtctgg
2940

agctacgggg tgaccgtttg ggagttgatg acctttggat ccaagccata tgacggaatc
3000

cctgccagcg agatctcctc catcctggag aaaggagaac gcctccctca gccacccata
3060

tgtaccatcg atgtctacat gatcatggtc aagtgctgga tgatagacgc agatagtcgc
3120

ccaaagttcc gtgagttgat catcgaattc tccaaaatgg cccgagaccc ccagcgctac
3180

cttgtcattc agggggatga aagaatgcat ttgccaagtc ctacagactc caacttctac
3240

cgtgccctga tggatgaaga agacatggac gacgtggtgg atgccgacga gtacctcatc
3300

ccacagcagg gcttcttcag cagcccctcc acgtcacgga ctcccctcct gagctctctg
3360

agtgcaacca gcaacaattc caccgtggct tgcattgata gaaatgggct gcaaagctgt
3420

cccatcaagg aagacagctt cttgcagcga tacagctcag accccacagg cgccttgact
3480

gaggacagca tagacgacac cttcctccca gtgcctgaat acataaacca gtccgttccc
3540

aaaaggcccg ctggctctgt gcagaatcct gtctatcaca atcagcctct gaaccccgcg
3600

cccagcagag acccacacta ccaggacccc cacagcactg cagtgggcaa ccccgagtat
3660

ctcaacactg tccagcccac ctgtgtcaac agcacattcg acagccctgc ccactgggcc
3720

cagaaaggca gccaccaaat tagcctggac aaccctgact accagcagga cttctttccc
3780

aaggaagcca agccaaatgg catctttaag ggctccacag ctgaaaatgc agaataccta
3840

agggtcgcgc cacaaagcag tgaatttatt ggagcatgac cacggaggat agtatgagcc
3900

ctaaaaatcc agactctttc gatacccagg accaagccac agcaggtcct ccatcccaac
3960

agccatgccc gcattagctc ttagacccac agactggttt tgcaacgttt acaccgacta
4020

gccaggaagt acttccacct cgggcacatt ttgggaagtt gcattccttt gtcttcaaac
4080

tgtgaagcat ttacagaaac gcatccagca agaatattgt ccctttgagc agaaatttat
4140

ctttcaaaga ggtatatttg aaaaaaaaaa aaagtatatg tgaggatttt tattgattgg
4200

ggatcttgga gtttttcatt gtcgctattg atttttactt caatgggctc ttccaacaag
4260

gaagaagctt gctggtagca cttgctaccc tgagttcatc caggcccaac tgtgagcaag
4320

gagcacaagc cacaagtctt ccagaggatg cttgattcca gtggttctgc ttcaaggctt
4380

ccactgcaaa acactaaaga tccaagaagg ccttcatggc cccagcaggc cggatcggta
4440

ctgtatcaag tcatggcagg tacagtagga taagccactc tgtcccttcc tgggcaaaga
4500

agaaacggag gggatggaat tcttccttag acttactttt gtaaaaatgt ccccacggta
4560

cttactcccc actgatggac cagtggtttc cagtcatgag cgttagactg acttgtttgt
4620

cttccattcc attgttttga aactcagtat gctgcccctg tcttgctgtc atgaaatcag
4680

caagagagga tgacacatca aataataact cggattccag cccacattgg attcatcagc
4740

atttggacca atagcccaca gctgagaatg tggaatacct aaggatagca ccgcttttgt
4800

tctcgcaaaa acgtatctcc taatttgagg ctcagatgaa atgcatcagg tcctttgggg
4860

catagatcag aagactacaa aaatgaagct gctctgaaat ctcctttagc catcacccca
4920

accccccaaa attagtttgt gttacttatg gaagatagtt ttctcctttt acttcacttc
4980

aaaagctttt tactcaaaga gtatatgttc cctccaggtc agctgccccc aaaccccctc
5040

cttacgcttt gtcacacaaa aagtgtctct gccttgagtc atctattcaa gcacttacag
5100

ctctggccac aacagggcat tttacaggtg cgaatgacag tagcattatg agtagtgtgg
5160

aattcaggta gtaaatatga aactagggtt tgaaattgat aatgctttca caacatttgc
5220

agatgtttta gaaggaaaaa agttccttcc taaaataatt tctctacaat tggaagattg
5280

gaagattcag ctagttagga gcccaccttt tttcctaatc tgtgtgtgcc ctgtaacctg
5340

actggttaac agcagtcctt tgtaaacagt gttttaaact ctcctagtca atatccaccc
5400

catccaattt atcaaggaag aaatggttca gaaaatattt tcagcctaca gttatgttca
5460

gtcacacaca catacaaaat gttccttttg cttttaaagt aatttttgac tcccagatca
5520

gtcagagccc ctacagcatt gttaagaaag tatttgattt ttgtctcaat gaaaataaaa
5580

ctatattcat ttccactcta aaaaaaaaaa aaaaaa
5616


<210>  48
<211>  19
<212>  RNA
<213>  Artificial

<220>
<223>  Human EGFR siRNA 1 sense strand

<400>  48
gcagaggaau uaugaucuu
19


<210>  49
<211>  19
<212>  RNA
<213>  Artificial

<220>
<223>  Human EGFR siRNA 2 sense strand

<400>  49
uagcagucuu aucuaacua
19


<210>  50
<211>  19
<212>  RNA
<213>  Artificial

<220>
<223>  Human EGFR siRNA 3 sense strand

<400> 50
ucagcaacau gucgaugga
19

<210> 51
<211> 19
<212> RNA
<213> Artificial

<220>
<223> Human EGFR siRNA 4 sense strand

<400> 51
ccauaaaugc uacgaauau
19

<210> 52
<211> 19
<212> RNA
<213> Artificial

<220>
<223> Human EGFR siRNA 5 sense strand

<400> 52
caagcaacau ggucaguuu
19

<210> 53
<211> 19
<212> RNA
<213> Artificial

<220>
<223> Human EGFR siRNA 6 sense strand

<400> 53
cguucggcac gguguauaa
19

<210> 54
<211> 19
<212> RNA
<213> Artificial

<220>
<223> Human EGFR siRNA 7 sense strand

<400> 54
accguggcuu gcauugaua
19

<210>  55
<211>  19
<212>  RNA
<213>  Artificial

<220>
<223>  Human EGFR siRNA 8 sense strand

<400>  55
uggcuugcau ugauagaaa
19


<210>  56
<211>  1255
<212>  PRT
<213>  Homo sapiens

<400>  56

<210>  57
<211>  4473

<212>  DNA
<213>  Homo sapiens

<400>  57
aaggggaggt aaccctggcc cctttggtcg gggccccggg cagccgcgcg ccccttccca
60

cggggccctt tactgcgccg cgcgcccggc ccccacccct cgcagcaccc cgcgccccgc
120

gccctcccag ccgggtccag ccggagccat ggggccggag ccgcagtgag caccatggag
180

ctggcggcct tgtgccgctg ggggctcctc ctcgccctct tgccccccgg agccgcgagc
240

acccaagtgt gcaccggcac agacatgaag ctgcggctcc ctgccagtcc cgagacccac
300

ctggacatgc tccgccacct ctaccagggc tgccaggtgg tgcagggaaa cctggaactc
360

acctacctgc ccaccaatgc cagcctgtcc ttcctgcagg atatccagga ggtgcagggc
420

tacgtgctca tcgctcacaa ccaagtgagg caggtcccac tgcagaggct gcggattgtg
480

cgaggcaccc agctctttga ggacaactat gccctggccg tgctagacaa tggagacccg
540

ctgaacaata ccacccctgt cacaggggcc tccccaggag gcctgcggga gctgcagctt
600

cgaagcctca cagagatctt gaaaggaggg gtcttgatcc agcggaaccc ccagctctgc
660

taccaggaca cgattttgtg gaaggacatc ttccacaaga acaaccagct ggctctcaca
720

ctgatagaca ccaaccgctc tcgggcctgc cacccctgtt ctccgatgtg taagggctcc
780

cgctgctggg gagagagttc tgaggattgt cagagcctga cgcgcactgt ctgtgccggt
840

ggctgtgccc gctgcaaggg gccactgccc actgactgct gccatgagca gtgtgctgcc
900

ggctgcacgg gccccaagca ctctgactgc ctggcctgcc tccacttcaa ccacagtggc
960

atctgtgagc tgcactgccc agccctggtc acctacaaca cagacacgtt tgagtccatg
1020

cccaatcccg agggccggta tacattcggc gccagctgtg tgactgcctg tccctacaac
1080

taccttteta cggacgtggg atcctgcacc ctcgtctgcc ccctgcacaa ccaagaggtg
1140

acagcagagg atggaacaca gcggtgtgag aagtgcagca agccctgtgc ccgagtgtgc
1200

tatggtctgg gcatggagca cttgcgagag gtgagggcag ttaccagtgc caatatccag
1260

gagtttgctg gctgcaagaa gatctttggg agcctggcat ttctgccgga gagctttgat
1320

ggggacccag cctccaacac tgccccgctc cagccagagc agctccaagt gtttgagact
1380

ctggaagaga tcacaggtta cctatacatc tcagcatggc cggacagcct gcctgacctc
1440

agcgtcttcc agaacctgca agtaatccgg ggacgaattc tgcacaatgg cgcctactcg
1500

ctgaccctgc aagggctggg catcagctgg ctggggctgc gctcactgag ggaactgggc
1560

agtggactgg ccctcatcca ccataacacc cacctctgct tcgtgcacac ggtgccctgg
1620

gaccagctct ttcggaaccc gcaccaagct ctgctccaca ctgccaaccg gccagaggac
1680

gagtgtgtgg gcgagggcct ggcctgccac cagctgtgcg cccgagggca ctgctggggt
1740

ccagggccca cccagtgtgt caactgcagc cagttccttc ggggccagga gtgcgtggag
1800

gaatgccgag tactgcaggg gctccccagg gagtatgtga atgccaggca ctgtttgccg
1860

tgccaccctg agtgtcagcc ccagaatggc tcagtgacct gttttggacc ggaggctgac
1920

cagtgtgtgg cctgtgccca ctataaggac cctcccttct gcgtggcccg ctgccccagc
1980

ggtgtgaaac ctgacctctc ctacatgccc atctggaagt ttccagatga ggagggcgca
2040

tgccagcctt gccccatcaa ctgcacccac tcctgtgtgg acctggatga caagggctgc
2100

cccgccgagc agagagccag ccctctgacg tccatcatct ctgcggtggt tggcattctg
2160

ctggtcgtgg tcttgggggt ggtctttggg atcctcatca agcgacggca gcagaagatc
2220

cggaagtaca cgatgcggag actgctgcag gaaacggagc tggtggagcc gctgacacct
2280
agcggagcga tgcccaacca ggcgcagatg cggatcctga aagagacgga gctgaggaag
2340
gtgaaggtgc ttggatctgg cgcttttggc acagtctaca agggcatctg gatccctgat
2400
ggggagaatg tgaaaattcc agtggccatc aaagtgttga gggaaaacac atcccccaaa
2460
gccaacaaag aaatcttaga cgaagcatac gtgatggctg gtgtgggctc cccatatgtc
2520
tcccgccttc tgggcatctg cctgacatcc acggtgcagc tggtgacaca gcttatgccc
2580
tatggctgcc tcttagacca tgtccgggaa aaccgcggac gcctgggctc ccaggacctg
2640
ctgaactggt gtatgcagat tgccaagggg atgagctacc tggaggatgt gcggctcgta
2700
cacagggact tggccgctcg gaacgtgctg gtcaagagtc ccaaccatgt caaaattaca
2760
gacttcgggc tggctcggct gctggacatt gacgagacag agtaccatgc agatgggggc
2820
aaggtgccca tcaagtggat ggcgctggag tccattctcc gccggcggtt cacccaccag
2880
agtgatgtgt ggagttatgg tgtgactgtg tgggagctga tgacttttgg ggccaaacct
2940
tacgatggga tcccagcccg ggagatccct gacctgctgg aaaaggggga gcggctgccc
3000
cagcccccca tctgcaccat tgatgtctac atgatcatgg tcaaatgttg gatgattgac
3060
tctgaatgtc ggccaagatt ccgggagttg gtgtctgaat tctcccgcat ggccagggac
3120
ccccagcgct ttgtggtcat ccagaatgag gacttgggcc cagccagtcc cttggacagc
3180
accttctacc gctcactgct ggaggacgat gacatggggg acctggtgga tgctgaggag
3240
tatctggtac cccagcaggg cttcttctgt ccagaccctg ccccgggcgc tgggggcatg
3300
gtccaccaca ggcaccgcag ctcatctacc aggagtggcg gtggggacct gacactaggg
3360

ctggagccct ctgaagagga ggcccccagg tctccactgg caccctccga aggggctggc
3420

tccgatgtat ttgatggtga cctgggaatg ggggcagcca aggggctgca aagcctcccc
3480

acacatgacc ccagccctct acagcggtac agtgaggacc ccacagtacc cctgccctct
3540

gagactgatg gctacgttgc ccccctgacc tgcagccccc agcctgaata tgtgaaccag
3600

ccagatgttc ggccccagcc cccttcgccc cgagagggcc ctctgcctgc tgcccgacct
3660

gctggtgcca ctctggaaag gcccaagact ctctccccag ggaagaatgg ggtcgtcaaa
3720

gacgtttttg cctttggggg tgccgtggag aaccccgagt acttgacacc ccagggagga
3780

gctgcccctc agccccaccc tcctcctgcc ttcagcccag ccttcgacaa cctctattac
3840

tgggaccagg acccaccaga gcggggggct ccacccagca ccttcaaagg gacacctacg
3900

gcagagaacc cagagtacct gggtctggac gtgccagtgt gaaccagaag gccaagtccg
3960

cagaagccct gatgtgtcct cagggagcag ggaaggcctg acttctgctg gcatcaagag
4020

gtgggagggc cctccgacca cttccagggg aacctgccat gccaggaacc tgtcctaagg
4080

aaccttcctt cctgcttgag ttcccagatg gctggaaggg gtccagcctc gttggaagag
4140

gaacagcact ggggagtctt tgtggattct gaggccctgc ccaatgagac tctagggtcc
4200

agtggatgcc acagcccagc ttggcccttt ccttccagat cctgggtact gaaagcctta
4260

gggaagctgg cctgagaggg gaagcggccc taagggagtg tctaagaaca aaagcgaccc
4320

attcagagac tgtccctgaa acctagtact gccccccatg aggaaggaac agcaatggtg
4380

tcagtatcca ggctttgtac agagtgcttt tctgtttagt ttttactttt tttgttttgt
4440

ttttttaaag atgaaataaa gacccagggg gag
4473

<210> 58
<211> 19
<212> RNA
<213> Artificial

<220>
<223> Human c-erb-B-2 siRNA 1 sense strand

<400> 58
aaauuccagu ggccaucaa
19


<210> 59
<211> 19
<212> RNA
<213> Artificial

<220>
<223> Human c-erb-B-2 siRNA 2 sense strand

<400> 59
ccuucgacaa ccucuauua
19


<210> 60
<211> 19
<212> RNA
<213> Artificial

<220>
<223> Human c-erb-B-2 siRNA 3 sense strand

<400> 60
ucaguaucca ggcuuugua
19


<210> 61
<211> 19
<212> RNA
<213> Artificial

<220>
<223> Human c-erb-B-2 siRNA 4 sense strand

<400> 61
acaacuaccu uucuacgga
19


<210> 62
<211> 19
<212> RNA

<213>  Artificial

<220>
<223>  Human c-erb-B-2 siRNA 5 sense strand

<400>  62
aauuccagug gccaucaaa
19


<210>  63
<211>  19
<212>  RNA
<213>  Artificial

<220>
<223>  Human c-erb-B-2 siRNA 6 sense strand

<400>  63
agaaaucuua gacgaagca
19


<210>  64
<211>  19
<212>  RNA
<213>  Artificial

<220>
<223>  Human c-erb-B-2 siRNA 7 sense strand

<400>  64
aaaucuuaga cgaagcaua
19


<210>  65
<211>  19
<212>  RNA
<213>  Artificial

<220>
<223>  Human c-erb-B-2 siRNA 8 sense strand

<400>  65
uuaccagugc caauaucca
19


<210>  66
<211>  19
<212>  RNA
<213>  Artificial

<220>
<223>  Human c-erb-B-2 siRNA 9 sense strand

<400>  66
aucacagguu accuauaca
19


<210>  67
<211>  433
<212>  PRT
<213>  Homo sapiens

<400>  67

Met Ala Leu Arg Val Thr Arg Asn Ser Lys Ile Asn Ala Glu Asn Lys
1               5                   10                  15

Ala Lys Ile Asn Met Ala Gly Ala Lys Arg Val Pro Thr Ala Pro Ala
            20                  25                  30

Ala Thr Ser Lys Pro Gly Leu Arg Pro Arg Thr Ala Leu Gly Asp Ile
        35                  40                  45

Gly Asn Lys Val Ser Glu Gln Leu Gln Ala Lys Met Pro Met Lys Lys
    50                  55                  60

Glu Ala Lys Pro Ser Ala Thr Gly Lys Val Ile Asp Lys Lys Leu Pro
65                  70                  75                  80

Lys Pro Leu Glu Lys Val Pro Met Leu Val Pro Val Pro Val Ser Glu
                85                  90                  95

Pro Val Pro Glu Pro Glu Pro Glu Pro Glu Pro Glu Pro Val Lys Glu
            100                 105                 110

Glu Lys Leu Ser Pro Glu Pro Ile Leu Val Asp Thr Ala Ser Pro Ser
        115                 120                 125

Pro Met Glu Thr Ser Gly Cys Ala Pro Ala Glu Glu Asp Leu Cys Gln
    130                 135                 140

Ala Phe Ser Asp Val Ile Leu Ala Val Asn Asp Val Asp Ala Glu Asp
145                 150                 155                 160

Gly Ala Asp Pro Asn Leu Cys Ser Glu Tyr Val Lys Asp Ile Tyr Ala
                165                 170                 175

Tyr Leu Arg Gln Leu Glu Glu Glu Gln Ala Val Arg Pro Lys Tyr Leu

180                 185                 190

Leu Gly Arg Glu Val Thr Gly Asn Met Arg Ala Ile Leu Ile Asp Trp
        195                 200                 205

Leu Val Gln Val Gln Met Lys Phe Arg Leu Leu Gln Glu Thr Met Tyr
    210                 215                 220

Met Thr Val Ser Ile Ile Asp Arg Phe Met Gln Asn Asn Cys Val Pro
225                 230                 235                 240

Lys Lys Met Leu Gln Leu Val Gly Val Thr Ala Met Phe Ile Ala Ser
                245                 250                 255

Lys Tyr Glu Glu Met Tyr Pro Pro Glu Ile Gly Asp Phe Ala Phe Val
            260                 265                 270

Thr Asp Asn Thr Tyr Thr Lys His Gln Ile Arg Gln Met Glu Met Lys
        275                 280                 285

Ile Leu Arg Ala Leu Asn Phe Gly Leu Gly Arg Pro Leu Pro Leu His
    290                 295                 300

Phe Leu Arg Arg Ala Ser Lys Ile Gly Glu Val Asp Val Glu Gln His
305                 310                 315                 320

Thr Leu Ala Lys Tyr Leu Met Glu Leu Thr Met Leu Asp Tyr Asp Met
                325                 330                 335

Val His Phe Pro Pro Ser Gln Ile Ala Ala Gly Ala Phe Cys Leu Ala
            340                 345                 350

Leu Lys Ile Leu Asp Asn Gly Glu Trp Thr Pro Thr Leu Gln His Tyr
        355                 360                 365

Leu Ser Tyr Thr Glu Glu Ser Leu Leu Pro Val Met Gln His Leu Ala
    370                 375                 380

Lys Asn Val Val Met Val Asn Gln Gly Leu Thr Lys His Met Thr Val
385                 390                 395                 400

Lys Asn Lys Tyr Ala Thr Ser Lys His Ala Lys Ile Ser Thr Leu Pro
                405                 410                 415

Gln Leu Asn Ser Ala Leu Val Gln Asp Leu Ala Lys Ala Val Ala Lys
            420                 425                 430

Val

<210>  68
<211>  2101
<212>  DNA
<213>  Homo sapiens

<400>  68
   acgaacaggc caataaggag ggagcagtgc ggggtttaaa tctgaggcta ggctggctct
   60
   tctcggcgtg ctgcggcgga acggctgttg gtttctgctg ggtgtaggtc cttggctggt
   120
   cgggcctccg gtgttctgct tctccccgct gagctgctgc ctggtgaaga ggaagccatg
   180
   gcgctccgag tcaccaggaa ctcgaaaatt aatgctgaaa ataaggcgaa gatcaacatg
   240
   gcaggcgcaa agcgcgttcc tacggcccct gctgcaacct ccaagcccgg actgaggcca
   300
   agaacagctc ttggggacat tggtaacaaa gtcagtgaac aactgcaggc caaaatgcct
   360
   atgaagaagg aagcaaaacc ttcagctact ggaaaagtca ttgataaaaa actaccaaaa
   420
   cctcttgaaa aggtacctat gctggtgcca gtgccagtgt ctgagccagt gccagagcca
   480
   gaacctgagc cagaacctga gcctgttaaa gaagaaaaac tttcgcctga gcctattttg
   540
   gttgatactg cctctccaag cccaatggaa acatctggat gtgcccctgc agaagaagac
   600
   ctgtgtcagg ctttctctga tgtaattctt gcagtaaatg atgtggatgc agaagatgga
   660
   gctgatccaa acctttgtag tgaatatgtg aaagatattt atgcttatct gagacaactt
   720
   gaggaagagc aagcagtcag accaaaatac ctactgggtc gggaagtcac tggaaacatg
   780
   agagccatcc taattgactg gctagtacag gttcaaatga aattcaggtt gttgcaggag
   840
   accatgtaca tgactgtctc cattattgat cggttcatgc agaataattg tgtgcccaag
   900
   aagatgctgc agctggttgg tgtcactgcc atgtttattg caagcaaata tgaagaaatg
   960
   taccctccag aaattggtga ctttgctttt gtgactgaca acacttatac taagcaccaa
   1020
   atcagacaga tggaaatgaa gattctaaga gctttaaact ttggtctggg tcggcctcta
   1080
   cctttgcact tccttcggag agcatctaag attggagagg ttgatgtcga gcaacatact
   1140
   ttggccaaat acctgatgga actaactatg ttggactatg acatggtgca ctttcctcct
   1200
   tctcaaattg cagcaggagc tttttgctta gcactgaaaa ttctggataa tggtgaatgg
   1260
   acaccaactc tacaacatta cctgtcatat actgaagaat ctcttcttcc agttatgcag
   1320
   cacctggcta agaatgtagt catggtaaat caaggactta caaagcacat gactgtcaag
   1380
   aacaagtatg ccacatcgaa gcatgctaag atcagcactc taccacagct gaattctgca
   1440
   ctagttcaag atttagccaa ggctgtggca aaggtgtaac ttgtaaactt gagttggagt
   1500
   actatattta caaataaaat tggcaccatg tgccatctgt acatattact gttgcattta
   1560
   cttttaataa agcttgtggc ccttttactt tttttatagc ttaactaatt tgaatgtggt
   1620
   tacttcctac tgtagggtag cggaaaagtt gtcttaaaag gtatggtggg gatattttta
   1680
   aaaactcctt ttggtttacc tggggatcca attgatgtat atgtttatat actgggttct
   1740
   tgttttatat acctggcttt tactttatta atatgagtta ctgaaggtga tggaggtatt
   1800
   tgaaaatttt acttccatag gacatactgc atgtaagcca agtcatggag aatctgctgc
   1860
   atagctctat tttaaagtaa aagtctacca ccgaatccct agtccccctg ttttctgttt
   1920
   cttcttgtga ttgctgccat aattctaagt tatttacttt taccactatt taagttatca
   1980
   actttagcta gtatcttcaa actttcactt tgaaaaatga gaattttata ttctaagcca
   2040
   gttttcattt tggttttgtg ttttggttaa taaaacaata ctcaaataca aaaaaaaaaa
   2100
   a
   2101


<210> 69
<211> 19
<212> RNA
<213> Artificial

<220>
<223> Human cyclin B1 (CCNB1) siRNA 1 sense strand

<400> 69
aucgguucau gcagaauaa
19


<210> 70
<211> 19
<212> RNA
<213> Artificial

<220>
<223> Human cyclin B1 (CCNB1) siRNA 2 sense strand

<400> 70
uaacuauguu ggacuauga
19


<210> 71
<211> 19
<212> RNA
<213> Artificial

<220>
<223> Human cyclin B1 (CCNB1) siRNA 3 sense strand

<400> 71
caacauuacc ugucauaua
19


<210> 72
<211> 19
<212> RNA
<213> Artificial

<220>
<223> Human cyclin B1 (CCNB1) siRNA 4 sense strand

<400>  72
agaauguagu caugguaaa
19


<210>  73
<211>  19
<212>  RNA
<213>  Artificial

<220>
<223>  Human cyclin B1 (CCNB1) siRNA 5 sense strand

<400>  73
gcaaaggugu aacuuguaa
19


<210>  74
<211>  19
<212>  RNA
<213>  Artificial

<220>
<223>  Human cyclin B1 (CCNB1) siRNA 6 sense strand

<400>  74
auguagucau gguaaauca
19


<210>  75
<211>  19
<212>  RNA
<213>  Artificial

<220>
<223>  Human cyclin B1 (CCNB1) siRNA 7 sense strand

<400>  75
gugacugaca acacuuaua
19


<210>  76
<211>  295
<212>  PRT
<213>  Homo sapiens

<400>  76

Met Glu His Gln Leu Leu Cys Cys Glu Val Glu Thr Ile Arg Arg Ala
1               5                   10                  15


Tyr Pro Asp Ala Asn Leu Leu Asn Asp Arg Val Leu Arg Ala Met Leu

            20                  25                  30

Lys Ala Glu Glu Thr Cys Ala Pro Ser Val Ser Tyr Phe Lys Cys Val
        35                  40                  45

Gln Lys Glu Val Leu Pro Ser Met Arg Lys Ile Val Ala Thr Trp Met
    50                  55                  60

Leu Glu Val Cys Glu Glu Gln Lys Cys Glu Glu Glu Val Phe Pro Leu
65                  70                  75                  80

Ala Met Asn Tyr Leu Asp Arg Phe Leu Ser Leu Glu Pro Val Lys Lys
                85                  90                  95

Ser Arg Leu Gln Leu Leu Gly Ala Thr Cys Met Phe Val Ala Ser Lys
            100                 105                 110

Met Lys Glu Thr Ile Pro Leu Thr Ala Glu Lys Leu Cys Ile Tyr Thr
        115                 120                 125

Asp Asn Ser Ile Arg Pro Glu Glu Leu Leu Gln Met Glu Leu Leu Leu
    130                 135                 140

Val Asn Lys Leu Lys Trp Asn Leu Ala Ala Met Thr Pro His Asp Phe
145                 150                 155                 160

Ile Glu His Phe Leu Ser Lys Met Pro Glu Ala Glu Glu Asn Lys Gln
                165                 170                 175

Ile Ile Arg Lys His Ala Gln Thr Phe Val Ala Leu Cys Ala Thr Asp
            180                 185                 190

Val Lys Phe Ile Ser Asn Pro Pro Ser Met Val Ala Ala Gly Ser Val
        195                 200                 205

Val Ala Ala Val Gln Gly Leu Asn Leu Arg Ser Pro Asn Asn Phe Leu
    210                 215                 220

Ser Tyr Tyr Arg Leu Thr Arg Phe Leu Ser Arg Val Ile Lys Cys Asp
225                 230                 235                 240

Pro Asp Cys Leu Arg Ala Cys Gln Glu Gln Ile Glu Ala Leu Leu Glu
                245                 250                 255

Ser Ser Leu Arg Gln Ala Gln Gln Asn Met Asp Pro Lys Ala Ala Glu
            260                 265                 270

Glu Glu Glu Glu Glu Glu Glu Glu Val Asp Leu Ala Cys Thr Pro Thr
        275                 280                 285

Asp Val Arg Asp Val Asp Ile
    290                 295

<210>  77
<211>  4306
<212>  DNA
<213>  Homo sapiens

<400>  77
cacacggact acaggggagt tttgttgaag ttgcaaagtc ctggagcctc cagagggctg
60

tcggcgcagt agcagcgagc agcagagtcc gcacgctccg gcgaggggca gaagagcgcg
120

agggagcgcg gggcagcaga agcgagagcc gagcgcggac ccagccagga cccacagccc
180

tccccagctg cccaggaaga gccccagcca tggaacacca gctcctgtgc tgcgaagtgg
240

aaaccatccg ccgcgcgtac cccgatgcca acctcctcaa cgaccgggtg ctgcgggcca
300

tgctgaaggc ggaggagacc tgcgcgccct cggtgtccta cttcaaatgt gtgcagaagg
360

aggtcctgcc gtccatgcgg aagatcgtcg ccacctggat gctggaggtc tgcgaggaac
420

agaagtgcga ggaggaggtc ttcccgctgg ccatgaacta cctggaccgc ttcctgtcgc
480

tggagcccgt gaaaaagagc cgcctgcagc tgctgggggc cacttgcatg ttcgtggcct
540

ctaagatgaa ggagaccatc cccctgacgg ccgagaagct gtgcatctac accgacaact
600

ccatccggcc cgaggagctg ctgcaaatgg agctgctcct ggtgaacaag ctcaagtgga
660

acctggccgc aatgaccccg cacgatttca ttgaacactt cctctccaaa atgccagagg
720

cggaggagaa caaacagatc atccgcaaac acgcgcagac cttcgttgcc ctctgtgcca
780

cagatgtgaa gttcatttcc aatccgccct ccatggtggc agcggggagc gtggtggccg
840

cagtgcaagg cctgaacctg aggagcccca acaacttcct gtcctactac cgcctcacac
900

gcttcctctc cagagtgatc aagtgtgacc cagactgcct ccgggcctgc caggagcaga
960

tcgaagccct gctggagtca agcctgcgcc aggcccagca gaacatggac cccaaggccg
1020

ccgaggagga ggaagaggag gaggaggagg tggacctggc ttgcacaccc accgacgtgc
1080

gggacgtgga catctgaggg cgccaggcag gcgggcgcca ccgccacccg cagcgagggc
1140

ggagccggcc ccaggtgctc ccctgacagt ccctcctctc cggagcattt tgataccaga
1200

agggaaagct tcattctcct tgttgttggt tgttttttcc tttgctcttt cccccttcca
1260

tctctgactt aagcaaaaga aaaagattac ccaaaaactg tctttaaaag agagagagag
1320

aaaaaaaaaa tagtatttgc ataaccctga gcggtggggg aggagggttg tgctacagat
1380

gatagaggat tttatacccc aataatcaac tcgtttttat attaatgtac ttgtttctct
1440

gttgtaagaa taggcattaa cacaaaggag gcgtctcggg agaggattag gttccatcct
1500

ttacgtgttt aaaaaaaagc ataaaaacat tttaaaaaca tagaaaaatt cagcaaacca
1560

tttttaaagt agaagagggt tttaggtaga aaaacatatt cttgtgcttt tcctgataaa
1620

gcacagctgt agtggggttc taggcatctc tgtactttgc ttgctcatat gcatgtagtc
1680

actttataag tcattgtatg ttattatatt ccgtaggtag atgtgtaacc tcttcacctt
1740

attcatggct gaagtcacct cttggttaca gtagcgtagc gtggccgtgt gcatgtcctt
1800

tgcgcctgtg accaccaccc caacaaacca tccagtgaca aaccatccag tggaggtttg
1860

tcgggcacca gccagcgtag cagggtcggg aaaggccacc tgtcccactc ctacgatacg
1920

ctactataaa gagaagacga aatagtgaca taatatattc tatttttata ctcttcctat
1980

ttttgtagtg acctgtttat gagatgctgg ttttctaccc aacggccctg cagccagctc
2040

acgtccaggt tcaacccaca gctacttggt ttgtgttctt cttcatattc taaaaccatt
2100

ccatttccaa gcactttcag tccaataggt gtaggaaata gcgctgtttt tgttgtgtgt
2160

gcagggaggg cagttttcta atggaatggt ttgggaatat ccatgtactt gtttgcaagc
2220

aggactttga ggcaagtgtg ggccactgtg gtggcagtgg aggtggggtg tttgggaggc
2280

tgcgtgccag tcaagaagaa aaaggtttgc attctcacat tgccaggatg ataagttcct
2340

ttccttttct ttaaagaagt tgaagtttag gaatcctttg gtgccaactg gtgtttgaaa
2400

gtagggacct cagaggttta cctagagaac aggtggtttt taagggttat cttagatgtt
2460

tcacaccgga aggtttttaa acactaaaat atataattta tagttaaggc taaaaagtat
2520

atttattgca gaggatgttc ataaggccag tatgatttat aaatgcaatc tccccttgat
2580

ttaaacacac agatacacac acacacacac acacacacac aaaccttctg cctttgatgt
2640

tacagattta atacagttta tttttaaaga tagatccttt tataggtgag aaaaaaacaa
2700

tctggaagaa aaaaaccaca caaagacatt gattcagcct gtttggcgtt tcccagagtc
2760

atctgattgg acaggcatgg gtgcaaggaa aattagggta ctcaacctaa gttcggttcc
2820

gatgaattct tatcccctgc cccttccttt aaaaaactta gtgacaaaat agacaatttg
2880

cacatcttgg ctatgtaatt cttgtaattt ttatttagga agtgttgaag ggaggtggca
2940

agagtgtgga ggctgacgtg tgagggagga caggcgggag gaggtgtgag gaggaggctc
3000

ccgaggggaa ggggcggtgc ccacaccggg gacaggccgc agctccattt tcttattgcg
3060

ctgctaccgt tgacttccag gcacggtttg gaaatattca catcgcttct gtgtatctct
3120

ttcacattgt ttgctgctat tggaggatca gttttttgtt ttacaatgtc atatactgcc
3180

atgtactagt tttagttttc tcttagaaca ttgtattaca gatgcctttt ttgtagtttt
3240

tttttttttt atgtgatcaa ttttgactta atgtgattac tgctctattc caaaaaggtt
3300

gctgtttcac aatacctcat gcttcactta gccatggtgg acccagcggg caggttctgc
3360

ctgctttggc gggcagacac gcgggcgcga tcccacacag gctggcgggg gccggccccg
3420

aggccgcgtg cgtgagaacc gcgccggtgt ccccagagac caggctgtgt ccctcttctc
3480

ttccctgcgc ctgtgatgct gggcacttca tctgatcggg ggcgtagcat catagtagtt
3540

tttacagctg tgttattctt tgcgtgtagc tatggaagtt gcataattat tattattatt
3600

attataacaa gtgtgtctta cgtgccacca cggcgttgta cctgtaggac tctcattcgg
3660

gatgattgga atagcttctg gaatttgttc aagttttggg tatgtttaat ctgttatgta
3720

ctagtgttct gtttgttatt gttttgttaa ttacaccata atgctaattt aaagagactc
3780

caaatctcaa tgaagccagc tcacagtgct gtgtgccccg gtcacctagc aagctgccga
3840

accaaaagaa tttgcacccc gctgcgggcc cacgtggttg gggccctgcc ctggcagggt
3900

catcctgtgc tcggaggcca tctcgggcac aggcccaccc cgccccaccc ctccagaaca
3960

cggctcacgc ttacctcaac catcctggct gcggcgtctg tctgaaccac gcgggggcct
4020

tgagggacgc tttgtctgtc gtgatggggc aagggcacaa gtcctggatg ttgtgtgtat
4080

cgagaggcca aaggctggtg gcaagtgcac ggggcacagc ggagtctgtc ctgtgacgcg
4140

caagtctgag ggtctgggcg gcgggcggct gggtctgtgc atttctggtt gcaccgcggc
4200

gcttcccagc accaacatgt aaccggcatg tttccagcag aagacaaaaa gacaaacatg
4260

aaagtctaga aataaaactg gtaaaacccc aaaaaaaaaa aaaaaa
4306

<210> 78
<211> 19
<212> RNA
<213> Artificial

<220>
<223> Human cyclin D1 siRNA 1 sense strand

<400> 78
ugcauguagu cacuuuaua
19

<210> 79
<211> 19
<212> RNA
<213> Artificial

<220>
<223> Human cyclin D1 siRNA 2 sense strand

<400> 79
gcauguaguc acuuuauaa
19

<210> 80
<211> 19
<212> RNA
<213> Artificial

<220>
<223> Human cyclin D1 siRNA 3 sense strand

<400> 80
cuacgauacg cuacuauaa
19

<210> 81
<211> 19
<212> RNA
<213> Artificial

<220>
<223> Human cyclin D1 siRNA 4 sense strand

<400> 81
caaaccaucc agugacaaa
19

<210> 82
<211> 19
<212> RNA
<213> Artificial

<220>
<223> Human cyclin D1 siRNA 5 sense strand

<400> 82
uacgauacgc uacuauaaa
19

<210> 83
<211> 19
<212> RNA
<213> Artificial

<220>
<223> Human cyclin D1 siRNA 6 sense strand

<400> 83
auacgcuacu auaaagaga
19

<210> 84
<211> 19
<212> RNA
<213> Artificial

<220>
<223> Human cyclin D1 siRNA 7 sense strand

<400> 84
acacacacac acacacaaa
19

<210> 85
<211> 19
<212> RNA
<213> Artificial

<220>
<223> Human cyclin D1 siRNA 8 sense strand

<400> 85
uaaguucggu uccgaugaa
19

<210> 86
<211> 19
<212> RNA
<213> Artificial

<220>
<223> Human cyclin D1 siRNA 9 sense strand

<400> 86
cuauggaagu ugcauaauu
19


<210> 87
<211> 239
<212> PRT
<213> Homo sapiens

<400> 87

Met Ala His Ala Gly Arg Thr Gly Tyr Asp Asn Arg Glu Ile Val Met
1               5                   10                  15

Lys Tyr Ile His Tyr Lys Leu Ser Gln Arg Gly Tyr Glu Trp Asp Ala
            20                  25                  30

Gly Asp Val Gly Ala Ala Pro Pro Gly Ala Ala Pro Ala Pro Gly Ile
        35                  40                  45

Phe Ser Ser Gln Pro Gly His Thr Pro His Thr Ala Ala Ser Arg Asp
    50                  55                  60

Pro Val Ala Arg Thr Ser Pro Leu Gln Thr Pro Ala Ala Pro Gly Ala
65                  70                  75                  80

Ala Ala Gly Pro Ala Leu Ser Pro Val Pro Pro Val Val His Leu Thr
                85                  90                  95

Leu Arg Gln Ala Gly Asp Asp Phe Ser Arg Arg Tyr Arg Arg Asp Phe
            100                 105                 110

Ala Glu Met Ser Arg Gln Leu His Leu Thr Pro Phe Thr Ala Arg Gly
        115                 120                 125

Arg Phe Ala Thr Val Val Glu Glu Leu Phe Arg Asp Gly Val Asn Trp
    130                 135                 140

Gly Arg Ile Val Ala Phe Phe Glu Phe Gly Gly Val Met Cys Val Glu

145                 150                 155                 160

Ser Val Asn Arg Glu Met Ser Pro Leu Val Asp Asn Ile Ala Leu Trp
                165                 170                 175

Met Thr Glu Tyr Leu Asn Arg His Leu His Thr Trp Ile Gln Asp Asn
            180                 185                 190

Gly Gly Trp Asp Ala Phe Val Glu Leu Tyr Gly Pro Ser Met Arg Pro
        195                 200                 205

Leu Phe Asp Phe Ser Trp Leu Ser Leu Lys Thr Leu Leu Ser Leu Ala
    210                 215                 220

Leu Val Gly Ala Cys Ile Thr Leu Gly Ala Tyr Leu Gly His Lys
225                 230                 235

<210>  88
<211>  6030
<212>  DNA
<213>  Homo sapiens

<400>  88
gttggccccc gttacttttc ctctgggaaa tatggcgcac gctgggagaa cagggtacga
60

taaccgggag atagtgatga agtacatcca ttataagctg tcgcagaggg gctacgagtg
120

ggatgcggga gatgtgggcg ccgcgccccc gggggccgcc cccgcgccgg gcatcttctc
180

ctcgcagccc gggcacacgc cccatacagc cgcatcccgg gacccggtcg ccaggacctc
240

gccgctgcag accccggctg cccccggcgc cgccgcgggg cctgcgctca gcccggtgcc
300

acctgtggtc cacctgaccc tccgccaggc cggcgacgac ttctcccgcc gctaccgccg
360

cgacttcgcc gagatgtcca ggcagctgca cctgacgccc ttcaccgcgc ggggacgctt
420

tgccacggtg gtggaggagc tcttcaggga cggggtgaac tgggggagga ttgtggcctt
480

ctttgagttc ggtggggtca tgtgtgtgga gagcgtcaac cgggagatgt cgcccctggt
540

ggacaacatc gccctgtgga tgactgagta cctgaaccgg cacctgcaca cctggatcca
600

ggataacgga ggctgggatg cctttgtgga actgtacggc cccagcatgc ggcctctgtt
660

tgatttctcc tggctgtctc tgaagactct gctcagtttg gccctggtgg gagcttgcat
720

caccctgggt gcctatctgg gccacaagtg aagtcaacat gcctgcccca aacaaatatg
780

caaaaggttc actaaagcag tagaaataat atgcattgtc agtgatgttc catgaaacaa
840

agctgcaggc tgtttaagaa aaaataacac acatataaac atcacacaca cagacagaca
900

cacacacaca caacaattaa cagtcttcag gcaaaacgtc gaatcagcta tttactgcca
960

aagggaaata tcatttattt tttacattat taagaaaaaa agatttattt atttaagaca
1020

gtcccatcaa aactcctgtc tttggaaatc cgaccactaa ttgccaagca ccgcttcgtg
1080

tggctccacc tggatgttct gtgcctgtaa acatagattc gctttccatg ttgttggccg
1140

gatcaccatc tgaagagcag acggatggaa aaaggacctg atcattgggg aagctggctt
1200

tctggctgct ggaggctggg gagaaggtgt tcattcactt gcatttcttt gccctggggg
1260

ctgtgatatt aacagaggga gggttcctgt ggggggaagt ccatgcctcc ctggcctgaa
1320

gaagagactc tttgcatatg actcacatga tgcatacctg gtgggaggaa aagagttggg
1380

aacttcagat ggacctagta cccactgaga tttccacgcc gaaggacagc gatgggaaaa
1440

atgcccttaa atcataggaa agtatttttt taagctacca attgtgccga gaaaagcatt
1500

ttagcaattt atacaatatc atccagtacc ttaagccctg attgtgtata ttcatatatt
1560

ttggatacgc accccccaac tcccaatact ggctctgtct gagtaagaaa cagaatcctc
1620

tggaacttga ggaagtgaac atttcggtga cttccgcatc aggaaggcta gagttaccca
1680

gagcatcagg ccgccacaag tgcctgcttt taggagaccg aagtccgcag aacctgcctg
1740

tgtcccagct tggaggcctg gtcctggaac tgagccgggg ccctcactgg cctcctccag
1800

ggatgatcaa cagggcagtg tggtctccga atgtctggaa gctgatggag ctcagaattc
1860

cactgtcaag aaagagcagt agaggggtgt ggctgggcct gtcaccctgg ggccctccag
1920

gtaggcccgt tttcacgtgg agcatgggag ccacgaccct tcttaagaca tgtatcactg
1980

tagagggaag gaacagaggc cctgggccct tcctatcaga aggacatggt gaaggctggg
2040

aacgtgagga gaggcaatgg ccacggccca ttttggctgt agcacatggc acgttggctg
2100

tgtggccttg gcccacctgt gagtttaaag caaggcttta aatgactttg gagagggtca
2160

caaatcctaa aagaagcatt gaagtgaggt gtcatggatt aattgacccc tgtctatgga
2220

attacatgta aaacattatc ttgtcactgt agtttggttt tatttgaaaa cctgacaaaa
2280

aaaaagttcc aggtgtggaa tatgggggtt atctgtacat cctggggcat taaaaaaaaa
2340

atcaatggtg gggaactata aagaagtaac aaaagaagtg acatcttcag caaataaact
2400

aggaaatttt tttttcttcc agtttagaat cagccttgaa acattgatgg aataactctg
2460

tggcattatt gcattatata ccatttatct gtattaactt tggaatgtac tctgttcaat
2520

gtttaatgct gtggttgata tttcgaaagc tgctttaaaa aaatacatgc atctcagcgt
2580

tttttgtttt ttaattgtat ttagttatgg cctatacact atttgtgagc aaaggtgatc
2640

gttttctgtt tgagattttt atctcttgat tcttcaaaag cattctgaga aggtgagata
2700

agccctgagt ctcagctacc taagaaaaac ctggatgtca ctggccactg aggagctttg
2760

tttcaaccaa gtcatgtgca tttccacgtc aacagaattg tttattgtga cagttatatc
2820

tgttgtccct ttgaccttgt ttcttgaagg tttcctcgtc cctgggcaat tccgcattta
2880

attcatggta ttcaggatta catgcatgtt tggttaaacc catgagattc attcagttaa
2940

aaatccagat ggcaaatgac cagcagattc aaatctatgg tggtttgacc tttagagagt
3000

tgctttacgt ggcctgtttc aacacagacc cacccagagc cctcctgccc tccttccgcg
3060

ggggctttct catggctgtc cttcagggtc ttcctgaaat gcagtggtgc ttacgctcca
3120

ccaagaaagc aggaaacctg tggtatgaag ccagacctcc ccggcgggcc tcagggaaca
3180

gaatgatcag acctttgaat gattctaatt tttaagcaaa atattatttt atgaaaggtt
3240

tacattgtca aagtgatgaa tatggaatat ccaatcctgt gctgctatcc tgccaaaatc
3300

attttaatgg agtcagtttg cagtatgctc cacgtggtaa gatcctccaa gctgctttag
3360

aagtaacaat gaagaacgtg gacgctttta atataaagcc tgttttgtct tctgttgttg
3420

ttcaaacggg attcacagag tatttgaaaa atgtatatat attaagaggt cacgggggct
3480

aattgctggc tggctgcctt ttgctgtggg gttttgttac ctggttttaa taacagtaaa
3540

tgtgcccagc ctcttggccc cagaactgta cagtattgtg gctgcacttg ctctaagagt
3600

agttgatgtt gcattttcct tattgttaaa aacatgttag aagcaatgaa tgtatataaa
3660

agcctcaact agtcattttt ttctcctctt cttttttttc attatatcta attattttgc
3720

agttgggcaa cagagaacca tccctatttt gtattgaaga gggattcaca tctgcatctt
3780

aactgctctt tatgaatgaa aaaacagtcc tctgtatgta ctcctcttta cactggccag
3840

ggtcagagtt aaatagagta tatgcacttt ccaaattggg gacaagggct ctaaaaaaag
3900

ccccaaaagg agaagaacat ctgagaacct cctcggccct cccagtccct cgctgcacaa
3960

atactccgca agagaggcca gaatgacagc tgacagggtc tatggccatc gggtcgtctc
4020

cgaagatttg gcaggggcag aaaactctgg caggcttaag atttggaata aagtcacaga
4080

atcaaggaag cacctcaatt tagttcaaac aagacgccaa cattctctcc acagctcact
4140

tacctctctg tgttcagatg tggccttcca tttatatgtg atctttgttt tattagtaaa
4200

tgcttatcat ctaaagatgt agctctggcc cagtgggaaa aattaggaag tgattataaa
4260

tcgagaggag ttataataat caagattaaa tgtaaataat cagggcaatc ccaacacatg
4320

tctagctttc acctccagga tctattgagt gaacagaatt gcaaatagtc tctatttgta
4380

attgaactta tcctaaaaca aatagtttat aaatgtgaac ttaaactcta attaattcca
4440

actgtacttt taaggcagtg gctgttttta gactttctta tcacttatag ttagtaatgt
4500

acacctactc tatcagagaa aaacaggaaa ggctcgaaat acaagccatt ctaaggaaat
4560

tagggagtca gttgaaattc tattctgatc ttattctgtg gtgtcttttg cagcccagac
4620

aaatgtggtt acacactttt taagaaatac aattctacat tgtcaagctt atgaaggttc
4680

caatcagatc tttattgtta ttcaatttgg atctttcagg gatttttttt ttaaattatt
4740

atgggacaaa ggacatttgt tggaggggtg ggagggagga acaattttta aatataaaac
4800

attcccaagt ttggatcagg gagttggaag ttttcagaat aaccagaact aagggtatga
4860

aggacctgta ttggggtcga tgtgatgcct ctgcgaagaa ccttgtgtga caaatgagaa
4920

acattttgaa gtttgtggta cgacctttag attccagaga catcagcatg gctcaaagtg
4980

cagctccgtt tggcagtgca atggtataaa tttcaagctg gatatgtcta atgggtattt
5040

aaacaataaa tgtgcagttt taactaacag gatatttaat gacaaccttc tggttggtag
5100

ggacatctgt ttctaaatgt ttattatgta caatacagaa aaaaatttta taaaattaag
5160

caatgtgaaa ctgaattgga gagtgataat acaagtcctt tagtcttacc cagtgaatca
5220

ttctgttcca tgtctttgga caaccatgac cttggacaat catgaaatat gcatctcact
5280

ggatgcaaag aaaatcagat ggagcatgaa tggtactgta ccggttcatc tggactgccc
5340

cagaaaaata acttcaagca aacatcctat caacaacaag gttgttctgc ataccaagct
5400

gagcacagaa gatgggaaca ctggtggagg atggaaaggc tcgctcaatc aagaaaattc
5460

tgagactatt aataaataag actgtagtgt agatactgag taaatccatg cacctaaacc
5520

ttttggaaaa tctgccgtgg gccctccaga tagctcattt cattaagttt ttccctccaa
5580

ggtagaattt gcaagagtga cagtggattg catttctttt ggggaagctt tcttttggtg
5640

gttttgttta ttataccttc ttaagttttc aaccaaggtt tgcttttgtt ttgagttact
5700

ggggttattt ttgttttaaa taaaaataag tgtacaataa gtgtttttgt attgaaagct
5760

tttgttatca agattttcat acttttacct tccatggctc tttttaagat tgatactttt
5820

aagaggtggc tgatattctg caacactgta cacataaaaa atacggtaag gatactttac
5880

atggttaagg taaagtaagt ctccagttgg ccaccattag ctataatggc actttgtttg
5940

tgttgttgga aaaagtcaca ttgccattaa actttccttg tctgtctagt taatattgtg
6000

aagaaaaata aagtacagtg tgagatactg
6030


<210> 89
<211> 19
<212> RNA
<213> Artificial

<220>
<223> Human bcl-2 siRNA 1 sense strand

<400>  89
aaauccagau ggcaaauga
19


<210>  90
<211>  19
<212>  RNA
<213>  Artificial

<220>
<223>  Human bcl-2 siRNA 2 sense strand

<400>  90
gcaccucaau uuaguucaa
19


<210>  91
<211>  19
<212>  RNA
<213>  Artificial

<220>
<223>  Human bcl-2 siRNA 3 sense strand

<400>  91
auguggccuu ccauuuaua
19


<210>  92
<211>  19
<212>  RNA
<213>  Artificial

<220>
<223>  Human bcl-2 siRNA 4 sense strand

<400>  92
uaaaucgaga ggaguuaua
19


<210>  93
<211>  19
<212>  RNA
<213>  Artificial

<220>
<223>  Human bcl-2 siRNA 5 sense strand

<400>  93
agagacucuu ugcauauga
19

<210> 94
<211> 19
<212> RNA
<213> Artificial

<220>
<223> Human bcl-2 siRNA 6 sense strand

<400> 94
uaccaauugu gccgagaaa
19


<210> 95
<211> 19
<212> RNA
<213> Artificial

<220>
<223> Human bcl-2 siRNA 7 sense strand

<400> 95
acacacacac acacaacaa
19


<210> 96
<211> 19
<212> RNA
<213> Artificial

<220>
<223> Human bcl-2 siRNA 8 sense strand

<400> 96
cacacacaca caacaauua
19


<210> 97
<211> 19
<212> RNA
<213> Artificial

<220>
<223> Human bcl-2 siRNA 9 sense strand

<400> 97
uuaacagucu ucaggcaaa
19


<210> 98
<211> 19
<212> RNA
<213> Artificial

<220>
<223>  Human bcl-2 siRNA 10 sense strand

<400>  98
acgucgaauc agcuauuua
19


<210>  99
<211>  830
<212>  PRT
<213>  Homo sapiens

<400>  99

Met Gly Leu Gly Leu Leu Leu Pro Leu Leu Leu Leu Trp Thr Arg Gly
1               5                   10                  15

Thr Gln Gly Ser Glu Leu Asp Pro Lys Gly Gln His Val Cys Val Ala
            20                  25                  30

Ser Ser Pro Ser Ala Glu Leu Gln Cys Cys Ala Gly Trp Arg Gln Lys
        35                  40                  45

Asp Gln Glu Cys Thr Ile Pro Ile Cys Glu Gly Pro Asp Ala Cys Gln
    50                  55                  60

Lys Asp Glu Val Cys Val Lys Pro Gly Leu Cys Arg Cys Lys Pro Gly
65                  70                  75                  80

Phe Phe Gly Ala His Cys Ser Ser Arg Cys Pro Gly Gln Tyr Trp Gly
                85                  90                  95

Pro Asp Cys Arg Glu Ser Cys Pro Cys His Pro His Gly Gln Cys Glu
            100                 105                 110

Pro Ala Thr Gly Ala Cys Gln Cys Gln Ala Asp Arg Trp Gly Ala Arg
        115                 120                 125

Cys Glu Phe Pro Cys Ala Cys Gly Pro His Gly Arg Cys Asp Pro Ala
    130                 135                 140

Thr Gly Val Cys His Cys Glu Pro Gly Trp Trp Ser Ser Thr Cys Arg
145                 150                 155                 160

Arg Pro Cys Gln Cys Asn Thr Ala Ala Ala Arg Cys Glu Gln Ala Thr
                165                 170                 175

Gly Ala Cys Val Cys Lys Pro Gly Trp Trp Gly Arg Arg Cys Ser Phe
            180                 185                 190

Arg Cys Asn Cys His Gly Ser Pro Cys Glu Gln Asp Ser Gly Arg Cys
        195                 200                 205

Ala Cys Arg Pro Gly Trp Trp Gly Pro Glu Cys Gln Gln Gln Cys Glu
    210                 215                 220

Cys Val Arg Gly Arg Cys Ser Ala Ala Ser Gly Glu Cys Thr Cys Pro
225                 230                 235                 240

Pro Gly Phe Arg Gly Ala Arg Cys Glu Leu Pro Cys Pro Ala Gly Ser
                245                 250                 255

His Gly Val Gln Cys Ala His Ser Cys Gly Arg Cys Lys His Asn Glu
            260                 265                 270

Pro Cys Ser Pro Asp Thr Gly Ser Cys Glu Ser Cys Glu Pro Gly Trp
        275                 280                 285

Asn Gly Thr Gln Cys Gln Gln Pro Cys Leu Pro Gly Thr Phe Gly Glu
    290                 295                 300

Ser Cys Glu Gln Gln Cys Pro His Cys Arg His Gly Glu Ala Cys Glu
305                 310                 315                 320

Pro Asp Thr Gly His Cys Gln Arg Cys Asp Pro Gly Trp Leu Gly Pro
                325                 330                 335

Arg Cys Glu Asp Pro Cys Pro Thr Gly Thr Phe Gly Glu Asp Cys Gly
            340                 345                 350

Ser Thr Cys Pro Thr Cys Val Gln Gly Ser Cys Asp Thr Val Thr Gly
        355                 360                 365

Asp Cys Val Cys Ser Ala Gly Tyr Trp Gly Pro Ser Cys Asn Ala Ser
    370                 375                 380

Cys Pro Ala Gly Phe His Gly Asn Asn Cys Ser Val Pro Cys Glu Cys
385                 390                 395                 400

Pro Glu Gly Leu Cys His Pro Val Ser Gly Ser Cys Gln Pro Gly Ser
                405                 410                 415

Gly Ser Arg Asp Thr Ala Leu Ile Val Gly Ser Leu Val Pro Leu Leu
            420                 425                 430

Leu Leu Phe Leu Gly Leu Ala Cys Cys Ala Cys Cys Cys Trp Ala Pro
        435                 440                 445

Arg Ser Asp Leu Lys Asp Arg Pro Ala Arg Asp Gly Ala Thr Val Ser
    450                 455                 460

Arg Met Lys Leu Gln Val Trp Gly Thr Leu Thr Ser Leu Gly Ser Thr
465                 470                 475                 480

Leu Pro Cys Arg Ser Leu Ser Ser His Lys Leu Pro Trp Val Thr Val
                485                 490                 495

Ser His His Asp Pro Glu Val Pro Phe Asn His Ser Phe Ile Glu Pro
            500                 505                 510

Pro Ser Ala Gly Trp Ala Thr Asp Asp Ser Phe Ser Ser Asp Pro Glu
        515                 520                 525

Ser Gly Glu Ala Asp Glu Val Pro Ala Tyr Cys Val Pro Pro Gln Glu
    530                 535                 540

Gly Met Val Pro Val Ala Gln Ala Gly Ser Ser Glu Ala Ser Leu Ala
545                 550                 555                 560

Ala Gly Ala Phe Pro Pro Pro Glu Asp Ala Ser Thr Pro Phe Ala Ile
                565                 570                 575

Pro Arg Thr Ser Ser Leu Ala Arg Ala Lys Arg Pro Ser Val Ser Phe
            580                 585                 590

Ala Glu Gly Thr Lys Phe Ala Pro Gln Ser Arg Arg Ser Ser Gly Glu
        595                 600                 605

Leu Ser Ser Pro Leu Arg Lys Pro Lys Arg Leu Ser Arg Gly Ala Gln
    610                 615                 620

Ser Gly Pro Glu Gly Arg Glu Ala Glu Glu Ser Thr Gly Pro Asp Glu
625                 630                 635                 640

Ala Glu Ala Pro Glu Ser Phe Pro Ala Ala Ala Ser Pro Gly Asp Ser
                645                 650                 655

Ala Thr Gly His Arg Arg Pro Pro Leu Gly Ser Arg Thr Val Ala Glu
            660                 665                 670

His Val Glu Ala Ile Glu Gly Ser Val Gln Glu Ser Ser Gly Pro Val
        675                 680                 685

Thr Thr Ile Tyr Met Leu Ala Gly Lys Pro Arg Gly Ser Glu Gly Pro
    690                 695                 700

Val Arg Ser Val Phe Arg His Phe Gly Ser Phe Gln Lys Gly Gln Ala
705                 710                 715                 720

Glu Ala Lys Val Lys Arg Ala Ile Pro Lys Pro Pro Arg Gln Ala Leu
                725                 730                 735

Asn Arg Lys Lys Gly Ser Pro Gly Leu Ala Ser Gly Ser Val Gly Gln
            740                 745                 750

Ser Pro Asn Ser Ala Pro Lys Ala Gly Leu Pro Gly Ala Thr Gly Pro
        755                 760                 765

Met Ala Val Arg Pro Glu Glu Ala Val Arg Gly Leu Gly Ala Gly Thr
    770                 775                 780

Glu Ser Ser Arg Arg Ala Gln Glu Pro Val Ser Gly Cys Gly Ser Pro
785                 790                 795                 800

Glu Gln Asp Pro Gln Lys Gln Ala Glu Glu Glu Arg Gln Glu Glu Pro
                805                 810                 815

Glu Tyr Glu Asn Val Val Pro Ile Ser Arg Pro Pro Glu Pro
            820                 825                 830

<210>  100
<211>  3408
<212>  DNA
<213>  Homo sapiens

<400> 100
cgagcgctcc caccgagctg cctgccatgg ggctggggct gctgctcccg ctgctgctgc
60

tctggactcg gggggactcag gggtccgagc tggaccccaa agggcagcac gtctgtgtgg
120

ccagcagccc ctctgctgag ctgcagtgct gcgcaggctg gaggcagaag gatcaagaat
180

gcaccatccc catctgtgag gggccggacg cctgccagaa agacgaggtg tgtgtgaagc
240

cgggcctctg tcgatgcaag cctggattct ttgggcccca ctgcagctcc cgctgcccgg
300

gccagtactg gggccccgac tgccgtgaga gctgcccctg ccacccgcac ggccagtgcg
360

agccagccac gggcgcgtgc cagtgccagg ccgaccgctg gggagcccgc tgcgagttcc
420

cgtgcgcctg cggcccccac gggcgctgcg accccgcgac cggcgtgtgc cactgcgaac
480

ccggctggtg gtcgtccacg tgccgccgcc cgtgccagtg caacaccgcg gcggcgcgct
540

gcgagcaggc cacgggcgcc tgcgtgtgca agccgggctg gtgggggcgc cgctgcagct
600

tccgctgcaa ctgccacggc tccccgtgcg agcaggactc cggccgctgc gcctgccggc
660

cgggctggtg gggtcccgaa tgccagcagc agtgcgagtg tgtgcggggc cgctgcagcg
720

ccgcctccgg cgagtgcacc tgcccgcccg gcttccgcgg agcgcgctgc gagctgccct
780

gcccggcagg cagccacggg gtgcagtgcg cacacagctg tggccgctgc aaacacaatg
840

agccgtgctc tccagacaca ggcagctgtg agtcctgcga gccgggctgg aacggaccc
900

agtgccagca gccctgcctg cctggcacct ttggcgagag ctgcgaacag cagtgccctc
960

actgccgaca tggggaggcc tgtgagccag atactggcca ctgtcagcgc tgtgaccctg
1020

gctggctggg gcccaggtgt gaagacccct gccccactgg tacctttggg gaagactgtg
1080

gctctacctg ccccacctgt gttcaggggt cctgtgatac tgtgacaggg gactgtgtct
1140

gcagtgccgg ctactggggg cccagctgca acgcctcctg cccagccggt ttccatggaa
1200

acaactgctc agttccttgt gaatgcccag agggactctg ccaccctgtc tctgggtcct
1260

gccagccagg ctctggcagt cgggacactg ccctcatcgt gggcagcctt gtgcctctgc
1320

tgctgctctt cctgggtctt gcctgctgtg cctgctgctg ctgggccccc cgatcagacc
1380

tcaaggacag gccagcgaga gatggagcta ccgtgtccag gatgaagctg caggtctggg
1440

ggacactgac cagcttgggc tccacgctgc cctgccgttc cctcagctcc cacaagctac
1500

cctgggtgac agtctcacat cacgacccgg aggtcccctt caaccacagc ttcatcgagc
1560

cgccctctgc cggctgggcc actgatgact ccttctcatc cgatcctgag tctggagagg
1620

cagatgaggt tcctgcctac tgtgtgccac cccaagaagg gatggtccct gtggcccagg
1680

cagggtcgtc agaggccagc ctggctgcag gtgctttccc gccccctgag gacgcctcca
1740

cgccattcgc catcccgcgc acctccagcc tagctcgggc caagcggcca tcggtctcct
1800

tcgcggaagg taccaagttt gcaccacaga gtcgccgaag ctcaggggag ctctccagcc
1860

cgctccgaaa gcccaagagg ctctcccggg gggcgcagtc gggtcctgag ggccgggaag
1920

ccgaagagtc cacaggccca gacgaagcag aagcccccga gtcctttccg gcggctgcca
1980

gtcccgggga ttcagccact ggccaccggc ggcccccact tggtagccgg acagtggctg
2040

agcacgtgga agccattgag ggcagcgtcc aggagagctc gggccctgtg accacgatct
2100

acatgctggc agggaagccc cgcggatccg aaggccctgt ccgctctgtc ttccgccatt
2160

ttggtagctt ccagaaaggc caggcggaag ccaaggtcaa gagggccatc cctaagcctc
2220

cgcgccaggc cctgaatcgg aaaaagggca gccctggcct tgcctctggc tctgtcggcc
2280

agagccccaa ctcagcccca aaagctgggc ttcctggggc cacagggcct atggcagtca
2340

gaccagagga agcggtccgg gggctggggg ctggcaccga gagttcaagg agagcccagg
2400

agccagtctc tggctgtggc tccccagaac aggatcccca gaagcaggct gaagaggaaa
2460

ggcaggagga acctgagtat gagaatgttg tacccatctc caggccacca gaaccctgat
2520

gaccttgaat ttggggagtg gggagagtgg atggactaga ctgtgctgtg tgctggaaaa
2580

tgatcccggg gccaggacag acaaaccaga gcctctgcgc ctccacaggg aaaaggcaag
2640

gcttccaggc cagttggccc aggcccctgg cagtgctccc ggagggcccc aggaaggcct
2700

gggcagagac cctgtaggat ggggtcagga agggttgcct gcagggactt ttgctctgct
2760

gtcctggacc ctgtgtgcct cataagggct attctttctt tcacgtgcaa aacatttttc
2820

tgaaatagca aacaacctac atgtttgctg ataaaagatt ggctaaacaa aatttttttt
2880

ttttttgaga cagaatctcc ctctgtcccc caggctggag tgcagtggtg cgatctcggc
2940

tcactgcaag ctctgcctcc cgggttcacg cccttctcct gcctcagcct cccgagtagc
3000

tgggactaca ggtgccctcc accacgcttg gctaattttt ttgtatattt aatagagaca
3060

gggtttcacc atgttagcca ggatggtctc gatctcctga cctcgtgatc cacctgcctc
3120

ggcctcccca aagtgctggg atgacaggca tgagccacca cgcctggtct atgaactttt
3180

taaaaaggat gtatgtgtat aaacagattc aagggaaagg cactaaatgg ttttttcctc
3240

tggaagatga gattgtaggt gatatttatt ttcttctgaa acttttgtat agtttgcaaa
3300

ttttctacag tgaacattct tttttacttt tgttactaga ttgaatttga taaagtataa
3360

taaaaagcaa tgatctttgt taaaaaataa aaagtactaa cattacag
3408

<210> 101
<211> 19
<212> RNA
<213> Artificial

<220>
<223> Human acetyl LDL receptor siRNA 1 sense strand

<400> 101
gccgcugcaa acacaauga
19


<210> 102
<211> 19
<212> RNA
<213> Artificial

<220>
<223> Human acetyl LDL receptor siRNA 2 sense strand

<400> 102
cuaccguguc caggaugaa
19


<210> 103
<211> 19
<212> RNA
<213> Artificial

<220>
<223> Human acetyl LDL receptor siRNA 3 sense strand

<400> 103
ugaccacgau cuacaugcu
19


<210> 104
<211> 19
<212> RNA
<213> Artificial

<220>
<223> Human acetyl LDL receptor siRNA 4 sense strand

<400> 104
cagccgguuu ccauggaaa
19


<210> 105
<211> 1335
<212> PRT

<213>  Homo sapiens

<400>  105

Met Glu Ser Glu Met Leu Gln Ser Pro Leu Leu Gly Leu Gly Glu Glu
1               5                   10                  15

Asp Glu Ala Asp Leu Thr Asp Trp Asn Leu Pro Leu Ala Phe Met Lys
            20                  25                  30

Lys Arg His Cys Glu Lys Ile Glu Gly Ser Lys Ser Leu Ala Gln Ser
        35                  40                  45

Trp Arg Met Lys Asp Arg Met Lys Thr Val Ser Val Ala Leu Val Leu
    50                  55                  60

Cys Leu Asn Val Gly Val Asp Pro Pro Asp Val Val Lys Thr Thr Pro
65                  70                  75                  80

Cys Ala Arg Leu Glu Cys Trp Ile Asp Pro Leu Ser Met Gly Pro Gln
                85                  90                  95

Lys Ala Leu Glu Thr Ile Gly Ala Asn Leu Gln Lys Gln Tyr Glu Asn
            100                 105                 110

Trp Gln Pro Arg Ala Arg Tyr Lys Gln Ser Leu Asp Pro Thr Val Asp
        115                 120                 125

Glu Val Lys Lys Leu Cys Thr Ser Leu Arg Arg Asn Ala Lys Glu Glu
    130                 135                 140

Arg Val Leu Phe His Tyr Asn Gly His Gly Val Pro Arg Pro Thr Val
145                 150                 155                 160

Asn Gly Glu Val Trp Val Phe Asn Lys Asn Tyr Thr Gln Tyr Ile Pro
                165                 170                 175

Leu Ser Ile Tyr Asp Leu Gln Thr Trp Met Gly Ser Pro Ser Ile Phe
            180                 185                 190

Val Tyr Asp Cys Ser Asn Ala Gly Leu Ile Val Lys Ser Phe Lys Gln
        195                 200                 205

Phe Ala Leu Gln Arg Glu Gln Glu Leu Glu Val Ala Ala Ile Asn Pro

    210                 215                 220

Asn His Pro Leu Ala Gln Met Pro Leu Pro Pro Ser Met Lys Asn Cys
225                 230                 235                 240

Ile Gln Leu Ala Ala Cys Glu Ala Thr Glu Leu Leu Pro Met Ile Pro
                245                 250                 255

Asp Leu Pro Ala Asp Leu Phe Thr Ser Cys Leu Thr Thr Pro Ile Lys
            260                 265                 270

Ile Ala Leu Arg Trp Phe Cys Met Gln Lys Cys Val Ser Leu Val Pro
        275                 280                 285

Gly Val Thr Leu Asp Leu Ile Glu Lys Ile Pro Gly Arg Leu Asn Asp
    290                 295                 300

Arg Arg Thr Pro Leu Gly Glu Leu Asn Trp Ile Phe Thr Ala Ile Thr
305                 310                 315                 320

Asp Thr Ile Ala Trp Asn Val Leu Pro Arg Asp Leu Phe Gln Lys Leu
                325                 330                 335

Phe Arg Gln Asp Leu Leu Val Ala Ser Leu Phe Arg Asn Phe Leu Leu
            340                 345                 350

Ala Glu Arg Ile Met Arg Ser Tyr Asn Cys Thr Pro Val Ser Ser Pro
        355                 360                 365

Arg Leu Pro Pro Thr Tyr Met His Ala Met Trp Gln Ala Trp Asp Leu
    370                 375                 380

Ala Val Asp Ile Cys Leu Ser Gln Leu Pro Thr Ile Ile Glu Glu Gly
385                 390                 395                 400

Thr Ala Phe Arg His Ser Pro Phe Phe Ala Glu Gln Leu Thr Ala Phe
                405                 410                 415

Gln Val Trp Leu Thr Met Gly Val Glu Asn Arg Asn Pro Pro Glu Gln
            420                 425                 430

Leu Pro Ile Val Leu Gln Val Leu Leu Ser Gln Val His Arg Leu Arg
        435                 440                 445

Ala Leu Asp Leu Leu Gly Arg Phe Leu Asp Leu Gly Pro Trp Ala Val
    450                 455                 460

Ser Leu Ala Leu Ser Val Gly Ile Phe Pro Tyr Val Leu Lys Leu Leu
465                 470                 475                 480

Gln Ser Ser Ala Arg Glu Leu Arg Pro Leu Leu Val Phe Ile Trp Ala
                485                 490                 495

Lys Ile Leu Ala Val Asp Ser Ser Cys Gln Ala Asp Leu Val Lys Asp
            500                 505                 510

Asn Gly His Lys Tyr Phe Leu Ser Val Leu Ala Asp Pro Tyr Met Pro
        515                 520                 525

Ala Glu His Arg Thr Met Thr Ala Phe Ile Leu Ala Val Ile Val Asn
    530                 535                 540

Ser Tyr His Thr Gly Gln Glu Ala Cys Leu Gln Gly Asn Leu Ile Ala
545                 550                 555                 560

Ile Cys Leu Glu Gln Leu Asn Asp Pro His Pro Leu Leu Arg Gln Trp
                565                 570                 575

Val Ala Ile Cys Leu Gly Arg Ile Trp Gln Asn Phe Asp Ser Ala Arg
            580                 585                 590

Trp Cys Gly Val Arg Asp Ser Ala His Glu Lys Leu Tyr Ser Leu Leu
        595                 600                 605

Ser Asp Pro Ile Pro Glu Val Arg Cys Ala Ala Val Phe Ala Leu Gly
    610                 615                 620

Thr Phe Val Gly Asn Ser Ala Glu Arg Thr Asp His Ser Thr Thr Ile
625                 630                 635                 640

Asp His Asn Val Ala Met Met Leu Ala Gln Leu Val Ser Asp Gly Ser
                645                 650                 655

Pro Met Val Arg Lys Glu Leu Val Val Ala Leu Ser His Leu Val Val
            660                 665                 670

Gln Tyr Glu Ser Asn Phe Cys Thr Val Ala Leu Gln Phe Ile Glu Glu
        675                 680                 685

Glu Lys Asn Tyr Ala Leu Pro Ser Pro Ala Thr Thr Glu Gly Gly Ser
    690                 695                 700

Leu Thr Pro Val Arg Asp Ser Pro Cys Thr Pro Arg Leu Arg Ser Val
705                 710                 715                 720

Ser Ser Tyr Gly Asn Ile Arg Ala Val Ala Thr Ala Arg Ser Leu Asn
                725                 730                 735

Lys Ser Leu Gln Asn Leu Ser Leu Thr Glu Glu Ser Gly Gly Ala Val
            740                 745                 750

Ala Phe Ser Pro Gly Asn Leu Ser Thr Ser Ser Ser Ala Ser Ser Thr
        755                 760                 765

Leu Gly Ser Pro Glu Asn Glu Glu His Ile Leu Ser Phe Glu Thr Ile
    770                 775                 780

Asp Lys Met Arg Arg Ala Ser Ser Tyr Ser Ser Leu Asn Ser Leu Ile
785                 790                 795                 800

Gly Val Ser Phe Asn Ser Val Tyr Thr Gln Ile Trp Arg Val Leu Leu
                805                 810                 815

His Leu Ala Ala Asp Pro Tyr Pro Glu Val Ser Asp Val Ala Met Lys
            820                 825                 830

Val Leu Asn Ser Ile Ala Tyr Lys Ala Thr Val Asn Ala Arg Pro Gln
        835                 840                 845

Arg Val Leu Asp Thr Ser Ser Leu Thr Gln Ser Ala Pro Ala Ser Pro
    850                 855                 860

Thr Asn Lys Gly Val His Ile His Gln Ala Gly Gly Ser Pro Pro Ala
865                 870                 875                 880

Ser Ser Thr Ser Ser Ser Ser Leu Thr Asn Asp Val Ala Lys Gln Pro
                885                 890                 895

Val Ser Arg Asp Leu Pro Ser Gly Arg Pro Gly Thr Thr Gly Pro Ala
            900                 905                 910

Gly Ala Gln Tyr Thr Pro His Ser His Gln Phe Pro Arg Thr Arg Lys
        915                 920                 925

Met Phe Asp Lys Gly Pro Glu Gln Thr Ala Asp Asp Ala Asp Asp Ala
    930                 935                 940

Ala Gly His Lys Ser Phe Ile Ser Ala Thr Val Gln Thr Gly Phe Cys
945                 950                 955                 960

Asp Trp Ser Ala Arg Tyr Phe Ala Gln Pro Val Met Lys Ile Pro Glu
                965                 970                 975

Glu His Asp Leu Glu Ser Gln Ile Arg Lys Glu Arg Glu Trp Arg Phe
            980                 985                 990

Leu Arg Asn Ser Arg Val Arg Arg Gln Ala Gln Gln Val Ile Gln Lys
        995                 1000                1005

Gly Ile Thr Arg Leu Asp Asp Gln Ile Phe Leu Asn Arg Asn Pro
    1010                1015                1020

Gly Val Pro Ser Val Val Lys Phe His Pro Phe Thr Pro Cys Ile
    1025                1030                1035

Ala Val Ala Asp Lys Asp Ser Ile Cys Phe Trp Asp Trp Glu Lys
    1040                1045                1050

Gly Glu Lys Leu Asp Tyr Phe His Asn Gly Asn Pro Arg Tyr Thr
    1055                1060                1065

Arg Val Thr Ala Met Glu Tyr Leu Asn Gly Gln Asp Cys Ser Leu
    1070                1075                1080

Leu Leu Thr Ala Thr Asp Asp Gly Ala Ile Arg Val Trp Lys Asn
    1085                1090                1095

Phe Ala Asp Leu Glu Lys Asn Pro Glu Met Val Thr Ala Trp Gln
    1100                1105                1110

Gly Leu Ser Asp Met Leu Pro Thr Thr Arg Gly Ala Gly Met Val

1115                    1120                    1125

Val Asp  Trp Glu Gln Glu Thr  Gly Leu Leu Met Ser  Ser Gly Asp
    1130                    1135                    1140

Val Arg  Ile Val Arg Ile Trp  Asp Thr Asp Arg Glu  Met Lys Val
    1145                    1150                    1155

Gln Asp  Ile Pro Thr Gly Ala  Asp Ser Cys Val Thr  Ser Leu Ser
    1160                    1165                    1170

Cys Asp  Ser His Arg Ser Leu  Ile Val Ala Gly Leu  Gly Asp Gly
    1175                    1180                    1185

Ser Ile  Arg Val Tyr Asp Arg  Arg Met Ala Leu Ser  Glu Cys Arg
    1190                    1195                    1200

Val Met  Thr Tyr Arg Glu His  Thr Ala Trp Val Val  Lys Ala Ser
    1205                    1210                    1215

Leu Gln  Lys Arg Pro Asp Gly  His Ile Val Ser Val  Ser Val Asn
    1220                    1225                    1230

Gly Asp  Val Arg Ile Phe Asp  Pro Arg Met Pro Glu  Ser Val Asn
    1235                    1240                    1245

Val Leu  Gln Ile Val Lys Gly  Leu Thr Ala Leu Asp  Ile His Pro
    1250                    1255                    1260

Gln Ala  Asp Leu Ile Ala Cys  Gly Ser Val Asn Gln  Phe Thr Ala
    1265                    1270                    1275

Ile Tyr  Asn Ser Ser Gly Glu  Leu Ile Asn Asn Ile  Lys Tyr Tyr
    1280                    1285                    1290

Asp Gly  Phe Met Gly Gln Arg  Val Gly Ala Ile Ser  Cys Leu Ala
    1295                    1300                    1305

Phe His  Pro His Trp Pro His  Leu Ala Val Gly Ser  Asn Asp Tyr
    1310                    1315                    1320

Tyr Ile  Ser Val Tyr Ser Val  Glu Lys Arg Val Arg
    1325                    1330                    1335

<210> 106
<211> 6431
<212> DNA
<213> Homo sapiens

<400> 106
gagagcaggg tcatcgtgag gcctgaagtc tcttacgctt ttggcagctc ccctcgcagc
60

ccctctggaa acgtacagcc tcaggagcag ccagtggctt gggacctggg gtggtgtgtg
120

tctgcggagc ttcttgggct gccccatttc ctagcggccc ccacctcccc acttcccgct
180

cagagttaga gataaggatc tcagactttt gcctgagtaa gggtctccgc actctttatc
240

catttggttt tcgatttccc gtttttgttt cttatttcac caattctggt acacgctagt
300

ttttaaggct ggaggttctc gagcgcttgc tgccaaggac tcccccaccc cctcccccac
360

tgatggagtc cgaaatgctg caatcgcctc ttctgggcct gggggaggaa gatgaggctg
420

atcttacaga ctggaaccta cctttggctt ttatgaaaaa gaggcactgt gagaaaattg
480

aaggctccaa atccttagct cagagctgga ggatgaagga tcggatgaag acagtcagtg
540

ttgccttagt tttgtgcctg aatgttggtg tggaccctcc cgatgtggtg aagaccacgc
600

cctgtgcacg cttggaatgc tggatcgatc ctctgtcgat gggtcctcag aaagctctgg
660

aaaccatcgg tgcaaattta cagaagcagt acgagaactg gcagccaagg gcccggtaca
720

agcagagcct tgacccaact gtggatgaag tcaagaagct ctgcacgtcc ttacgtcgca
780

acgccaagga ggagcgagtc ctctttcact acaatggcca cggggtgccc cggcccacag
840

tcaacgggga ggtctgggtc ttcaacaaga actacacgca gtacatccct ctgtccatat
900

atgacctgca gacgtggatg ggcagcccgt cgatcttcgt ctacgactgc tccaatgctg
960

gcttgatcgt caagtccttc aagcagttcg cactacagcg ggagcaggag ctggaggtag
1020

ctgcaatcaa cccaaatcac cctcttgctc agatgccttt gcctccgtcg atgaaaaact
1080

gcatccagct ggcagcctgc gaggccaccg agctgctgcc catgatcccc gacctcccgg
1140

ctgacctatt cacctcctgc ctcaccaccc ccatcaagat cgccctgcgc tggttttgca
1200

tgcagaaatg tgtcagtctg gtgcctggcg tcacactgga tttgatagaa aagatccctg
1260

gccgcctgaa cgacaggagg acgcccctgg gtgaactgaa ctggatcttc acagccatca
1320

cagacaccat cgcgtggaac gtgctccccc gggatctctt ccaaaagctc ttcagacagg
1380

acttgctggt ggctagtctg tttcgaaatt ttttattggc ggaaaggatt atgaggtcgt
1440

ataactgcac tcccgtcagc agcccgcgtc tgccgcccac gtacatgcac gccatgtggc
1500

aagcctggga cctggctgtt gacatctgtc tgtctcagct gccgacgatc atcgaggaag
1560

gcactgcgtt tcggcacagc ccgttcttcg ccgagcagct gaccgcattc caggtgtggc
1620

tcaccatggg cgtggagaac cgaaacccac ccgaacagct gcccatcgtc ctgcaggtgc
1680

tgttaagcca agtgcaccgg ctgagagcat tggacttgct tggaagattt ttggacctgg
1740

gtccctgggc agtgagcctg gccttgtctg tcggcatctt cccctacgtg ctgaagctgc
1800

tccagagctc ggcccgagag ctgcggccac ttctcgtttt catctgggcc aagatcctcg
1860

cagtggacag ctcgtgccag gcggacctcg tgaaggacaa cggccacaag tacttcctgt
1920

cggtcctggc ggacccctac atgccagctg aacaccggac catgacggct ttcattctcg
1980

ccgtgatcgt caacagctat cacacggggc aggaagcctg ccttcaggga aacctcattg
2040

ccatctgcct ggagcagctc aacgacccgc accccttgct gcgccagtgg gtggccatct
2100

gcctcggcag gatctggcag aacttcgact cggcgaggtg gtgcggcgtg agggacagcg
2160

ctcatgagaa gctctacagc ctcctctccg accccattcc cgaggtccgc tgcgcagcgg
2220

tcttcgccct tggcacgttc gtgggcaact ctgcagagag gacggaccac tccaccacca
2280

tcgaccacaa cgtggccatg atgctggccc agctggtcag cgacgggagc cccatggtcc
2340

ggaaggagct ggtggtggct ctgagtcatc ttgtggttca gtatgaaagc aatttctgca
2400

ccgtggccct gcagttcata gaagaggaaa agaactacgc cttgccttct ccagcaacca
2460

cagagggagg gagtttgacc ccagtgcgag acagcccgtg cacccccaga cttcgttctg
2520

tgagctccta tggaaacatc cgtgctgtcg ccacagccag gagcctcaac aaatctttgc
2580

agaacctgag tttgacagag gaatctggtg gcgcggtggc gttctccccc ggaaacctca
2640

gcaccagcag cagcgccagc agcaccctgg gcagccccga gaatgaggag catatcctgt
2700

ccttcgagac catcgacaag atgcgccgcg ccagctccta ctcctccctc aactccctca
2760

tcggagtttc ctttaacagt gtttacactc agatttggag agtcctgctg cacctggctg
2820

ctgaccccta tccagaggtc tcggacgtgg ccatgaaagt actcaacagc atcgcctaca
2880

aggccaccgt gaacgcccgg ccgcagcgcg tcctggacac ctcctccctc acgcagtcgg
2940

cccccgccag ccccaccaac aagggcgtgc acatccacca ggcggggggc tcccctccgg
3000

cgtccagcac cagcagctcc agcctgacca acgatgtggc caagcagccg gtcagccgag
3060

acttgccttc tggccggccg ggcaccacag gccccgctgg ggcgcagtac acccctcact
3120

cccaccagtt cccccggaca cggaagatgt tcgacaaggg cccagagcag actgcggacg
3180

acgcggacga tgctgctgga cacaaaagtt tcatctccgc cacggtgcag acggggttct
3240

gcgactggag cgcccgctat tttgcccagc ccgtcatgaa gatcccagaa gagcacgacc
3300

tggagagtca gatccgcaag gagcgggagt ggcggttcct gcgaaacagc cgtgtcagga
3360

ggcaggccca gcaagtcatt cagaagggca ttacgagatt ggacgaccaa atatttctga
3420

acaggaaccc cggcgtcccc tctgtggtga aattccaccc cttcacgccg tgcatcgctg
3480

tagccgacaa ggacagcatc tgcttttggg actgggagaa aggggagaag ctggattatt
3540

tccacaatgg gaaccctcgg tacacgaggg tcactgccat ggagtatctg aatggccagg
3600

actgctcgct tctgctgacg gccacagacg atggtgccat cagggtctgg aagaatttg
3660

ctgatttgga aaagaaccca gagatggtga ccgcgtggca ggggctctcg gacatgctgc
3720

caacgacgcg aggagctggg atggtggtgg actgggagca ggagaccggc ctcctcatga
3780

gctcaggaga cgtgcggatc gtccggatct gggacacaga ccgtgagatg aaggtgcagg
3840

acatccctac gggcgcagac agctgtgtga cgagtctgtc ctgtgattcc caccgctcac
3900

tcatcgtggc tggcctcggt gacggctcca tccgcgtcta cgacagaagg atggcactca
3960

gcgaatgccg cgtcatgacg taccgggagc acacagcctg ggtggtgaag gcctccctgc
4020

agaagcgtcc cgacggccac atcgtgagtg tgagcgtcaa tggagatgtg cgcatctttg
4080

atccccggat gcctgagtcg gtaaatgtgc ttcagatcgt gaaggggctg acggccctgg
4140

acatccaccc ccaggcggac ctgatcgcat gtggctccgt caatcagttc accgccatct
4200

acaacagcag cggagagctc atcaacaaca tcaagtacta cgacggcttc atgggccagc
4260

gggtcggcgc catcagctgc ctggccttcc acccgcactg gcctcacctg gccgtgggaa
4320

gcaacgacta ctacatctcc gtgtactcgg tggagaagcg tgtcagatag cggcgtgacc
4380

cgggcccacc aggccacggc cgcctgctgt acatagtgaa gctgtcactc gccggggcac
4440

ggggcgtcgg ctgctgcggc cccgcagtgt gaacgttggc tgctgcctta gctgctgatg
4500

acggcaggag ggccctgcta ctcgcttttg tctgtcttcg ctgtcgtgtc tggaatgtca
4560

gggaagggga gggctcgggt tgacggtggc ttcccactga gcaccagcat ccaggtgcac
4620

ccccgcggcc acggcgcctc tgtccctctc ctgttctgtg tttctctgag acgctgaaag
4680

gggaaacacc tcactttatt tccatgtaat cagagcatta gctgcagaaa aacccccaga
4740

cagagccctg gcggagaggc aggcgctggg gctcctacgg gtccctgggg cagctgtccc
4800

catcaggcca agagcgagcg agaggcgctg ccccagccag gcccaccacc tctcacagtc
4860

agtgcacgca agcaggacat tttcctagcc agctggggga cactggaaat tcgggaaacc
4920

aagagagagg aagaaggaga cgcccctcca actggcgggt gtgaaggaag ccgcccaggg
4980

gtccgggctg tccttggccg ctggcagcat cactgagcag gaagcgcaca gcccaccctc
5040

cccgcacctc caggtctctg gactccagtt ttggcccctc tcacacagag ctgtcagcag
5100

gggccgctgt ggcggtgcac aggggaggca ggtccttggc gaggtagccc ctgccttaat
5160

ccacggggct cctttccctc cgaagggctg ctcttcccca caggcgcggg gacagcagcc
5220

cgacctgtgg tctccatgcc tgtgccctca cacaggtgta gcacacgcat gtgcagatgg
5280

caccacggcc ggcacctggg ggcacacaca tgcaggcggc gtggtctccc tgctctgtcc
5340

ccacacgttc ctcacataca ggcaagaggc actgccgggt cccggacggc tccgggtgac
5400

accagccccg tctccagcct tgagccgccc atgctgatgc gacctcggct gacagctggg
5460

cctgtggtgc agacaggagc tgtgtggaca gtcccgccca ggaggggccg cagggcgtgt
5520

atgagcagtt ttgcaaacag aacacaacca caatgatggt attttgaaaa gtgttctttc
5580

cgtgttcgtc gggaatcagg attattgaga ggtgaaggag ccaggtggct tcattctggc
5640

ggtgagaggc ccatgaccac gggagtgaga gctggtgtgg cgaggcccgg ctctcctgcg
5700

gtgtggctgg tggcctgccg tggccaagag catcttctgg gtggatggaa ccctgcctgg
5760

tcacatttgg ccagagacac acctggccct cagggggctg agctggagac tgagctgggg
5820

ctggccggga cgtgacaagg caggacagag gcggcccctc cgctgctcct ttttggaatg
5880

cgagctccca ccagaagaag gttccggcac gaatcccatc cccacgtctg ggccgagaaa
5940

gcagcccggg tccggaaggt gtagagagtc ccggcctcac tcagctcaca gggcgtgcca
6000

ggcggcaaca ccagaatctt ccagaagccc agctccaccc gcacacgcag cttcccatcc
6060

agtccttcaa ctcaattctt acccaacacg cgtttccgtt tgttttgaga caaaatcacc
6120

acctgtcaaa aggcaggtgg ctccagaggg gtcaagaccc ccccccccgc ccccgctcca
6180

ccctggagcc cacccccatg ggcactgcgt gccgcctgca cgtgggctgt cttcacaggt
6240

ctgatgtgaa aattcaatca cgacgttaac cggctcgaga gagcgccggc ctagaggctc
6300

attatctatt tattttacca aacgcgaatt gagacggact ttgacaaaac acgaaatggt
6360

aatgtgaagc taagagcaga gagtgaccaa cagtaaacaa cacgcgcaga aaaaaaaaaa
6420

aaaaaaaaaa a
6431


<210>  107
<211>  19
<212>  RNA
<213>  Artificial

<220>
<223>  Human mTor siRNA 1 sense strand

<400>  107
aaccaucggu gcaaauuua
19


<210>  108
<211>  19
<212>  RNA
<213>  Artificial

<220>
<223>  Human mTor siRNA 2 sense strand

<400>  108
aaaccaucgg ugcaaauuu
19


<210>  109
<211>  19
<212>  RNA
<213>  Artificial

<220>
<223>  Human mTor siRNA 3 sense strand

<400>  109
ccaucggugc aaauuuaca
19


<210>  110
<211>  19
<212>  RNA
<213>  Artificial

<220>
<223>  Human mTor siRNA 4 sense strand

<400>  110
ucaagcaguu cgcacuaca
19


<210>  111
<211>  19
<212>  RNA
<213>  Artificial

<220>
<223>  Human mTor siRNA 5 sense strand

<400>  111
ugacgagucu gugguguga
19

<210>  112
<211>  19
<212>  RNA
<213>  Artificial

<220>
<223>  Human mTor siRNA 6 sense strand

<400>  112
ccacugaugg aguccgaaa
19


<210>  113
<211>  480
<212>  PRT
<213>  Artificial

<220>
<223>  Homo sapiens clone FLH018874.01X v-akt murine thymoma viral
       oncogene-like 1 (AKT1) protein

<400>  113

Met Ser Asp Val Ala Ile Val Lys Glu Gly Trp Leu His Lys Arg Gly
1               5                   10                  15

Glu Tyr Ile Lys Thr Trp Arg Pro Arg Tyr Phe Leu Leu Lys Asn Asp
            20                  25                  30

Gly Thr Phe Ile Gly Tyr Lys Glu Arg Pro Gln Asp Val Asp Gln Arg
        35                  40                  45

Glu Ala Pro Leu Asn Asn Phe Ser Val Ala Gln Cys Gln Leu Met Lys
    50                  55                  60

Thr Glu Arg Pro Arg Pro Asn Thr Phe Ile Ile Arg Cys Leu Gln Trp
65                  70                  75                  80

Thr Thr Val Ile Glu Arg Thr Phe His Val Glu Thr Pro Glu Glu Arg
                85                  90                  95

Glu Glu Trp Thr Thr Ala Ile Gln Thr Val Ala Asp Gly Leu Lys Lys
            100                 105                 110

Gln Glu Glu Glu Glu Met Asp Phe Arg Ser Gly Ser Pro Ser Asp Asn
        115                 120                 125

Ser Gly Ala Glu Glu Met Glu Val Ser Leu Ala Lys Pro Lys His Arg

130                 135                 140

Val Thr Met Asn Glu Phe Glu Tyr Leu Lys Leu Leu Gly Lys Gly Thr
145                 150                 155                 160

Phe Gly Lys Val Ile Leu Val Lys Glu Lys Ala Thr Gly Arg Tyr Tyr
                165                 170                 175

Ala Met Lys Ile Leu Lys Lys Glu Val Ile Val Ala Lys Asp Glu Val
            180                 185                 190

Ala His Thr Leu Thr Glu Asn Arg Val Leu Gln Asn Ser Arg His Pro
        195                 200                 205

Phe Leu Thr Ala Leu Lys Tyr Ser Phe Gln Thr His Asp Arg Leu Cys
    210                 215                 220

Phe Val Met Glu Tyr Ala Asn Gly Gly Glu Leu Phe Phe His Leu Ser
225                 230                 235                 240

Arg Glu Arg Val Phe Ser Glu Asp Arg Ala Arg Phe Tyr Gly Ala Glu
                245                 250                 255

Ile Val Ser Ala Leu Asp Tyr Leu His Ser Glu Lys Asn Val Val Tyr
            260                 265                 270

Arg Asp Leu Lys Leu Glu Asn Leu Met Leu Asp Lys Asp Gly His Ile
        275                 280                 285

Lys Ile Thr Asp Phe Gly Leu Cys Lys Glu Gly Ile Lys Asp Gly Ala
    290                 295                 300

Thr Met Lys Thr Phe Cys Gly Thr Pro Glu Tyr Leu Ala Pro Glu Val
305                 310                 315                 320

Leu Glu Asp Asn Asp Tyr Gly Arg Ala Val Asp Trp Trp Gly Leu Gly
                325                 330                 335

Val Val Met Tyr Glu Met Met Cys Gly Arg Leu Pro Phe Tyr Asn Gln
            340                 345                 350

Asp His Glu Lys Leu Phe Glu Leu Ile Leu Met Glu Glu Ile Arg Phe
        355                 360                 365

Pro Arg Thr Leu Gly Pro Glu Ala Lys Ser Leu Leu Ser Gly Leu Leu
    370                 375                 380

Lys Lys Asp Pro Lys Gln Arg Leu Gly Gly Gly Ser Glu Asp Ala Lys
385                 390                 395                 400

Glu Ile Met Gln His Arg Phe Phe Ala Gly Ile Val Trp Gln His Val
                405                 410                 415

Tyr Glu Lys Lys Leu Ser Pro Pro Phe Lys Pro Gln Val Thr Ser Glu
            420                 425                 430

Thr Asp Thr Arg Tyr Phe Asp Glu Glu Phe Thr Ala Gln Met Ile Thr
        435                 440                 445

Ile Thr Pro Pro Asp Gln Asp Asp Ser Met Glu Cys Val Asp Ser Glu
    450                 455                 460

Arg Arg Pro His Phe Pro Gln Phe Ser Tyr Ser Ala Ser Ser Thr Ala
465                 470                 475                 480

<210>  114
<211>  1443
<212>  DNA
<213>  Artificial

<220>
<223>  Homo sapiens clone FLH018874.01X v-akt murine thymoma viral
       oncogene-like 1 (AKT1) cDNA

<400>  114
atgagcgacg tggctattgt gaaggagggt tggctgcaca aacgagggga gtacatcaag
60

acctggcggc cacgctactt cctcctcaag aatgatggca ccttcattgg ctacaaggag
120

cggccgcagg atgtggacca acgtgaggct cccctcaaca acttctctgt ggcgcagtgc
180

cagctgatga agacggagcg gccccggccc aacaccttca tcatccgctg cctgcagtgg
240

accactgtca tcgaacgcac cttccatgtg gagactcctg aggagcggga ggagtggaca
300

accgccatcc agactgtggc tgacggcctc aagaagcagg aggaggagga gatggacttc
360

cggtcgggct cacccagtga caactcaggg gctgaagaga tggaggtgtc cctggccaag
420

cccaagcacc gcgtgaccat gaacgagttt gagtacctga agctgctggg caagggcact
480

ttcggcaagg tgatcctggt gaaggagaag gccacaggcc gctactacgc catgaagatc
540

ctcaagaagg aagtcatcgt ggccaaggac gaggtggccc acacactcac cgagaaccgc
600

gtcctgcaga actccaggca ccccttcctc acagccctga agtactcttt ccagacccac
660

gaccgcctct gctttgtcat ggagtacgcc aacgggggcg agctgttctt ccacctgtcc
720

cgggagcgtg tgttctccga ggaccgggcc cgcttctatg gcgctgagat tgtgtcagcc
780

ctggactacc tgcactcgga gaagaacgtg gtgtaccggg acctcaagct ggagaacctc
840

atgctggaca aggacgggca cattaagatc acagacttcg ggctgtgcaa ggaggggatc
900

aaggacggtg ccaccatgaa gaccttttgc ggcacacctg agtacctggc ccccgaggtg
960

ctggaggaca atgactacgg ccgtgcagtg gactggtggg ggctgggcgt ggtcatgtac
1020

gagatgatgt gcggtcgcct gcccttctac aaccaggacc atgagaagct ttttgagctc
1080

atcctcatgg aggagatccg cttcccgcgc acgcttggtc ccgaggccaa gtccttgctt
1140

tcagggctgc tcaagaagga ccccaagcag aggcttggcg ggggctccga ggacgccaag
1200

gagatcatgc agcatcgctt ctttgccggt atcgtgtggc agcacgtgta cgagaagaag
1260

ctcagcccac ccttcaagcc ccaggtcacg tcggagactg acaccaggta ttttgatgag
1320

gagttcacgg cccagatgat caccatcaca ccacctgacc aagatgacag catggagtgt
1380

gtggacagcg agcgcaggcc ccacttcccc cagttctcct actcggccag cagcacggcc
1440

tag
1443

<210>  115
<211>  19
<212>  RNA
<213>  Artificial

<220>
<223>  Human AKT1 siRNA 1 sense strand

<400>  115
acuuccuccu caagaauga
19


<210>  116
<211>  19
<212>  RNA
<213>  Artificial

<220>
<223>  Human AKT1 siRNA 2 sense strand

<400>  116
ugaccaugaa cgaguuuga
19


<210>  117
<211>  19
<212>  RNA
<213>  Artificial

<220>
<223>  Human AKT1 siRNA 3 sense strand

<400>  117
acguggcuau ugugaagga
19


<210>  118
<211>  19
<212>  RNA
<213>  Artificial

<220>
<223>  Human AKT1 siRNA 4 sense strand

<400>  118
ugaacgaguu ugaguaccu
19


<210>  119
<211>  19
<212>  RNA

<213>  Artificial

<220>
<223>  Human AKT1 siRNA 5 sense strand

<400>  119
guggaccacu gucaucgaa
19


<210>  120
<211>  19
<212>  RNA
<213>  Artificial

<220>
<223>  Human AKT1 siRNA 6 sense strand

<400>  120
cgugaccaug aacgaguuu
19


<210>  121
<211>  19
<212>  RNA
<213>  Artificial

<220>
<223>  Human AKT1 siRNA 7 sense strand

<400>  121
gcgacguggc uauugugaa
19


<210>  122
<211>  19
<212>  RNA
<213>  Artificial

<220>
<223>  Human AKT1 siRNA 8 sense strand

<400>  122
agcgacgugg cuauuguga
19


<210>  123
<211>  19
<212>  RNA
<213>  Artificial

<220>
<223>  Human AKT1 siRNA 9 sense strand

<400>  123
acgaguuuga guaccugaa
19

<210>  124
<211>  19
<212>  RNA
<213>  Artificial

<220>
<223>  Human AKT1 siRNA 10 sense strand

<400>  124
cgcuacuacg ccaugaaga
19

<210>  125
<211>  481
<212>  PRT
<213>  Homo sapiens

<400>  125

<210> 126
<211> 1599
<212> DNA
<213> Homo sapiens

<400> 126
gagactgtgc cctgtccacg gtgcctcctg catgtcctgc tgccctgagc tgtcccgagc
60

taggtgacag cgtaccacgc tgccaccatg aatgaggtgt ctgtcatcaa agaaggctgg
120

ctccacaagc gtggtgaata catcaagacc tggaggccac ggtacttcct gctgaagagc
180

gacggctcct tcattgggta caaggagagg cccgaggccc ctgatcagac tctacccccc
240

ttaaacaact tctccgtagc agaatgccag ctgatgaaga ccgagaggcc gcgacccaac
300

acctttgtca tacgctgcct gcagtggacc acagtcatcg agaggacctt ccacgtggat
360

tctccagacg agagggagga gtggatgcgg gccatccaga tggtcgccaa cagcctcaag
420

cagcgggccc caggcgagga ccccatggac tacaagtgtg gctcccccag tgactcctcc
480

acgactgagg agatggaagt ggcggtcagc aaggcacggg ctaaagtgac catgaatgac
540

ttcgactatc tcaaactcct tggcaaggga acctttggca aagtcatcct ggtgcgggag
600

aaggccactg gccgctacta cgccatgaag atcctgcgaa aggaagtcat cattgccaag
660

gatgaagtcg ctcacacagt caccgagagc cgggtcctcc agaacaccag gcacccgttc
720

ctcactgcgc tgaagtatgc cttccagacc cacgaccgcc tgtgctttgt gatggagtat
780

gccaacgggg gtgagctgtt cttccacctg tcccgggagc gtgtcttcac agaggagcgg
840

gcccggtttt atggtgcaga gattgtctcg gctcttgagt acttgcactc gcgggacgtg
900

gtataccgcg acatcaagct ggaaaacctc atgctggaca aagatggcca catcaagatc
960

actgactttg gcctctgcaa agagggcatc agtgacgggg ccaccatgaa aaccttctgt
1020

gggaccccgg agtacctggc gcctgaggtg ctggaggaca atgactatgg ccgggccgtg
1080

gactggtggg ggctgggtgt ggtcatgtac gagatgatgt gcggccgcct gcccttctac
1140

aaccaggacc acgagcgcct cttcgagctc atcctcatgg aagagatccg cttcccgcgc
1200

acgctcagcc ccgaggccaa gtccctgctt gctgggctgc ttaagaagga ccccaagcag
1260

aggcttggtg gggggcccag cgatgccaag gaggtcatgg agcacaggtt cttcctcagc
1320

atcaactggc aggacgtggt ccagaagaag ctcctgccac ccttcaaacc tcaggtcacg
1380

tccgaggtcg acacaaggta cttcgatgat gaatttaccg cccagtccat cacaatcaca
1440

ccccctgacc gctatgacag cctgggctta ctggagctgg accagcggac ccacttcccc
1500

cagttctcct actcggccag catccgcgag tgagcagtct gcccacgcag aggacgcacg
1560

ctcgctgcca tcaccgctgg gtggtttttt acccctgcc
1599


<210> 127
<211> 19
<212> RNA
<213> Artificial

<220>
<223> Human AKT2 siRNA 1 sense strand

<400> 127
ugacuucgac uaucucaaa
19


<210> 128
<211> 19
<212> RNA
<213> Artificial

<220>
<223> Human AKT2 siRNA 2 sense strand

<400> 128
ccaugaagau ccugcgaaa
19


<210> 129
<211> 19
<212> RNA
<213> Artificial

<220>
<223> Human AKT2 siRNA 3 sense strand

<400> 129
cacgguacuu ccugcugaa
19


<210> 130
<211> 19
<212> RNA
<213> Artificial

<220>
<223> Human AKT2 siRNA 4 sense strand

<400>  130
aaacaacuuc uccguagca
19


<210>  131
<211>  19
<212>  RNA
<213>  Artificial

<220>
<223>  Human AKT2 siRNA 5 sense strand

<400>  131
agcgugguga auacaucaa
19


<210>  132
<211>  19
<212>  RNA
<213>  Artificial

<220>
<223>  Human AKT2 siRNA 6 sense strand

<400>  132
cguggugaau acaucaaga
19


<210>  133
<211>  19
<212>  RNA
<213>  Artificial

<220>
<223>  Human AKT2 siRNA 7 sense strand

<400>  133
gaagucgcuc acacaguca
19


<210>  134
<211>  479
<212>  PRT
<213>  Homo sapiens

<400>  134

Met Ser Asp Val Thr Ile Val Lys Glu Gly Trp Val Gln Lys Arg Gly
1               5                   10                  15


Glu Tyr Ile Lys Asn Trp Arg Pro Arg Tyr Phe Leu Leu Lys Thr Asp
            20                  25                  30

Gly Ser Phe Ile Gly Tyr Lys Glu Lys Pro Gln Asp Val Asp Leu Pro
        35                  40                  45

Tyr Pro Leu Asn Asn Phe Ser Val Ala Lys Cys Gln Leu Met Lys Thr
    50                  55                  60

Glu Arg Pro Lys Pro Asn Thr Phe Ile Ile Arg Cys Leu Gln Trp Thr
65                  70                  75                  80

Thr Val Ile Glu Arg Thr Phe His Val Asp Thr Pro Glu Glu Arg Glu
                85                  90                  95

Glu Trp Thr Glu Ala Ile Gln Ala Val Ala Asp Arg Leu Gln Arg Gln
            100                 105                 110

Glu Glu Glu Arg Met Asn Cys Ser Pro Thr Ser Gln Ile Asp Asn Ile
        115                 120                 125

Gly Glu Glu Glu Met Asp Ala Ser Thr Thr His His Lys Arg Lys Thr
    130                 135                 140

Met Asn Asp Phe Asp Tyr Leu Lys Leu Leu Gly Lys Gly Thr Phe Gly
145                 150                 155                 160

Lys Val Ile Leu Val Arg Glu Lys Ala Ser Gly Lys Tyr Tyr Ala Met
                165                 170                 175

Lys Ile Leu Lys Lys Glu Val Ile Ile Ala Lys Asp Glu Val Ala His
            180                 185                 190

Thr Leu Thr Glu Ser Arg Val Leu Lys Asn Thr Arg His Pro Phe Leu
        195                 200                 205

Thr Ser Leu Lys Tyr Ser Phe Gln Thr Lys Asp Arg Leu Cys Phe Val
    210                 215                 220

Met Glu Tyr Val Asn Gly Gly Glu Leu Phe Phe His Leu Ser Arg Glu
225                 230                 235                 240

Arg Val Phe Ser Glu Asp Arg Thr Arg Phe Tyr Gly Ala Glu Ile Val
                245                 250                 255

Ser Ala Leu Asp Tyr Leu His Ser Gly Lys Ile Val Tyr Arg Asp Leu
            260                 265                 270

Lys Leu Glu Asn Leu Met Leu Asp Lys Asp Gly His Ile Lys Ile Thr
        275                 280                 285

Asp Phe Gly Leu Cys Lys Glu Gly Ile Thr Asp Ala Ala Thr Met Lys
    290                 295                 300

Thr Phe Cys Gly Thr Pro Glu Tyr Leu Ala Pro Glu Val Leu Glu Asp
305                 310                 315                 320

Asn Asp Tyr Gly Arg Ala Val Asp Trp Trp Gly Leu Gly Val Val Met
                325                 330                 335

Tyr Glu Met Met Cys Gly Arg Leu Pro Phe Tyr Asn Gln Asp His Glu
            340                 345                 350

Lys Leu Phe Glu Leu Ile Leu Met Glu Asp Ile Lys Phe Pro Arg Thr
        355                 360                 365

Leu Ser Ser Asp Ala Lys Ser Leu Leu Ser Gly Leu Leu Ile Lys Asp
    370                 375                 380

Pro Asn Lys Arg Leu Gly Gly Gly Pro Asp Asp Ala Lys Glu Ile Met
385                 390                 395                 400

Arg His Ser Phe Phe Ser Gly Val Asn Trp Gln Asp Val Tyr Asp Lys
                405                 410                 415

Lys Leu Val Pro Pro Phe Lys Pro Gln Val Thr Ser Glu Thr Asp Thr
            420                 425                 430

Arg Tyr Phe Asp Glu Glu Phe Thr Ala Gln Thr Ile Thr Ile Thr Pro
        435                 440                 445

Pro Glu Lys Tyr Asp Glu Asp Gly Met Asp Cys Met Asp Asn Glu Arg
    450                 455                 460

Arg Pro His Phe Pro Gln Phe Ser Tyr Ser Ala Ser Gly Arg Glu
465                 470                 475

<210> 135
<211> 1547
<212> DNA
<213> Homo sapiens

<400> 135
gggagtcatc atgagcgatg ttaccattgt gaaagaaggt tgggttcaga agaggggaga
60

atatataaaa aactggaggc caagatactt ccttttgaag acagatggct cattcatagg
120

atataaagag aaacctcaag atgtggattt accttatccc ctcaacaact tttcagtggc
180

aaaatgccag ttaatgaaaa cagaacgacc aaagccaaac acatttataa tcagatgtct
240

ccagtggact actgttatag agagaacatt tcatgtagat actccagagg aaagggaaga
300

atggacagaa gctatccagg ctgtagcaga cagactgcag aggcaagaag aggagagaat
360

gaattgtagt ccaacttcac aaattgataa tataggagag gaagagatgg atgcctctac
420

aacccatcat aaaagaaaga caatgaatga ttttgactat ttgaaactac taggtaaagg
480

cacttttggg aaagttattt tggttcgaga gaaggcaagt ggaaaatact atgctatgaa
540

gattctgaag aaagaagtca ttattgcaaa ggatgaagtg gcacacactc taactgaaag
600

cagagtatta aagaacacta gacatccctt tttaacatcc ttgaaatatt ccttccagac
660

aaaagaccgt ttgtgttttg tgatggaata tgttaatggg ggcgagctgt ttttccattt
720

gtcgagagag cgggtgttct ctgaggaccg cacacgtttc tatggtgcag aaattgtctc
780

tgccttggac tatctacatt ccggaaagat tgtgtaccgt gatctcaagt tggagaatct
840

aatgctggac aaagatggcc acataaaaat tacagatttt ggactttgca aagaagggat
900

cacagatgca gccaccatga agacattctg tggcactcca gaatatctgg caccagaggt
960

gttagaagat aatgactatg gccgagcagt agactggtgg ggcctagggg ttgtcatgta
1020

tgaaatgatg tgtgggaggt tacctttcta caaccaggac catgagaaac tttttgaatt
1080

aatattaatg gaagacatta aatttcctcg aacactctct tcagatgcaa aatcattgct
1140

ttcagggctc ttgataaagg atccaaataa acgccttggt ggaggaccag atgatgcaaa
1200

agaaattatg agacacagtt tcttctctgg agtaaactgg caagatgtat atgataaaaa
1260

gcttgtacct ccttttaaac ctcaagtaac atctgagaca gatactagat attttgatga
1320

agaatttaca gctcagacta ttacaataac accacctgaa aaatatgatg aggatggtat
1380

ggactgcatg gacaatgaga ggcggccgca tttccctcaa ttttcctact ctgcaagtgg
1440

acgagaataa gtctctttca ttctgctact tcactgtcat cttcaattta ttactgaaaa
1500

tgattcctgg acatcaccag tcctagctct tacacatagc aggggca
1547

<210> 136
<211> 19
<212> RNA
<213> Artificial

<220>
<223> Human Akt-3 siRNA 1 sense strand

<400> 136
ccaguggacu acuguuaua
19

<210> 137
<211> 19
<212> RNA
<213> Artificial

<220>
<223> Human Akt-3 siRNA 2 sense strand

<400> 137
gaauuguagu ccaacuuca
19

<210> 138
<211> 19
<212> RNA

<213>  Artificial

<220>
<223>  Human Akt-3 siRNA 3 sense strand

<400>  138
agaaucuaau gcuggacaa
19


<210>  139
<211>  19
<212>  RNA
<213>  Artificial

<220>
<223>  Human Akt-3 siRNA 4 sense strand

<400>  139
cagagguguu agaagauaa
19


<210>  140
<211>  19
<212>  RNA
<213>  Artificial

<220>
<223>  Human Akt-3 siRNA 5 sense strand

<400>  140
ugcaagugga cgagaauaa
19


<210>  141
<211>  556
<212>  PRT
<213>  Homo sapiens

<400>  141

Met Ala Arg Thr Thr Ser Gln Leu Tyr Asp Ala Val Pro Ile Gln Ser
1               5                   10                  15


Ser Val Val Leu Cys Ser Cys Pro Ser Pro Ser Met Val Arg Thr Gln
            20                  25                  30


Thr Glu Ser Ser Thr Pro Pro Gly Ile Pro Gly Gly Ser Arg Gln Gly
        35                  40                  45


Pro Ala Met Asp Gly Thr Ala Ala Glu Pro Arg Pro Gly Ala Gly Ser
    50                  55                  60

Leu Gln His Ala Gln Pro Pro Pro Gln Pro Arg Lys Lys Arg Pro Glu
65                  70                  75                  80

Asp Phe Lys Phe Gly Lys Ile Leu Gly Glu Gly Ser Phe Ser Thr Val
                85                  90                  95

Val Leu Ala Arg Glu Leu Ala Thr Ser Arg Glu Tyr Ala Ile Lys Ile
            100                 105                 110

Leu Glu Lys Arg His Ile Ile Lys Glu Asn Lys Val Pro Tyr Val Thr
        115                 120                 125

Arg Glu Arg Asp Val Met Ser Arg Leu Asp His Pro Phe Phe Val Lys
    130                 135                 140

Leu Tyr Phe Thr Phe Gln Asp Asp Glu Lys Leu Tyr Phe Gly Leu Ser
145                 150                 155                 160

Tyr Ala Lys Asn Gly Glu Leu Leu Lys Tyr Ile Arg Lys Ile Gly Ser
                165                 170                 175

Phe Asp Glu Thr Cys Thr Arg Phe Tyr Thr Ala Glu Ile Val Ser Ala
            180                 185                 190

Leu Glu Tyr Leu His Gly Lys Gly Ile Ile His Arg Asp Leu Lys Pro
        195                 200                 205

Glu Asn Ile Leu Leu Asn Glu Asp Met His Ile Gln Ile Thr Asp Phe
    210                 215                 220

Gly Thr Ala Lys Val Leu Ser Pro Glu Ser Lys Gln Ala Arg Ala Asn
225                 230                 235                 240

Ser Phe Val Gly Thr Ala Gln Tyr Val Ser Pro Glu Leu Leu Thr Glu
                245                 250                 255

Lys Ser Ala Cys Lys Ser Ser Asp Leu Trp Ala Leu Gly Cys Ile Ile
            260                 265                 270

Tyr Gln Leu Val Ala Gly Leu Pro Pro Phe Arg Ala Gly Asn Glu Tyr
        275                 280                 285

Leu Ile Phe Gln Lys Ile Ile Lys Leu Glu Tyr Asp Phe Pro Glu Lys
    290                 295                 300

Phe Phe Pro Lys Ala Arg Asp Leu Val Glu Lys Leu Leu Val Leu Asp
305                 310                 315                 320

Ala Thr Lys Arg Leu Gly Cys Glu Glu Met Glu Gly Tyr Gly Pro Leu
                325                 330                 335

Lys Ala His Pro Phe Phe Glu Ser Val Thr Trp Glu Asn Leu His Gln
            340                 345                 350

Gln Thr Pro Pro Lys Leu Thr Ala Tyr Leu Pro Ala Met Ser Glu Asp
        355                 360                 365

Asp Glu Asp Cys Tyr Gly Asn Tyr Asp Asn Leu Leu Ser Gln Phe Gly
    370                 375                 380

Cys Met Gln Val Ser Ser Ser Ser Ser Ser His Ser Leu Ser Ala Ser
385                 390                 395                 400

Asp Thr Gly Leu Pro Gln Arg Ser Gly Ser Asn Ile Glu Gln Tyr Ile
                405                 410                 415

His Asp Leu Asp Ser Asn Ser Phe Glu Leu Asp Leu Gln Phe Ser Glu
            420                 425                 430

Asp Glu Lys Arg Leu Leu Leu Glu Lys Gln Ala Gly Gly Asn Pro Trp
        435                 440                 445

His Gln Phe Val Glu Asn Asn Leu Ile Leu Lys Met Gly Pro Val Asp
    450                 455                 460

Lys Arg Lys Gly Leu Phe Ala Arg Arg Arg Gln Leu Leu Leu Thr Glu
465                 470                 475                 480

Gly Pro His Leu Tyr Tyr Val Asp Pro Val Asn Lys Val Leu Lys Gly
                485                 490                 495

Glu Ile Pro Trp Ser Gln Glu Leu Arg Pro Glu Ala Lys Asn Phe Lys
            500                 505                 510

Thr Phe Phe Val His Thr Pro Asn Arg Thr Tyr Tyr Leu Met Asp Pro
        515                 520                 525

Ser Gly Asn Ala His Lys Trp Cys Arg Lys Ile Gln Glu Val Trp Arg
    530                 535                 540

Gln Arg Tyr Gln Ser His Pro Asp Ala Ala Val Gln
545                 550                 555

<210>  142
<211>  1891
<212>  DNA
<213>  Homo sapiens

<400>  142
ccgcttcggg gaggaggacg ctgaggaggc gccgagccgc gcagcgctgc gggggaggcg
60

cccgcgccga cgcggggccc atggccagga ccaccagcca gctgtatgac gccgtgccca
120

tccagtccag cgtggtgtta tgttcctgcc catccccatc aatggtgagg acccagactg
180

agtccagcac gccccctggc attcctggtg gcagcaggca gggccccgcc atggacggca
240

ctgcagccga gcctcggccc ggcgccggct ccctgcagca tgcccagcct ccgccgcagc
300

ctcggaagaa gcggcctgag gacttcaagt ttgggaaaat ccttggggaa ggctcttttt
360

ccacggttgt cctggctcga gaactggcaa cctccagaga atatgcgatt aaaattctgg
420

agaagcgaca tatcataaaa gagaacaagg tcccctatgt aaccagagag cgggatgtca
480

tgtcgcgcct ggatcacccc ttctttgtta agctttactt cacatttcag gacgacgaga
540

agctgtattt cggccttagt tatgccaaaa atggagaact acttaaatat attcgcaaaa
600

tcggttcatt cgatgagacc tgtacccgat tttacacggc tgagatcgtg tctgctttag
660

agtacttgca cggcaagggc atcattcaca gggaccttaa accggaaaac attttgttaa
720

atgaagatat gcacatccag atcacagatt ttggaacagc aaaagtctta tccccagaga
780

gcaaacaagc cagggccaac tcattcgtgg gaacagcgca gtacgtttct ccagagctgc
840

tcacggagaa gtccgcctgt aagagttcag acctttgggc tcttggatgc ataatatacc
900

agcttgtggc aggactccca ccattccgag ctggaaacga gtatcttata tttcagaaga
960

tcattaagtt ggaatatgac tttccagaaa aattcttccc taaggcaaga gacctcgtgg
1020

agaaactttt ggttttagat gccacaaagc ggttaggctg tgaggaaatg gaaggatacg
1080

gacctcttaa agcacacccg ttcttcgagt ccgtcacgtg ggagaacctg caccagcaga
1140

cgcctccgaa gctcaccgct tacctgccgg ctatgtcgga agacgacgag gactgctatg
1200

gcaattatga caatctcctg agccagtttg gctgcatgca ggtgtcttcg tcctcctcct
1260

cacactccct gtcagcctcc gacacgggcc tgccccagag gtcaggcagc aacatagagc
1320

agtacattca cgatctggac tcgaactcct ttgaactgga cttacagttt tccgaagatg
1380

agaagaggtt gttgttggag aagcaggctg gcggaaaccc ttggcaccag tttgtagaaa
1440

ataatttaat actaaagatg ggcccagtgg ataagcggaa gggtttattt gcaagacgac
1500

gacagctgtt gctcacagaa ggaccacatt tatattatgt ggatcctgtc aacaaagttc
1560

tgaaaggtga aattccttgg tcacaagaac ttcgaccaga ggccaagaat tttaaaactt
1620

tctttgtcca cacgcctaac aggacgtatt atctgatgga ccccagcggg aacgcacaca
1680

agtggtgcag gaagatccag gaggtttgga ggcagcgata ccagagccac ccggacgccg
1740

ctgtgcagtg acgtggcctg cggccgggct gcccttcgct gccaggacac ctgccccagc
1800

gcggcttggc cgccatccgg gacgcttcca gaccacctgc cagccatcac aaggggaacg
1860

cagaggcgga aaccttgcag cattttattt t
1891

<210>  143
<211>  19
<212>  RNA
<213>  Artificial

<220>
<223>  PDK1 siRNA 1 sense strand

<400>  143
ggacucgaac uccuuugaa
19


<210>  144
<211>  19
<212>  RNA
<213>  Artificial

<220>
<223>  PDK1 siRNA 2 sense strand

<400>  144
cgccuaacag gacguauua
19


<210>  145
<211>  19
<212>  RNA
<213>  Artificial

<220>
<223>  PDK1 siRNA 3 sense strand

<400>  145
acgacgagaa gcuguauuu
19


<210>  146
<211>  19
<212>  RNA
<213>  Artificial

<220>
<223>  PDK1 siRNA 4 sense strand

<400>  146
cggccuuagu uaugccaaa
19


<210>  147
<211>  1130
<212>  PRT
<213>  Homo sapiens

<400> 147

Met Leu Glu Ile Cys Leu Lys Leu Val Gly Cys Lys Ser Lys Lys Gly
1               5                   10                  15

Leu Ser Ser Ser Ser Ser Cys Tyr Leu Glu Glu Ala Leu Gln Arg Pro
            20                  25                  30

Val Ala Ser Asp Phe Glu Pro Gln Gly Leu Ser Glu Ala Ala Arg Trp
        35                  40                  45

Asn Ser Lys Glu Asn Leu Leu Ala Gly Pro Ser Glu Asn Asp Pro Asn
    50                  55                  60

Leu Phe Val Ala Leu Tyr Asp Phe Val Ala Ser Gly Asp Asn Thr Leu
65                  70                  75                  80

Ser Ile Thr Lys Gly Glu Lys Leu Arg Val Leu Gly Tyr Asn His Asn
                85                  90                  95

Gly Glu Trp Cys Glu Ala Gln Thr Lys Asn Gly Gln Gly Trp Val Pro
            100                 105                 110

Ser Asn Tyr Ile Thr Pro Val Asn Ser Leu Glu Lys His Ser Trp Tyr
        115                 120                 125

His Gly Pro Val Ser Arg Asn Ala Ala Glu Tyr Pro Leu Ser Ser Gly
    130                 135                 140

Ile Asn Gly Ser Phe Leu Val Arg Glu Ser Glu Ser Ser Pro Ser Gln
145                 150                 155                 160

Arg Ser Ile Ser Leu Arg Tyr Glu Gly Arg Val Tyr His Tyr Arg Ile
                165                 170                 175

Asn Thr Ala Ser Asp Gly Lys Leu Tyr Val Ser Ser Glu Ser Arg Phe
            180                 185                 190

Asn Thr Leu Ala Glu Leu Val His His His Ser Thr Val Ala Asp Gly
        195                 200                 205

Leu Ile Thr Thr Leu His Tyr Pro Ala Pro Lys Arg Asn Lys Pro Thr
    210                 215                 220

Val Tyr Gly Val Ser Pro Asn Tyr Asp Lys Trp Glu Met Glu Arg Thr
225                 230                 235                 240

Asp Ile Thr Met Lys His Lys Leu Gly Gly Gly Gln Tyr Gly Glu Val
                245                 250                 255

Tyr Glu Gly Val Trp Lys Lys Tyr Ser Leu Thr Val Ala Val Lys Thr
            260                 265                 270

Leu Lys Glu Asp Thr Met Glu Val Glu Glu Phe Leu Lys Glu Ala Ala
        275                 280                 285

Val Met Lys Glu Ile Lys His Pro Asn Leu Val Gln Leu Leu Gly Val
    290                 295                 300

Cys Thr Arg Glu Pro Pro Phe Tyr Ile Ile Thr Glu Phe Met Thr Tyr
305                 310                 315                 320

Gly Asn Leu Leu Asp Tyr Leu Arg Glu Cys Asn Arg Gln Glu Val Asn
                325                 330                 335

Ala Val Val Leu Leu Tyr Met Ala Thr Gln Ile Ser Ser Ala Met Glu
            340                 345                 350

Tyr Leu Glu Lys Lys Asn Phe Ile His Arg Asp Leu Ala Ala Arg Asn
        355                 360                 365

Cys Leu Val Gly Glu Asn His Leu Val Lys Val Ala Asp Phe Gly Leu
    370                 375                 380

Ser Arg Leu Met Thr Gly Asp Thr Tyr Thr Ala His Ala Gly Ala Lys
385                 390                 395                 400

Phe Pro Ile Lys Trp Thr Ala Pro Glu Ser Leu Ala Tyr Asn Lys Phe
                405                 410                 415

Ser Ile Lys Ser Asp Val Trp Ala Phe Gly Val Leu Leu Trp Glu Ile
            420                 425                 430

Ala Thr Tyr Gly Met Ser Pro Tyr Pro Gly Ile Asp Arg Ser Gln Val
        435                 440                 445

Tyr Glu Leu Leu Glu Lys Asp Tyr Arg Met Lys Arg Pro Glu Gly Cys
    450                 455                 460

Pro Glu Lys Val Tyr Glu Leu Met Arg Ala Cys Trp Gln Trp Asn Pro
465                 470                 475                 480

Ser Asp Arg Pro Ser Phe Ala Glu Ile His Gln Ala Phe Glu Thr Met
                485                 490                 495

Phe Gln Glu Ser Ser Ile Ser Asp Glu Val Glu Lys Glu Leu Gly Lys
            500                 505                 510

Gln Gly Val Arg Gly Ala Val Thr Thr Leu Leu Gln Ala Pro Glu Leu
        515                 520                 525

Pro Thr Lys Thr Arg Thr Ser Arg Arg Ala Ala Glu His Arg Asp Thr
    530                 535                 540

Thr Asp Val Pro Glu Met Pro His Ser Lys Gly Gln Gly Glu Ser Asp
545                 550                 555                 560

Pro Leu Asp His Glu Pro Ala Val Ser Pro Leu Leu Pro Arg Lys Glu
                565                 570                 575

Arg Gly Pro Pro Glu Gly Gly Leu Asn Glu Asp Glu Arg Leu Leu Pro
            580                 585                 590

Lys Asp Lys Lys Thr Asn Leu Phe Ser Ala Leu Ile Lys Lys Lys Lys
        595                 600                 605

Lys Thr Ala Pro Thr Pro Pro Lys Arg Ser Ser Ser Phe Arg Glu Met
    610                 615                 620

Asp Gly Gln Pro Glu Arg Arg Gly Ala Gly Glu Glu Glu Gly Arg Asp
625                 630                 635                 640

Ile Ser Asn Gly Ala Leu Ala Phe Thr Pro Leu Asp Thr Ala Asp Pro
                645                 650                 655

Ala Lys Ser Pro Lys Pro Ser Asn Gly Ala Gly Val Pro Asn Gly Ala
            660                 665                 670

Leu Arg Glu Ser Gly Gly Ser Gly Phe Arg Ser Pro His Leu Trp Lys
        675                 680                 685

Lys Ser Ser Thr Leu Thr Ser Ser Arg Leu Ala Thr Gly Glu Glu Glu
    690                 695                 700

Gly Gly Gly Ser Ser Ser Lys Arg Phe Leu Arg Ser Cys Ser Val Ser
705                 710                 715                 720

Cys Val Pro His Gly Ala Lys Asp Thr Glu Trp Arg Ser Val Thr Leu
                725                 730                 735

Pro Arg Asp Leu Gln Ser Thr Gly Arg Gln Phe Asp Ser Ser Thr Phe
            740                 745                 750

Gly Gly His Lys Ser Glu Lys Pro Ala Leu Pro Arg Lys Arg Ala Gly
        755                 760                 765

Glu Asn Arg Ser Asp Gln Val Thr Arg Gly Thr Val Thr Pro Pro Pro
    770                 775                 780

Arg Leu Val Lys Lys Asn Glu Glu Ala Ala Asp Glu Val Phe Lys Asp
785                 790                 795                 800

Ile Met Glu Ser Ser Pro Gly Ser Ser Pro Pro Asn Leu Thr Pro Lys
                805                 810                 815

Pro Leu Arg Arg Gln Val Thr Val Ala Pro Ala Ser Gly Leu Pro His
            820                 825                 830

Lys Glu Glu Ala Trp Lys Gly Ser Ala Leu Gly Thr Pro Ala Ala Ala
        835                 840                 845

Glu Pro Val Thr Pro Thr Ser Lys Ala Gly Ser Gly Ala Pro Arg Gly
    850                 855                 860

Thr Ser Lys Gly Pro Ala Glu Glu Ser Arg Val Arg Arg His Lys His
865                 870                 875                 880

Ser Ser Glu Ser Pro Gly Arg Asp Lys Gly Lys Leu Ser Lys Leu Lys
                885                 890                 895

Pro Ala Pro Pro Pro Pro Pro Ala Ala Ser Ala Gly Lys Ala Gly Gly

            900                 905                 910

Lys Pro Ser Gln Arg Pro Gly Gln Glu Ala Ala Gly Glu Ala Val Leu
        915                 920                 925

Gly Ala Lys Thr Lys Ala Thr Ser Leu Val Asp Ala Val Asn Ser Asp
    930                 935                 940

Ala Ala Lys Pro Ser Gln Pro Ala Glu Gly Leu Lys Lys Pro Val Leu
945                 950                 955                 960

Pro Ala Thr Pro Lys Pro His Pro Ala Lys Pro Ser Gly Thr Pro Ile
                965                 970                 975

Ser Pro Ala Pro Val Pro Leu Ser Thr Leu Pro Ser Ala Ser Ser Ala
            980                 985                 990

Leu Ala Gly Asp Gln Pro Ser Ser Thr Ala Phe Ile Pro Leu Ile Ser
        995                 1000                1005

Thr Arg Val Ser Leu Arg Lys Thr Arg Gln Pro Pro Glu Arg Ala
    1010                1015                1020

Ser Gly Ala Ile Thr Lys Gly Val Val Leu Asp Ser Thr Glu Ala
    1025                1030                1035

Leu Cys Leu Ala Ile Ser Gly Asn Ser Glu Gln Met Ala Ser His
    1040                1045                1050

Ser Ala Val Leu Glu Ala Gly Lys Asn Leu Tyr Thr Phe Cys Val
    1055                1060                1065

Ser Tyr Val Asp Ser Ile Gln Gln Met Arg Asn Lys Phe Ala Phe
    1070                1075                1080

Arg Glu Ala Ile Asn Lys Leu Glu Asn Asn Leu Arg Glu Leu Gln
    1085                1090                1095

Ile Cys Pro Ala Ser Ala Gly Ser Gly Pro Ala Ala Thr Gln Asp
    1100                1105                1110

Phe Ser Lys Leu Leu Ser Ser Val Lys Glu Ile Ser Asp Ile Val
    1115                1120                1125

Gln Arg
    1130

<210>  148
<211>  3840
<212>  DNA
<213>  Homo sapiens

<400>  148
   ggccttcccc ctgcgaggat cgccgttggc ccgggttggc tttggaaagc ggcggtggct
   60
   ttgggccggg ctcggcctcg ggaacgccag gggcccctgg gtgcggacgg gcgcggccag
   120
   gagggggtta aggcgcaggc ggcggcgggg cgggggcggg cctggcgggc gccctctccg
   180
   ggccctttgt taacaggcgc gtcccggcca gcggagacgc ggccgccctg ggcgggcgcg
   240
   ggcggcgggc ggcggtgagg gcggcctgcg gggcggcgcc cgggggccgg gccgagccgg
   300
   gcctgagccg ggcccggacc gagctgggag aggggctccg gcccgatcgt tcgcttggcg
   360
   caaaatgttg gagatctgcc tgaagctggt gggctgcaaa tccaagaagg ggctgtcctc
   420
   gtcctccagc tgttatctgg aagaagccct tcagcggcca gtagcatctg actttgagcc
   480
   tcagggtctg agtgaagccg ctcgttggaa ctccaaggaa aaccttctcg ctggacccag
   540
   tgaaaatgac cccaaccttt tcgttgcact gtatgatttt gtggccagtg gagataacac
   600
   tctaagcata actaaaggtg aaaagctccg ggtcttaggc tataatcaca atggggaatg
   660
   gtgtgaagcc caaaccaaaa atggccaagg ctgggtccca agcaactaca tcacgccagt
   720
   caacagtctg gagaaacact cctggtacca tgggcctgtg tcccgcaatg ccgctgagta
   780
   tccgctgagc agcgggatca atggcagctt cttggtgcgt gagagtgaga gcagtcctag
   840
   ccagaggtcc atctcgctga gatacgaagg gagggtgtac cattacagga tcaacactgc
   900
   ttctgatggc aagctctacg tctcctccga gagccgcttc aacaccctgg ccgagttggt
   960
   tcatcatcat tcaacggtgg ccgacgggct catcaccacg ctccattatc cagccccaaa
   1020
   gcgcaacaag cccactgtct atggtgtgtc ccccaactac gacaagtggg agatggaacg
   1080
   cacggacatc accatgaagc acaagctggg cgggggccag tacggggagg tgtacgaggg
   1140
   cgtgtggaag aaatacagcc tgacggtggc cgtgaagacc ttgaaggagg acaccatgga
   1200
   ggtggaagag ttcttgaaag aagctgcagt catgaaagag atcaaacacc ctaacctagt
   1260
   gcagctcctt ggggtctgca cccgggagcc cccgttctat atcatcactg agttcatgac
   1320
   ctacgggaac ctcctggact acctgaggga gtgcaaccgg caggaggtga acgccgtggt
   1380
   gctgctgtac atggccactc agatctcgtc agccatggag tacctagaga agaaaaactt
   1440
   catccacaga gatcttgctg cccgaaactg cctggtaggg gagaaccact tggtgaaggt
   1500
   agctgatttt ggcctgagca ggttgatgac aggggacacc tacacagccc atgctggagc
   1560
   caagttcccc atcaaatgga ctgcacccga gagcctggcc tacaacaagt tctccatcaa
   1620
   gtccgacgtc tgggcatttg gagtattgct ttgggaaatt gctacctatg gcatgtcccc
   1680
   ttacccggga attgaccgtt cccaggtgta tgagctgcta gagaaggact accgcatgaa
   1740
   gcgcccagaa ggctgcccag agaaggtcta tgaactcatg cgagcatgtt ggcagtggaa
   1800
   tccctctgac cggccctcct ttgctgaaat ccaccaagcc tttgaaacaa tgttccagga
   1860
   atccagtatc tcagacgaag tggaaaagga gctggggaaa caaggcgtcc gtggggctgt
   1920
   gactaccttg ctgcaggccc cagagctgcc caccaagacg aggacctcca ggagagctgc
   1980
   agagcacaga gacaccactg acgtgcctga gatgcctcac tccaagggcc agggagagag
   2040
   cgatcctctg gaccatgagc ctgccgtgtc tccattgctc cctcgaaaag agcgaggtcc
   2100
   cccggagggc ggcctgaatg aagatgagcg ccttctcccc aaagacaaaa agaccaactt
   2160
   gttcagcgcc ttgatcaaga agaagaagaa gacagcccca acccctccca aacgcagcag
   2220
   ctccttccgg gagatggacg gccagccgga gcgcagaggg gccggcgagg aagagggccg
   2280
   agacatcagc aacggggcac tggctttcac ccccttggac acagctgacc cagccaagtc
   2340
   cccaaagccc agcaatgggg ctggggtccc caatggagcc ctccgggagt ccgggggctc
   2400
   aggcttccgg tctccccacc tgtggaagaa gtccagcacg ctgaccagca gccgcctagc
   2460
   caccggcgag gaggagggcg gtggcagctc cagcaagcgc ttcctgcgct cttgctccgt
   2520
   ctcctgcgtt ccccatgggg ccaaggacac ggagtggagg tcagtcacgc tgcctcggga
   2580
   cttgcagtcc acgggaagac agtttgactc gtccacattt ggagggcaca aaagtgagaa
   2640
   gccggctctg cctcggaaga gggcagggga gaacaggtct gaccaggtga cccgaggcac
   2700
   agtaacgcct ccccccaggc tggtgaaaaa gaatgaggaa gctgctgatg aggtcttcaa
   2760
   agacatcatg gagtccagcc cgggctccag cccgcccaac ctgactccaa aacccctccg
   2820
   gcggcaggtc accgtggccc ctgcctcggg cctcccccac aaggaagaag cctggaaagg
   2880
   cagtgcctta gggacccctg ctgcagctga gccagtgacc cccaccagca aagcaggctc
   2940
   aggtgcacca aggggcacca gcaagggccc cgccgaggag tccagagtga ggaggcacaa
   3000
   gcactcctct gagtcgccag ggagggacaa ggggaaattg tccaagctca aacctgcccc
   3060
   gccgccccca ccagcagcct ctgcagggaa ggctggagga aagccctcgc agaggcccgg
   3120
   ccaggaggct gccggggagg cagtcttggg cgcaaagaca aaagccacga gtctggttga
   3180
   tgctgtgaac agtgacgctg ccaagcccag ccagccggca gagggcctca aaaagcccgt
   3240
   gctcccggcc actccaaagc cacaccccgc caagccgtcg gggaccccca tcagcccagc
   3300
   ccccgttccc ctttccacgt tgccatcagc atcctcggcc ttggcagggg accagccgtc
   3360
   ttccactgcc ttcatccctc tcatatcaac ccgagtgtct cttcggaaaa cccgccagcc
   3420
   tccagagcgg gccagcggcg ccatcaccaa gggcgtggtc ttggacagca ccgaggcgct
   3480
   gtgcctcgcc atctctggga actccgagca gatggccagc cacagcgcag tgctggaggc
   3540
   cggcaaaaac ctctacacgt tctgcgtgag ctatgtggat tccatccagc aaatgaggaa
   3600
   caagtttgcc ttccgagagg ccatcaacaa actggagaat aatctccggg agcttcagat
   3660
   ctgcccggcg tcagcaggca gtggtccggc ggccactcag gacttcagca agctcctcag
   3720
   ttcggtgaag gaaatcagtg acatagtgca gaggtagcag cagtcagggg tcaggtgtca
   3780
   ggcccgtcgg agctgcctgc agcacatgcg ggctcgccca tacccatgac agtggctgag
   3840


<210> 149
<211> 19
<212> RNA
<213> Artificial

<220>
<223> C-abl siRNA 1 sense strand

<400> 149
acacucuaag cauaacuaa
19


<210> 150
<211> 19
<212> RNA
<213> Artificial

<220>
<223> C-abl siRNA 2 sense strand

<400> 150

ucacugaguu caugaccua
19


<210>  151
<211>  19
<212>  RNA
<213>  Artificial

<220>
<223>  C-abl siRNA 3 sense strand

<400>  151
ggaucaacac ugcuucuga
19


<210>  152
<211>  19
<212>  RNA
<213>  Artificial

<220>
<223>  C-abl siRNA 4 sense strand

<400>  152
cacucuaagc auaacuaaa
19


<210>  153
<211>  439
<212>  PRT
<213>  Homo sapiens

<400>  153

Met Pro Leu Asn Val Ser Phe Thr Asn Arg Asn Tyr Asp Leu Asp Tyr
1               5                   10                  15

Asp Ser Val Gln Pro Tyr Phe Tyr Cys Asp Glu Glu Glu Asn Phe Tyr
            20                  25                  30

Gln Gln Gln Gln Gln Ser Glu Leu Gln Pro Pro Ala Pro Ser Glu Asp
        35                  40                  45

Ile Trp Lys Lys Phe Glu Leu Leu Pro Thr Pro Pro Leu Ser Pro Ser
    50                  55                  60

Arg Arg Ser Gly Leu Cys Ser Pro Ser Tyr Val Ala Val Thr Pro Phe
65                  70                  75                  80

Ser Leu Arg Gly Asp Asn Asp Gly Gly Gly Gly Ser Phe Ser Thr Ala
                85                  90                  95

Asp Gln Leu Glu Met Val Thr Glu Leu Leu Gly Gly Asp Met Val Asn
            100                 105                 110

Gln Ser Phe Ile Cys Asp Pro Asp Asp Glu Thr Phe Ile Lys Asn Ile
        115                 120                 125

Ile Ile Gln Asp Cys Met Trp Ser Gly Phe Ser Ala Ala Ala Lys Leu
    130                 135                 140

Val Ser Glu Lys Leu Ala Ser Tyr Gln Ala Ala Arg Lys Asp Ser Gly
145                 150                 155                 160

Ser Pro Asn Pro Ala Arg Gly His Ser Val Cys Ser Thr Ser Ser Leu
                165                 170                 175

Tyr Leu Gln Asp Leu Ser Ala Ala Ala Ser Glu Cys Ile Asp Pro Ser
            180                 185                 190

Val Val Phe Pro Tyr Pro Leu Asn Asp Ser Ser Ser Pro Lys Ser Cys
        195                 200                 205

Ala Ser Gln Asp Ser Ser Ala Phe Ser Pro Ser Ser Asp Ser Leu Leu
    210                 215                 220

Ser Ser Thr Glu Ser Ser Pro Gln Gly Ser Pro Glu Pro Leu Val Leu
225                 230                 235                 240

His Glu Glu Thr Pro Pro Thr Thr Ser Ser Asp Ser Glu Glu Glu Gln
                245                 250                 255

Glu Asp Glu Glu Glu Ile Asp Val Val Ser Val Glu Lys Arg Gln Ala
            260                 265                 270

Pro Gly Lys Arg Ser Glu Ser Gly Ser Pro Ser Ala Gly Gly His Ser
        275                 280                 285

Lys Pro Pro His Ser Pro Leu Val Leu Lys Arg Cys His Val Ser Thr
    290                 295                 300

His Gln His Asn Tyr Ala Ala Pro Pro Ser Thr Arg Lys Asp Tyr Pro

305                 310                 315                 320

Ala Ala Lys Arg Val Lys Leu Asp Ser Val Arg Val Leu Arg Gln Ile
                325                 330                 335

Ser Asn Asn Arg Lys Cys Thr Ser Pro Arg Ser Ser Asp Thr Glu Glu
            340                 345                 350

Asn Val Lys Arg Arg Thr His Asn Val Leu Glu Arg Gln Arg Arg Asn
        355                 360                 365

Glu Leu Lys Arg Ser Phe Phe Ala Leu Arg Asp Gln Ile Pro Glu Leu
    370                 375                 380

Glu Asn Asn Glu Lys Ala Pro Lys Val Val Ile Leu Lys Lys Ala Thr
385                 390                 395                 400

Ala Tyr Ile Leu Ser Val Gln Ala Glu Glu Gln Lys Leu Ile Ser Glu
                405                 410                 415

Glu Asp Leu Leu Arg Lys Arg Arg Glu Gln Leu Lys His Lys Leu Glu
            420                 425                 430

Gln Leu Arg Asn Ser Cys Ala
        435

<210>  154
<211>  2121
<212>  DNA
<213>  Homo sapiens

<400>  154
ctgctcgcgg ccgccaccgc cgggccccgg ccgtccctgg ctcccctcct gcctcgagaa
60

gggcagggct tctcagaggc ttggcgggaa aaaagaacgg agggagggat cgcgctgagt
120

ataaaagccg gttttcgggg ctttatctaa ctcgctgtag taattccagc gagaggcaga
180

gggagcgagc gggcggccgg ctagggtgga agagccgggc gagcagagct gcgctgcggg
240

cgtcctggga agggagatcc ggagcgaata gggggcttcg cctctggccc agccctcccg
300

cttgatcccc caggccagcg gtccgcaacc cttgccgcat ccacgaaact ttgcccatag
360

cagcgggcgg gcactttgca ctggaactta caacacccga gcaaggacgc gactctcccg
420

acgcggggag gctattctgc ccatttgggg acacttcccc gccgctgcca ggacccgctt
480

ctctgaaagg ctctccttgc agctgcttag acgctggatt tttttcgggt agtggaaaac
540

cagcagcctc ccgcgacgat gcccctcaac gttagcttca ccaacaggaa ctatgacctc
600

gactacgact cggtgcagcc gtatttctac tgcgacgagg aggagaactt ctaccagcag
660

cagcagcaga gcgagctgca gcccccggcg cccagcgagg atatctggaa gaaattcgag
720

ctgctgccca ccccgcccct gtcccctagc cgccgctccg ggctctgctc gccctcctac
780

gttgcggtca cacccttctc ccttcgggga gacaacgacg gcggtggcgg gagcttctcc
840

acggccgacc agctggagat ggtgaccgag ctgctgggag gagacatggt gaaccagagt
900

ttcatctgcg acccggacga cgagaccttc atcaaaaaca tcatcatcca ggactgtatg
960

tggagcggct tctcggccgc cgccaagctc gtctcagaga agctggcctc ctaccaggct
1020

gcgcgcaaag acagcggcag cccgaacccc gcccgcggcc acagcgtctg ctccacctcc
1080

agcttgtacc tgcaggatct gagcgccgcc gcctcagagt gcatcgaccc ctcggtggtc
1140

ttcccctacc ctctcaacga cagcagctcg cccaagtcct gcgcctcgca agactccagc
1200

gccttctctc cgtcctcgga ttctctgctc tcctcgacgg agtcctcccc gcagggcagc
1260

cccgagcccc tggtgctcca tgaggagaca ccgcccacca ccagcagcga ctctgaggag
1320

gaacaagaag atgaggaaga aatcgatgtt gtttctgtgg aaaagaggca ggctcctggc
1380

aaaaggtcag agtctggatc accttctgct ggaggccaca gcaaacctcc tcacagccca
1440

ctggtcctca agaggtgcca cgtctccaca catcagcaca actacgcagc gcctccctcc
1500

actcggaagg actatcctgc tgccaagagg gtcaagttgg acagtgtcag agtcctgaga
1560

cagatcagca acaaccgaaa atgcaccagc cccaggtcct cggacaccga ggagaatgtc
1620

aagaggcgaa cacacaacgt cttggagcgc cagaggagga acgagctaaa acggagcttt
1680

tttgccctgc gtgaccagat cccggagttg gaaaacaatg aaaaggcccc caaggtagtt
1740

atccttaaaa aagccacagc atacatcctg tccgtccaag cagaggagca aaagctcatt
1800

tctgaagagg acttgttgcg gaaacgacga gaacagttga aacacaaact tgaacagcta
1860

cggaactctt gtgcgtaagg aaaagtaagg aaaacgattc cttctaacag aaatgtcctg
1920

agcaatcacc tatgaacttg tttcaaatgc atgatcaaat gcaacctcac aaccttggct
1980

gagtcttgag actgaaagat ttagccataa tgtaaactgc ctcaaattgg actttgggca
2040

taaaagaact tttttatgct taccatcttt tttttttctt taacagattt gtatttaaga
2100

attgttttta aaaaatttta a
2121


<210> 155
<211> 19
<212> RNA
<213> Artificial

<220>
<223> C-myc oncogene siRNA 1 sense strand

<400> 155
ggaucgcgcu gaguauaaa
19


<210> 156
<211> 19
<212> RNA
<213> Artificial

<220>
<223> C-myc oncogene siRNA 2 sense strand

<400>  156
ccugagcaau caccuauga
19


<210>  157
<211>  19
<212>  RNA
<213>  Artificial

<220>
<223>  C-myc oncogene siRNA 3 sense strand

<400>  157
ucaacguuag cuucaccaa
19


<210>  158
<211>  19
<212>  RNA
<213>  Artificial

<220>
<223>  C-myc oncogene siRNA 4 sense strand

<400>  158
aacguuagcu ucaccaaca
19


<210>  159
<211>  19
<212>  RNA
<213>  Artificial

<220>
<223>  C-myc oncogene siRNA 5 sense strand

<400>  159
ggaacuauga ccucgacua
19


<210>  160
<211>  19
<212>  RNA
<213>  Artificial

<220>
<223>  C-myc oncogene siRNA 6 sense strand

<400>  160
cggugcagcc guauuucua
19

<210> 161
<211> 19
<212> RNA
<213> Artificial

<220>
<223> C-myc oncogene siRNA 7 sense strand

<400> 161
acgacgagac cuucaucaa
19


<210> 162
<211> 19
<212> RNA
<213> Artificial

<220>
<223> C-myc oncogene siRNA 8 sense strand

<400> 162
uaucuaacuc gcuguagua
19


<210> 163
<211> 160
<212> PRT
<213> Homo sapiens

<400> 163

Met Val Pro Ser Ala Gly Gln Leu Ala Leu Phe Ala Leu Gly Ile Val
1               5                   10                  15

Leu Ala Ala Cys Gln Ala Leu Glu Asn Ser Thr Ser Pro Leu Ser Ala
            20                  25                  30

Asp Pro Pro Val Ala Ala Ala Val Val Ser His Phe Asn Asp Cys Pro
        35                  40                  45

Asp Ser His Thr Gln Phe Cys Phe His Gly Thr Cys Arg Phe Leu Val
    50                  55                  60

Gln Glu Asp Lys Pro Ala Cys Val Cys His Ser Gly Tyr Val Gly Ala
65                  70                  75                  80

Arg Cys Glu His Ala Asp Leu Leu Ala Val Val Ala Ala Ser Gln Lys
                85                  90                  95

Lys Gln Ala Ile Thr Ala Leu Val Val Val Ser Ile Val Ala Leu Ala
            100                 105                 110

Val Leu Ile Ile Thr Cys Val Leu Ile His Cys Cys Gln Val Arg Lys
        115                 120                 125

His Cys Glu Trp Cys Arg Ala Leu Ile Cys Arg His Glu Lys Pro Ser
    130                 135                 140

Ala Leu Leu Lys Gly Arg Thr Ala Cys Cys His Ser Glu Thr Val Val
145                 150                 155                 160

<210>  164
<211>  4119
<212>  DNA
<213>  Homo sapiens

<400>  164
ctggagagcc tgctgcccgc ccgcccgtaa aatggtcccc tcggctggac agctcgccct
60

gttcgctctg ggtattgtgt tggctgcgtg ccaggccttg gagaacagca cgtccccgct
120

gagtgcagac ccgcccgtgg ctgcagcagt ggtgtcccat tttaatgact gcccagattc
180

ccacactcag ttctgcttcc atggaacctg caggtttttg gtgcaggagg acaagccagc
240

atgtgtctgc cattctgggt acgttggtgc acgctgtgag catgcggacc tcctggccgt
300

ggtggctgcc agccagaaga agcaggccat caccgccttg gtggtggtct ccatcgtggc
360

cctggctgtc cttatcatca catgtgtgct gatacactgc tgccaggtcc gaaaacactg
420

tgagtggtgc cgggccctca tctgccggca cgagaagccc agcgccctcc tgaagggaag
480

aaccgcttgc tgccactcag aaacagtggt ctgaagagcc cagaggagga gtttggccag
540

gtggactgtg gcagatcaat aaagaaaggc ttcttcagga cagcactgcc agagatgcct
600

gggtgtgcca cagaccttcc tacttggcct gtaatcacct gtgcagcctt ttgtgggcct
660

tcaaaactct gtcaagaact ccgtctgctt ggggttattc agtgtgacct agagaagaaa
720

tcagcggacc acgatttcaa gacttgttaa aaaagaactg caaagagacg gactcctgtt
780

cacctaggtg aggtgtgtgc agcagttggt gtctgagtcc acatgtgtgc agttgtcttc
840

tgccagccat ggattccagg ctatatattt ctttttaatg ggccacctcc ccacaacaga
900

attctgccca acacaggaga tttctatagt tattgttttc tgtcatttgc ctactgggga
960

agaaagtgaa ggaggggaaa ctgtttaata tcacatgaag accctagctt taagagaagc
1020

tgtatcctct aaccacgaga ctctcaacca gcccaacatc ttccatggac acatgacatt
1080

gaagaccatc ccaagctatc gccacccttg gagatgatgt cttatttatt agatggataa
1140

tggttttatt tttaatctct taagtcaatg taaaaagtat aaaacccctt cagacttcta
1200

cattaatgat gtatgtgttg ctgactgaaa agctatactg attagaaatg tctggcctct
1260

tcaagacagc taaggcttgg gaaaagtctt ccagggtgcg gagatggaac cagaggctgg
1320

gttactggta ggaataaagg taggggttca gaaatggtgc cattgaagcc acaaagccgg
1380

taaatgcctc aatacgttct gggagaaaac ttagcaaatc catcagcagg gatctgtccc
1440

ctctgttggg gagagaggaa gagtgtgtgt gtctacacag gataaaccca atacatattg
1500

tactgctcag tgattaaatg ggttcacttc ctcgtgagcc ctcggtaagt atgtttagaa
1560

atagaacatt agccacgagc cataggcatt tcaggccaaa tccatgaaag gggaccagt
1620

catttatttt ccattttgtt gcttggttgg tttgttgctt tatttttaaa aggagaagtt
1680

taactttgct atttattttc gagcactagg aaaactattc cagtaatttt tttttcctca
1740

tttccattca ggatgccggc tttattaaca aaaactctaa caagtcacct ccactatgtg
1800

ggtcttcctt tcccctcaag agaaggagca attgttcccc tgacatctgg gtccatctga
1860

cccatggggc ctgcctgtga gaaacagtgg gtcccttcaa atacatagtg gatagctcat
1920

ccctaggaat tttcattaaa atttggaaac agagtaatga agaaataata tataaactcc
1980

ttatgtgagg aaatgctact aatatctgaa aagtgaaaga tttctatgta ttaactctta
2040

agtgcaccta gcttattaca tcgtgaaagg tacatttaaa atatgttaaa ttggcttgaa
2100

attttcagag aattttgtct tcccctaatt cttcttcctt ggtctggaag aacaatttct
2160

atgaattttc tctttatttt ttttttataa ttcagacaat tctatgaccc gtgtcttcat
2220

ttttggcact cttatttaac aatgccacac ctgaagcact tggatctgtt cagagctgac
2280

cccctagcaa cgtagttgac acagctccag gtttttaaat tactaaaata agttcaagtt
2340

tacatccctt gggccagata tgtgggttga ggcttgactg tagcatcctg cttagagacc
2400

aatcaatgga cactggtttt tagacctcta tcaatcagta gttagcatcc aagagacttt
2460

gcagaggcgt aggaatgagg ctggacagat ggcggaacga gaggttccct gcgaagactt
2520

gagatttagt gtctgtgaat gttctagttc ctaggtccag caagtcacac ctgccagtgc
2580

cctcatcctt atgcctgtaa cacacatgca gtgagaggcc tcacatatac gcctccctag
2640

aagtgccttc caagtcagtc ctttggaaac cagcaggtct gaaaaagagg ctgcatcaat
2700

gcaagcctgg ttggaccatt gtccatgcct caggatagaa cagcctggct tatttgggga
2760

tttttcttct agaaatcaaa tgactgataa gcattggctc cctctgccat ttaatggcaa
2820

tggtagtctt tggttagctg caaaaatact ccatttcaag ttaaaaatgc atcttctaat
2880

ccatctctgc aagctccctg tgtttccttg ccctttagaa aatgaattgt tcactacaat
2940

tagagaatca tttaacatcc tgacctggta agctgccaca cacctggcag tggggagcat
3000

cgctgtttcc aatggctcag gagacaatga aaagccccca tttaaaaaaa taacaaacat
3060

tttttaaaag gcctccaata ctcttatgga gcctggattt ttcccactgc tctacaggct
3120

gtgacttttt ttaagcatcc tgacaggaaa tgttttcttc tacatggaaa gatagacagc
3180

agccaaccct gatctggaag acagggcccc ggctggacac acgtggaacc aagccaggga
3240

tgggctggcc attgtgtccc cgcaggagag atgggcagaa tggccctaga gttcttttcc
3300

ctgagaaagg agaaaaagat gggattgcca ctcacccacc cacactggta agggaggaga
3360

atttgtgctt ctggagcttc tcaagggatt gtgttttgca ggtacagaaa actgcctgtt
3420

atcttcaagc caggttttcg agggcacatg ggtcaccagt tgctttttca gtcaatttgg
3480

ccgggatgga ctaatgaggc tctaacactg ctcaggagac ccctgccctc tagttggttc
3540

tgggctttga tctcttccaa cctgcccagt cacagaagga ggaatgactc aaatgcccaa
3600

aaccaagaac acattgcaga agtaagacaa acatgtatat ttttaaatgt tctaacataa
3660

gacctgttct ctctagccat tgatttacca ggctttctga aagatctagt ggttcacaca
3720

gagagagaga gagtactgaa aaagcaactc ctcttcttag tcttaataat ttactaaaat
3780

ggtcaacttt tcattatctt tattataata aacctgatgc ttttttttag aactccttac
3840

tctgatgtct gtatatgttg cactgaaaag gttaatattt aatgttttaa tttattttgt
3900

gtggtaagtt aattttgatt tctgtaatgt gttaatgtga ttagcagtta ttttccttaa
3960

tatctgaatt atacttaaag agtagtgagc aatataagac gcaattgtgt ttttcagtaa
4020

tgtgcattgt tattgagttg tactgtacct tatttggaag gatgaaggaa tgaacctttt
4080

tttcctaaaa aaaaaaaaaa aaaaaaaaaa aaaaaaaaa
4119


<210>  165
<211>  19
<212>  RNA
<213>  Artificial

<220>
<223>  TGFalpha siRNA 1 sense strand

<400>  165
ugaggaaaug cuacuaaua
19


<210>  166
<211>  19
<212>  RNA
<213>  Artificial

<220>
<223>  TGFalpha siRNA 2 sense strand

<400>  166
ugcgaagacu ugagauuua
19


<210>  167
<211>  19
<212>  RNA
<213>  Artificial

<220>
<223>  TGFalpha siRNA 3 sense strand

<400>  167
uuacucugau gucuguaua
19


<210>  168
<211>  19
<212>  RNA
<213>  Artificial

<220>
<223>  TGFalpha siRNA 4 sense strand

<400>  168
gaugccggcu uuauuaaca
19


<210>  169

<211>  19
<212>  RNA
<213>  Artificial

<220>
<223>  TGFalpha siRNA 5 sense strand

<400>  169
augccggcuu uauuaacaa
19


<210>  170
<211>  19
<212>  RNA
<213>  Artificial

<220>
<223>  TGFalpha siRNA 6 sense strand

<400>  170
ugccggcuuu auuaacaaa
19


<210>  171
<211>  19
<212>  RNA
<213>  Artificial

<220>
<223>  TGFalpha siRNA 7 sense strand

<400>  171
acuguggcag aucaauaaa
19


<210>  172
<211>  19
<212>  RNA
<213>  Artificial

<220>
<223>  TGFalpha siRNA 8 sense strand

<400>  172
ucgugaaagg uacauuuaa
19


<210>  173
<211>  19
<212>  RNA
<213>  Artificial

<220>

<223>  TGFalpha siRNA 9 sense strand

<400>  173
gccgguaaau gccucaaua
19


<210>  174
<211>  19
<212>  RNA
<213>  Artificial

<220>
<223>  TGFalpha siRNA 10 sense strand

<400>  174
cgguaaguau guuuagaaa
19


<210>  175
<211>  781
<212>  PRT
<213>  Homo sapiens

<400>  175

<210> 176
<211> 3362
<212> DNA
<213> Homo sapiens

<400> 176
aagcctctcg gtctgtggca gcagcgttgg cccggccccg ggagcggaga gcgaggggag
60

gcggagacgg aggaaggtct gaggagcagc ttcagtcccc gccgagccgc caccgcaggt
120

cgaggacggt cggactcccg cggcgggagg agcctgttcc cctgagggta tttgaagtat
180

accatacaac tgttttgaaa atccagcgtg gacaatggct actcaagctg atttgatgga
240

gttggacatg gccatggaac cagacagaaa agcggctgtt agtcactggc agcaacagtc
300

ttacctggac tctggaatcc attctggtgc cactaccaca gctccttctc tgagtggtaa
360

aggcaatcct gaggaagagg atgtggatac ctcccaagtc ctgtatgagt gggaacaggg
420

attttctcag tccttcactc aagaacaagt agctgatatt gatggacagt atgcaatgac
480

tcgagctcag agggtacgag ctgctatgtt ccctgagaca ttagatgagg gcatgcagat
540

cccatctaca cagtttgatg ctgctcatcc cactaatgtc cagcgtttgg ctgaaccatc
600

acagatgctg aaacatgcag ttgtaaactt gattaactat caagatgatg cagaacttgc
660

cacacgtgca atccctgaac tgacaaaact gctaaatgac gaggaccagg tggtggttaa
720

taaggctgca gttatggtcc atcagctttc taaaaaggaa gcttccagac acgctatcat
780

gcgttctcct cagatggtgt ctgctattgt acgtaccatg cagaatacaa atgatgtaga
840

aacagctcgt tgtaccgctg ggaccttgca taacctttcc catcatcgtg agggcttact
900

ggccatcttt aagtctggag gcattcctgc cctggtgaaa atgcttggtt caccagtgga
960

ttctgtgttg ttttatgcca ttacaactct ccacaacctt ttattacatc aagaaggagc
1020

taaaatggca gtgcgtttag ctggtgggct gcagaaaatg gttgccttgc tcaacaaaac
1080

aaatgttaaa ttcttggcta ttacgacaga ctgccttcaa attttagctt atggcaacca
1140

agaaagcaag ctcatcatac tggctagtgg tggaccccaa gctttagtaa atataatgag
1200

gacctatact tacgaaaaac tactgtggac cacaagcaga gtgctgaagg tgctatctgt
1260

ctgctctagt aataagccgg ctattgtaga agctggtgga atgcaagctt taggacttca
1320

cctgacagat ccaagtcaac gtcttgttca gaactgtctt tggactctca ggaatctttc
1380

agatgctgca actaaacagg aagggatgga aggtctcctt gggactcttg ttcagcttct
1440

gggttcagat gatataaatg tggtcacctg tgcagctgga attctttcta acctcacttg
1500

caataattat aagaacaaga tgatggtctg ccaagtgggt ggtatagagg ctcttgtgcg
1560

tactgtcctt cgggctggtg acagggaaga catcactgag cctgccatct gtgctcttcg
1620

tcatctgacc agccgacacc aagaagcaga gatggcccag aatgcagttc gccttcacta
1680

tggactacca gttgtggtta agctcttaca cccaccatcc cactggcctc tgataaaggc
1740

tactgttgga ttgattcgaa atcttgccct ttgtcccgca aatcatgcac ctttgcgtga
1800

gcagggtgcc attccacgac tagttcagtt gcttgttcgt gcacatcagg atacccagcg
1860

ccgtacgtcc atgggtggga cacagcagca atttgtggag ggggtccgca tggaagaaat
1920

agttgaaggt tgtaccggag cccttcacat cctagctcgg gatgttcaca accgaattgt
1980

tatcagagga ctaaatacca ttccattgtt tgtgcagctg ctttattctc ccattgaaaa
2040

catccaaaga gtagctgcag gggtcctctg tgaacttgct caggacaagg aagctgcaga
2100

agctattgaa gctgagggag ccacagctcc tctgacagag ttacttcact ctaggaatga
2160

aggtgtggcg acatatgcag ctgctgtttt gttccgaatg tctgaggaca agccacaaga
2220

ttacaagaaa cggctttcag ttgagctgac cagctctctc ttcagaacag agccaatggc
2280

ttggaatgag actgctgatc ttggacttga tattggtgcc cagggagaac cccttggata
2340

tcgccaggat gatcctagct atcgttcttt tcactctggt ggatatggcc aggatgcctt
2400

gggtatggac cccatgatgg aacatgagat gggtggccac caccctggtg ctgactatcc
2460

agttgatggg ctgccagatc tggggcatgc ccaggacctc atggatgggc tgcctccagg
2520

tgacagcaat cagctggcct ggtttgatac tgacctgtaa atcatccttt agctgtattg
2580

tctgaacttg cattgtgatt ggcctgtaga gttgctgaga gggctcgagg ggtgggctgg
2640

tatctcagaa agtgcctgac acactaacca agctgagttt cctatgggaa caattgaagt
2700

aaactttttg ttctggtcct ttttggtcga ggagtaacaa tacaaatgga ttttgggagt
2760

gactcaagaa gtgaagaatg cacaagaatg gatcacaaga tggaatttag caaaccctag
2820

ccttgcttgt taaaattttt tttttttttt ttttaagaat atctgtaatg gtactgactt
2880

tgcttgcttt gaagtagctc tttttttttt tttttttttt tttttttgca gtaactgttt
2940

tttaagtctc tcgtagtgtt aagttatagt gaatactgct acagcaattt ctaattttta
3000

agaattgagt aatggtgtag aacactaatt aattcataat cactctaatt aattgtaatc
3060

tgaataaagt gtaacaattg tgtagccttt ttgtataaaa tagacaaata gaaaatggtc
3120

caattagttt cctttttaat atgcttaaaa taagcaggtg gatctatttc atgtttttga
3180

tcaaaaacta tttgggatat gtatgggtag ggtaaatcag taagaggtgt tatttggaac
3240

cttgttttgg acagtttacc agttgccttt tatcccaaag ttgttgtaac ctgctgtgat
3300

acgatgcttc aagagaaaat gcggttataa aaaatggttc agaattaaac ttttaattca
3360

tt
3362

<210> 177
<211> 19
<212> RNA
<213> Artificial

<220>
<223> Human beta-catenin siRNA 1 sense strand

<400> 177
cucguagugu uaaguuaua
19


<210> 178
<211> 19
<212> RNA
<213> Artificial

<220>
<223> Human beta-catenin siRNA 2 sense strand

<400> 178
gcugaaacau gcaguugua
19


<210> 179
<211> 19
<212> RNA
<213> Artificial

<220>
<223> Human beta-catenin siRNA 3 sense strand

<400> 179
guagaaacag cucguugua
19


<210> 180
<211> 19
<212> RNA
<213> Artificial

<220>
<223> Human beta-catenin siRNA 4 sense strand

<400> 180
agaacaagua gcugauauu
19


<210> 181
<211> 142
<212> PRT

<213>  Homo sapiens

<400>  181

Met Gly Ala Pro Thr Leu Pro Pro Ala Trp Gln Pro Phe Leu Lys Asp
1               5                   10                  15

His Arg Ile Ser Thr Phe Lys Asn Trp Pro Phe Leu Glu Gly Cys Ala
            20                  25                  30

Cys Thr Pro Glu Arg Met Ala Glu Ala Gly Phe Ile His Cys Pro Thr
        35                  40                  45

Glu Asn Glu Pro Asp Leu Ala Gln Cys Phe Phe Cys Phe Lys Glu Leu
    50                  55                  60

Glu Gly Trp Glu Pro Asp Asp Asp Pro Ile Glu Glu His Lys Lys His
65                  70                  75                  80

Ser Ser Gly Cys Ala Phe Leu Ser Val Lys Lys Gln Phe Glu Glu Leu
                85                  90                  95

Thr Leu Gly Glu Phe Leu Lys Leu Asp Arg Glu Arg Ala Lys Asn Lys
            100                 105                 110

Ile Ala Lys Glu Thr Asn Asn Lys Lys Lys Glu Phe Glu Glu Thr Ala
        115                 120                 125

Lys Lys Val Arg Arg Ala Ile Glu Gln Leu Ala Ala Met Asp
    130                 135                 140


<210>  182
<211>  1643
<212>  DNA
<213>  Homo sapiens

<400>  182
agatttgaat cgcgggaccc gttggcagag gtggcggcgg cggcatgggt gccccgacgt
60

tgccccctgc ctggcagccc tttctcaagg accaccgcat ctctacattc aagaactggc
120

ccttcttgga gggctgcgcc tgcaccccgg agcggatggc cgaggctggc ttcatccact
180

gccccactga gaacgagcca gacttggccc agtgtttctt ctgcttcaag gagctggaag
240

gctgggagcc agatgacgac cccatagagg aacataaaaa gcattcgtcc ggttgcgctt
300

tcctttctgt caagaagcag tttgaagaat taacccttgg tgaatttttg aaactggaca
360

gagaaagagc caagaacaaa attgcaaagg aaaccaacaa taagaagaaa gaatttgagg
420

aaactgcgaa gaaagtgcgc cgtgccatcg agcagctggc tgccatggat tgaggcctct
480

ggccggagct gcctggtccc agagtggctg caccacttcc agggtttatt ccctggtgcc
540

accagccttc ctgtgggccc cttagcaatg tcttaggaaa ggagatcaac attttcaaat
600

tagatgtttc aactgtgctc ttgttttgtc ttgaaagtgg caccagaggt gcttctgcct
660

gtgcagcggg tgctgctggt aacagtggct gcttctctct ctctctctct tttttggggg
720

ctcatttttg ctgttttgat tcccgggctt accaggtgag aagtgaggga ggaagaaggc
780

agtgtccctt ttgctagagc tgacagcttt gttcgcgtgg gcagagcctt ccacagtgaa
840

tgtgtctgga cctcatgttg ttgaggctgt cacagtcctg agtgtggact tggcaggtgc
900

ctgttgaatc tgagctgcag gttccttatc tgtcacacct gtgcctcctc agaggacagt
960

ttttttgttg tgtttttttt tttttttttt ggtagatgca tgacttgtgt gtgatgagag
1020

aatggagaca gagtccccgg ctcctctact gtttaacaac atggctttct tattttgttt
1080

gaattgttaa ttcacagaat agcacaaact acaattaaaa ctaagcacaa agccattcta
1140

agtcattggg gaaacggggt gaacttcagg tggatgagga gacagaatag agtgatagga
1200

agcgtctggc agatactcct tttgccactg ctgtgtgatt agacaggccc agtgagccgc
1260

ggggcacatg ctggccgctc ctccctcaga aaaaggcagt ggcctaaatc ctttttaaat
1320

gacttggctc gatgctgtgg gggactggct gggctgctgc aggccgtgtg tctgtcagcc
1380

caaccttcac atctgtcacg ttctccacac gggggagaga cgcagtccgc ccaggtcccc
1440

gctttctttg gaggcagcag ctcccgcagg gctgaagtct ggcgtaagat gatggatttg
1500

attcgccctc ctccctgtca tagagctgca gggtggattg ttacagcttc gctggaaacc
1560

tctggaggtc atctcggctg ttcctgagaa ataaaaagcc tgtcatttca aataaaaaaa
1620

aaaaaaaaaa aaaaaaaaaa aaa
1643

<210> 183
<211> 19
<212> RNA
<213> Artificial

<220>
<223> Human survivin siRNA 1 sense strand

<400> 183
agaauagcac aaacuacaa
19

<210> 184
<211> 19
<212> RNA
<213> Artificial

<220>
<223> Human survivin siRNA 2 sense strand

<400> 184
gcaaugucuu aggaaagga
19

<210> 185
<211> 21
<212> DNA/RNA
<213> Artificial

<220>
<223> siRNA 1 sense strand

<400> 185
agcugauauu gauggacagt t
21

<210> 186

<211>  21
<212>  DNA/RNA
<213>  Artificial

<220>
<223>  siRNA 1 antisense strand

<400>  186
ttucgacuau aacuaccugu c
21


<210>  187
<211>  21
<212>  DNA/RNA
<213>  Artificial

<220>
<223>  siRNA 2 sense strand

<400>  187
gugaagucaa caugccugct t
21


<210>  188
<211>  21
<212>  DNA/RNA
<213>  Artificial

<220>
<223>  siRNA 2 antisense strand

<400>  188
ttcacuucag uuguacggac g
21


<210>  189
<211>  21
<212>  DNA/RNA
<213>  Artificial

<220>
<223>  siRNA 3 sense strand

<400>  189
ccacgucugu aauccuuuat t
21


<210>  190
<211>  21
<212>  DNA/RNA
<213>  Artificial

<220>

<223>  siRNA 3 antisense strand

<400>  190
ttggugcaga cauuaggaaa u
21


<210>  191
<211>  21
<212>  DNA/RNA
<213>  Artificial

<220>
<223>  siRNA 4 sense strand

<400>  191
ggacaggagu gacacuauut t
21


<210>  192
<211>  21
<212>  DNA/RNA
<213>  Artificial

<220>
<223>  siRNA 4 antisense strand

<400>  192
ttccuguccu cacugugaua a
21


<210>  193
<211>  21
<212>  DNA/RNA
<213>  Artificial

<220>
<223>  siRNA 5 sense strand

<400>  193
ccgaagagcc cauuuacaut t
21


<210>  194
<211>  21
<212>  DNA/RNA
<213>  Artificial

<220>
<223>  siRNA 5 antisense strand

<400>  194
ttggcuucuc ggguaaaugu a
21

<210>  195
<211>  21
<212>  DNA/RNA
<213>  Artificial

<220>
<223>  siRNA 6 sense strand

<400>  195
ugccguucgc cuucauuaut t
21


<210>  196
<211>  21
<212>  DNA/RNA
<213>  Artificial

<220>
<223>  siRNA 6 antisense strand

<400>  196
ttacggcaag cggaaguaau a
21


<210>  197
<211>  19
<212>  DNA/RNA
<213>  Artificial

<220>
<223>  siRNA 7 sense strand

<400>  197
aggugguagu uaauaaatt
19


<210>  198
<211>  19
<212>  DNA/RNA
<213>  Artificial

<220>
<223>  siRNA 7 antisense strand

<400>  198
gguccaccau caauuauuu
19


<210>  199
<211>  19
<212>  DNA/RNA

<213> Artificial

<220>
<223> siRNA 8 sense strand

<400> 199
aacuauuucc gcaauuatt
19


<210> 200
<211> 19
<212> DNA/RNA
<213> Artificial

<220>
<223> siRNA 8 antisense strand

<400> 200
ttuugauaaa ggcguuaau
19